(19) Europäisches Patentamt
European Patent Office
Office européen des brevets

(11) EP 0 444 482 B1

(12) **EUROPÄISCHE PATENTSCHRIFT**

(45) Veröffentlichungstag und Bekanntmachung des Hinweises auf die Patenterteilung:
**17.01.1996 Patentblatt 1996/03**

(51) Int Cl.[6]: **C07D 305/12**, C07D 405/12, A61K 31/335, A61K 31/40

(21) Anmeldenummer: **91102151.7**

(22) Anmeldetag: **15.02.1991**

(54) **Oxetanone**

Oxetanones

Oxetanones

(84) Benannte Vertragsstaaten:
**AT BE CH DE DK ES FR GB GR IT LI LU NL SE**

(30) Priorität: **26.02.1990 CH 604/90**
**17.12.1990 CH 4005/90**

(43) Veröffentlichungstag der Anmeldung:
**04.09.1991 Patentblatt 1991/36**

(73) Patentinhaber: **F. HOFFMANN-LA ROCHE AG**
**CH-4002 Basel (CH)**

(72) Erfinder:
- **Derungs, Romano, Dr.**
  **CH-4125 Riehen (CH)**
- **Märki, Hans Peter, Dr.**
  **CH-4059 Basel (CH)**
- **Stalder, Henri, Dr.**
  **CH-4056 Basel (CH)**
- **Szente, André, Dr.**
  **CH-4125 Riehen (CH)**

(74) Vertreter: **Lederer, Franz, Dr. et al**
**D-80538 München (DE)**

(56) Entgegenhaltungen:
**FR-A- 2 379 531** **FR-A- 2 426 679**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 444 482 B1

## Beschreibung

Die vorliegende Erfindung betrifft neue Oxetanone, Verfahren zu deren Herstellung, pharmazeutische Präparate die solche Oxetanone enthalten, sowie die Verwendung dieser Oxetanone bei der Herstellung pharmazeutischer Präparate.

Diese Oxetanone haben die Formel

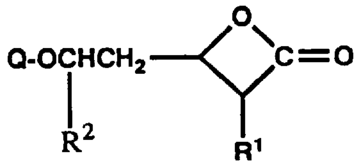

I

worin Q eine Gruppe der Formel

$(R^3,R^4)NCO(X)n\text{-}CO\text{-}$ Q1

$(R^3,R^4)NCO\text{-}X'\text{-}$ Q2

oder

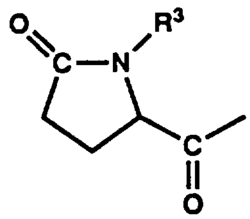

$Q^3$

ist und

$R^1$ und $R^2$ durch 1 bis 3 Halogenatome substituiertes Alkyl mit bis zu 18 C-Atomen oder gegebenenfalls durch eine 1,4-Arylengruppe unterbrochene, gegebenenfalls durch eine Arylgruppe in ω-Stellung und gegebenenfalls durch eine Aryl-$C_{1-4}$-alkylgruppe substituierte Alkyl-, Alkenyl-, Alkinyl- oder Alkadienylgruppen mit bis zu 20 C-Atomen sind, wobei $R^1$ in einer anderen als der α-Stellung zu einem ungesättigten C-Atom durch ein O- oder S-Atom oder durch eine Sulfinyl- oder Sulfonylgruppe unterbrochen sein kann, oder $R^1$ eine Gruppe Aryl-NH- oder Aryl-$C_{1-4}$-alkyl-OCONH-, $R^3$ und $R^4$ Wasserstoff oder $C_{1-4}$-Alkyl sind oder zusammen mit dem N-Atom, an dem sie gebunden sind, einen gesättigten 3- bis 6-gliedrigen, gegebenenfalls ein O- oder S-Atom in einer anderen als der α-Stellung zum N-Atom enthaltenden Ring bilden, n die Zahl 1 oder 0,

X eine gegebenenfalls durch ein O- oder S-Atom oder durch eine Sulfinyl- oder Sulfonylgruppe unterbrochene Alkylengruppe ist, die bis zu 6 C-Atome enthält und gegebenenfalls durch eine Hydroxy-, Mercapto-, Aryl-, Aryloxy-, Arylthio-, Aryl-$C_{1-4}$-alkyl-, Aryl-$C_{1-4}$-alkoxy-, Aryl-$C_{1-4}$-alkylthio-, Aryl-$C_{1-4}$-alkyliden-, $C_{3-7}$-Cycloalkyliden- oder $C_{1-6}$-Alkylidengruppe oder durch eine oder zwei $C_{1-6}$-Alkyl-, $C_{1-6}$-Alkoxy- oder $C_{1-6}$-Alkylthiogruppen substituiert ist, wobei zwei am gleichen C-Atom oder an zwei benachbarten C-Atomen gebundene $C_{1-6}$-Alkyl-, $C_{1-6}$-Alkoxy- oder $C_{1-6}$-Alkylthiogruppen einen gegebenenfalls monoungesättigten 3- bis 7-gliedrigen Ring bilden können und eine gegebenenfalls vorhandene Hydroxy- oder Mercaptogruppe oder gegebenenfalls vorhandenes ungesättigtes C-Atom in einer anderen als der α-Stellung zu einem gegebenenfalls vorhandenen O- oder S-Atom oder einer gegebenenfalls vorhandenen Sulfinyl- oder Sulfonylgruppe liegen muss, oder X eine Gruppe der Formel

$$=CHN(R,R^o) \text{ oder } -CHN(R,R^o)CH_2-$$

R und R° Wasserstoff, $C_{1-4}$-Alkyl, $C_{1-4}$-Alkyl(CO oder OCO)-, Aryl, Aryl(CO oder OCO)-, Aryl-$C_{1-4}$-alkyl oder Aryl-$C_{1-4}$-alkyl(CO oder OCO)- sind und

X' eine bis zu 6 C-Atome enthaltende Alkylengruppe ist, die durch eine $C_{1-4}$-Alkoxy-, Aryl-, Aryloxy-, Arylthio-, Aryl-$C_{1-4}$-alkyl-, Aryl-$C_{1-4}$-alkoxy- oder Aryl-$C_{1-4}$-alkylthiogruppe oder durch eine oder zwei $C_{1-6}$-Alkylgruppe(n) substituiert sein kann, wobei zwei an benachbarten C-Atome gebundene $C_{1-6}$-Alkylgruppen einen 3- bis 7-gliedrigen Ring bilden können.

Das in FR 2426679 bzw. FR 2379531 beschriebene Esterastin und seine Derivate enthalten eine Estergruppe der Formel:

$$OC(O)CH[NHC(O)CH_3]CH_2C(O)NH_2$$

Hingegen hat die vorliegende Estergruppe $OQ^1$ die Formel:

$$OC(O)CH[N(R,R^{o})]C(O)N(R^{3},R^{4})$$

oder

$$OC(O)CH_2CH[N(R,R^{o})]C(O)N(R^{3},R^{4}),$$

je nachdem X eine Gruppe der Formel

$$CHN(R,R^{o}) \text{ bzw. } CHN(R,R^{o})CH_2$$

ist.

Die Alkyl-, Alkenyl- und Alkadienylgruppen können geradkettig oder verzweigt sein. Beispiele von Alkylgruppen sind Methyl, Aethyl, Propyl, i-Propyl, Butyl, i-Butyl, Pentyl, Hexyl, Undecyl und Heptadecyl.

"Aryl" und "Arylen" bezeichnen Phenyl bzw. Phenylen oder durch bis zu 5 Halogenatome oder bis zu 3 $C_{1-4}$-Alkyl-, $C_{1-4}$-Alkoxy- oder Nitrogruppen substituiertes Phenyl bzw. Phenylen.

Bevorzugte Oxetanone der Formel I sind diejenigen, worin Q eine Gruppe $Q^1$, $R^1$ und $R^2$ gegebenenfalls durch eine 1,4-Phenylengruppe unterbrochene, gegebenenfalls durch eine Phenylgruppe in ω-Stellung und gegebenenfalls durch eine Phenyl-$C_{1-4}$-alkylgruppe substituierte Alkyl-, Alkenyl- oder Alkadienylgruppen mit bis zu 20 C-Atomen sind, wobei $R^1$ in einer anderen als der α-Stellung zu einem ungesättigten C-Atom durch ein O- oder S-Atom unterbrochen sein kann, X eine gegebenenfalls durch ein O- oder S-Atom unterbrochene Alkylengruppe ist, die bis zu 6 C-Atome enthält und gegebenenfalls durch eine Hydroxy-, Mercapto-, Phenyl-, Phenoxy-, Phenylthio-, Phenyl-$C_{1-4}$-alkyl-, Phenyl-$C_{1-4}$-alkoxy-, Phenyl-$C_{1-4}$-alkylthio-, Phenyl-$C_{1-4}$-alkyliden-, $C_{3-7}$-Cycloalkyliden- oder $C_{1-6}$-Alkylidengruppe oder durch eine oder zwei $C_{1-6}$-Alkyl-, $C_{1-6}$-Alkoxy- oder $C_{1-6}$-Alkylthiogruppen substituiert ist, wobei zwei am gleichen C-Atom gebundene $C_{1-6}$-Alkyl-, $C_{1-6}$-Alkoxy- oder $C_{1-6}$-Alkylthiogruppen einen 3- bis 7-gliedrigen Ring bilden können und eine gegebenenfalls vorhandene Hydroxy- oder Mercaptogruppe in einer anderen als der α-Stellung zu einem gegebenenfalls vorhandenen O- oder S-Atom vorliegen muss, oder X eine Gruppe $=CHN(R,R^o)$, R und $R^o$ Wasserstoff, $C_{1-4}$-Alkyl, $C_{1-4}$-Alkyl-(CO oder OCO)-, Phenyl oder Phenyl-(CO oder OCO)- sind und n, $R^3$ und $R^4$ die oben angegebene Bedeutung haben.

Weitere bevorzugte Oxetanone der Formel I, worin Q eine Gruppe $Q^1$ ist, sind diejenigen, worin $R^1$ und $R^2$ gegebenenfalls durch eine Arylgruppe in ω-Stellung substituierte Alkyl-, Alkenyl-, Alkinyl- oder Alkadienylgruppen mit bis zu 20 C-Atomen sind, wobei $R^1$ in einer anderen als der α-Stellung zu einem ungesättigten C-Atom durch ein S-Atom unterbrochen sein kann, oder $R^1$ Anilino, durch ein Halogenatom substituiertes Alkyl mit bis zu 18 C-Atomen oder eine Gruppe Phenyl-$C_{1-4}$-alkyl-OCONH-, $R^3$ und $R^4$ Wasserstoff oder $C_{1-4}$-Alkyl sind oder zusammen mit dem N-Atom, an dem sie gebunden sind, einen gesättigten O- oder S-Atom in einer anderen als der α-Stellung zum N-Atom enthaltenden 6-gliedrigen Ring bilden, n die Zahl 1 oder 0, X eine gegebenenfalls durch ein O- oder S-Atom oder durch eine Sulfinylgruppe unterbrochene Alkylengruppe ist, die bis zu 6 C-Atome enthält und gegebenenfalls durch eine oder zwei $C_{1-6}$-Alkyl- oder $C_{1-6}$-Alkoxygruppen substituiert ist, wobei zwei am gleichen C-Atom oder an zwei benachbarten C-Atome gebundene $C_{1-6}$-Alkyl- oder $C_{1-6}$-Alkoxygruppen einen gegebenenfalls monoungesättigten 3- bis 7-gliedrigen Ring bilden können, oder X eine Gruppe $=CHN(R,R^o)$ oder $-CHN(R,R^o)CH_2$-, und R und R° Wasserstoff, $C_{2-5}$-Alkanoyl oder Benzyloxycarbonyl sind.

Bevorzugt sind ferner die Oxetanone der Formel I, worin Q eine Gruppe $Q^2$; $R^1$ und $R^2$ $C_{1-20}$-Alkyl, $R^3$ und $R^4$ Wasserstoff und X' eine bis zu 6 C-Atome enthaltende Alkylengruppe, die durch eine $C_{1-4}$-Alkoxy oder durch eine oder zwei $C_{1-6}$-Alkygruppe(n) substituiert sein kann, wobei zwei an benachbarten C-Atome gebundene $C_{1-6}$-Alkylgruppen einen 3- bis 7-gliedrigen Ring bilden können.

Ebenfalls bevorzugt sind die Oxetanone der Formel I, worin Q eine Gruppe $Q^3$; $R^3$ Wasserstoff und $R^1$ und $R^2$ $C_{1-20}$-Alkyl, insbesondere Hexyl bzw. Undecyl, sind.

Besonders bevorzugt unter den Oxetanonen der Formel I, worin Q eine Gruppe $Q^1$ ist, sind diejenigen, worin $R^1$ Methyl, Aethyl, Propyl, Hexyl, 2-Butenyl, 3-Methyl-2-butenyl, 2-Propinyl, Methylthio, Pentylthio, 5-Chlorpentyl, Benzyl, Phenylthio, Benzylthio, Pentafluorbenzyl, Anilino oder Benzyloxycarbonylamino, $R^2$ Undecyl, Heptadecyl oder 8,11-Heptadecadienyl, $R^3$ und $R^4$ Wasserstoff, Methyl oder Isopropyl sind oder zusammen mit dem N-Atom eine Morpholino- oder Thiomorpholinogruppe bilden, n die Zahl 1 oder 0, und X die Gruppe $(CH_2)_{1-8}$, Aethyliden, Propyliden, Isopropyliden, Butyliden, Isobutyliden, Pentyliden, Isopentyliden, t-Butylmethylen, Dimethylvinyliden, Cyclopentyliden, Cyclohexyliden, Phenäthyliden, Phenylpropyliden, 1,2-Cyclohexylen, Cyclohex-3-en-1,6-ylen, Acetamidomethylen, Benzyloxycarbonylaminomethylen, 1-Benzyloxycarbonylamino-1,2-äthylen, Methylenoxymethylen, Methylenthiomethylen, Methylensulfinylmethylen, Aethylenthioäthylen, Aethylensulfinyläthylen, Methoxymethylen oder Aethylen- oder Propylendioxymethylen sind.

Besonders bevorzugt unter den Oxetanonen der Formel I, worin Q eine Gruppe $Q^2$ ist, sind diejenigen, worin $R^1$ Hexyl, $R^2$ Undecyl und X' Aethylen, 1-Methoxy-1,2-äthylen oder 1,2-Cyclohexylen ist.

Beispiele von solchen Verbindungen sind folgende:

(S)-1-[[(2S,3S)-3-Hexyl-4-oxo-2-oxetanyl]methyl]dodecyl-(S)-2-isopropylmalonamat,
(S)-1-[[(2S,3S)-3-Hexyl-4-oxo-2-oxetanyl]methyl]dodecyl-(S oder R)-2-carbamoylvalerat,
(all Z,S)-1-[[(2S,3S)-3-Aethyl-4-oxo-2-oxetanyl]methyl]-9,12-octadecadienyl-(S)-2-isopropylmalonamat,
(S)-1-[[(2S,3S oder 2R,3R)-4-Oxo-3-pentylthio-2-oxetanyl]methyl]dodecyl-(S)-2-isopropylmalonamat,
(S)-1-[[(2S,3S) oder 2R,3R)-4-Oxo-3-pentylthio-2-oxetanyl]methyl]dodecyl-[S:R(2:1)]-2-isopropylmalonamat,
(S)-1-[[(2S,3S)-3-Aethyl-4-oxo-2-oxetanyl]methyl]octadecyl-(S oder R)-2-t-butylmalonamat,
(S)-1-[[(2S,3S)-3-Aethyl-4-oxo-2-oxetanyl]methyl]octadecyl-1-carbamoylcydopentancarboxylat,
(S)-1-[[(2S,3S)-3-Hexyl-4-oxo-2-oxetanyl]methyl]dodecyl-(RS)-2-benzylmalonamat,
(S)-1-[[(2S,3S)-3-Hexyl-4-oxo-2-oxetanyl]methyl]dodecyl-3-[(2-carbamoyläthyl)thio]propionat,
5-Oxo-D-prolin-(S)-1-[[(2S,3S)-3-äthyl-4-oxo-2-oxetanyl]methyl]octadecylester,
5-Oxo-L-prolin-(S)-1-[[(2S,3S)-3-äthyl-4-oxo-2-oxetanyl]methyl]octadecylester und insbesondere
(S)1-[[(2S,3S)-3-Hexyl-4-oxo-2-oxetanyl]methyl]dodecyl-(S oder R)-2-isopropylmalonamat,
(S)-1-[[(2S,3S)-3-Hexyl-4-oxo-2-oxetanyl]methyl]dodecyl-(RS)-2-carbamoylvalerat (Epimere 1:1),
(all Z,S)-1-[[(2S,3S)-3-Aethyl-4-oxo-2-oxetanyl]methyl]-9,12-octadecadienyl-(S oder R)-2-isopropylmalonamat,
(S)-1-[[(2S,3S)-3-Hexyl-4-oxo-2-oxetanyl]methyl]dodecyl-(RS)-2-carbamoyl-4-methylvalerat (Epimere 1:1),
(S)-1-[[(2S,3S)-3-Hexyl-4-oxo-2-oxetanyl]methyl]dodecyl-1-carbamoylcyclohexancarboxylat,
(S)-1-[[(2S,3S)-3-Hexyl-4-oxo-2-oxetanyl]methyl]dodecyl-(RS)-2-methyl malonamat (Epimere 1:1),
(S)-1-[[(2S,3S)-3-Hexyl-4-oxo-2-oxetanyl]methyl]dodecyl-(RS)-2-äthylmalonamat (Epimere 1:1),
(S)-1-[[(2S,3S)-3-Hexyl-4-oxo-2-oxetanyl]methyl]dodecyl-(RS)-2-butylmalonamat (Epimere 1:1),
(S)-1-[[(2S,3S)-3-Aethyl-4-oxo-2-oxetanyl]methyl]octadecyl-1-carbamoylcyclohexancarboxylat,
(S)-1[[(2S,3S oder 2R,3R)-4-Oxo-3-pentylthio-2-oxetanyl]methyl]dodecyl-[S:R oder R:S(2:1)]-2-isopropylmalonamat,
(S)-1-[[(2R,3R)-3-Benzyl-4-oxo-2-oxetanyl]methyl]dodecyl-(S oder R)-2-isopropylmalonamat.

Die Oxetanone der Formel I enthalten zumindest drei asymmetrische C-Atome und können somit als optisch aktive Enantiomere, als Gemische davon, z.B. als Racemate oder als Diastereomere vorliegen.

Die Oxetanone der Formel I können in an sich bekannter Weise dadurch hergestellt werden, dass man

a) einen Alkohol der Formel

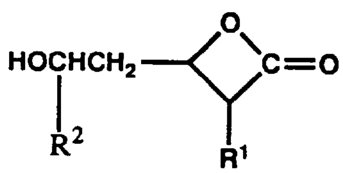

IIa

mit einer Säure der Formel $Q^a$-OH, worin $Q^a$ eine Gruppe der Formel $Q^1$ oder $Q^3$ ist, verestert oder

b) eine Säure der Formel

$$(Q\text{-}O,R^2)CHCH_2CH(OH)CH(R^1)\text{-}COOH \qquad IIb$$

cyclisiert oder

c) in einer Säure der Formel

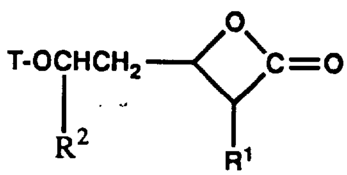

IIc

worin T eine Gruppe der Formel

$$HOCO(X)_n\text{-}CO\text{-} \qquad T^1$$

oder

$$HOCO\text{-}X'\text{-} \qquad T^2,$$

ist, die Carboxygruppe in der Gruppe T in eine Amidgruppe $(R^3,R^4)NCO$-umwandelt und

d) gewünschtenfalls ein Epimerengemisch der Formel I in die einzelnen Epimeren auftrennt.

Die Veresterung a) kann man in Gegenwart von Triphenylphosphin und einem Azodicarbonsäurediester, wie dem Di-t-butyl- oder Diisopropylester, in einem Lösungsmittel, z.B. einem Aether, wie Tetrahydrofuran (THF), bei Raumtemperatur oder unter Abkühlen, z.B. bis auf 0 bis -5°C durchführen.

Die Cyclisierung b) kann man in einem Lösungsmittel, wie Methylenchlorid, Dimethylformamid (DMF) oder Acetonitril mit Molekularsieb z.B. in Gegenwart von 2-(1H-Benzotriazol-1-yl)-1,1,3,3-tetramethyluronium-hexafluorophosphat (HBTU) und von einer Base, wie Triäthylamin, bei Raumtemperatur oder bei einer Temperatur bis zu 50°C durchführen.

Die Amidierung c) kann man mit einer Lösung von Ammoniak oder eines Amins der Formel ($R^3$,$R^4$)NH, z.B. in Acetonitril, in Gegenwart von HBTU bei Raumtemperatur oder bei einer Temperatur bis zu 40°C bewerkstelligen.

Die fakultative Auftrennung eines Epimerengemisches der Formel I kann man z.B. durch Chromatographie über Silicagel mit Aethylacetat/Hexan/Methylenchlorid als Eluierungsmittel durchführen.

Die Alkohole der Formel IIa sind bekannt, z.B. aus der europäischen Patentanmeldung Nr. 0 185 359 A2, oder können in Analogie zu den bekannten Alkoholen der Formel IIa bzw. wie in den Beispielen A bis I, M und O bis T beschrieben hergestellt werden.

Die Ausgangssäuren der Formeln IIb und IIc kann man in an sich bekannter Weise herstellen, z.B. ausgehend von entsprechenden Alkoholen der Formel IIa wie nachstehend in den Beispielen J und K (für die Säuren IIb) bzw. K, L und N (für die Säuren IIc) beschrieben.

Beispiel A

a) Unter Stickstoff werden zu 720 g 30%iger Natriummethylatlösung in 1200 ml Methanol 465 g Acetessigsäuremethylester und dann 458 g Aethylbromid zugegeben. Das Reaktionsgemisch wird anschliessend am Rückfluss gekocht. Nach Abdestillation des Methanols wird der Rückstand auf Eiswasser gegossen. Dann wird mit n-Hexan und Wasser extrahiert. Die organischen Phasen werden vereinigt und getrocknet. Nach Abdampfen des Lösungsmittels und Destillation erhält man 328 g 2-Acetylbuttersäuremethylester, Sdp. 77-79°/15 Torr.

b) Unter Argon werden 144,17 g des Methylesters von a) bei 0-5°C zu einer Suspension von 26,4 g Natriumhydrid in 1250 ml THF gegeben. Nach 1,5 Stunden Rühren bei 0-5°C wird auf -10°C abgekühlt. Bei dieser Temperatur werden 675 ml 1,56M Butyllithium in Hexan zugegeben. Nach 30 Minuten Rühren bei -10°C wird eine Lösung von 149,3 g Stearinsäuremethylester in 250 ml THF zugetropft. Nach 1,5 Stunden Rühren bei -10°C wird die Reaktionslösung unter Argon zu 250 ml 37% Salzsäure und 300 g Eis gegeben. Es wird mit Hexan und Wasser extrahiert. Die vereinigten organischen Phasen werden getrocknet, filtriert und eingedampft.

Der Rückstand wird in 2500 ml THF gelöst, mit 76,1 g 1,8-Diazabicyclo-[5.4.0]undec-7-en(1,5-5) (DBU) versetzt und unter Argon am Rückfluss gekocht. Die abgekühlte Reaktionslösung wird mit 37% Salzsäure und dann mit gesättigter Natriumchloridlösung extrahiert. Die vereinigten organischen Phasen werden getrocknet und eingedampft. Das Produkt wird in Essigsäureäthylester gelöst. Die Lösung wird auf Raumtemperatur abgekühlt und über Nacht bei 25°C gerührt. Das Kristallisat wird abgenutscht, mit Essigsäureäthylester gewaschen und getrocknet. Es resultieren 122,5 g 3-Aethyl-6-heptadecyl-4-hydroxy-2H-pyran-2-on, Smp. 101-102°C.

c) Zu 100 g des Pyrons von b) werden 100 g Raney-Nickel und 2000 ml THF gegeben. Nach 3 Tagen Hydrieren bei 25° wird der Katalysator abgenutscht und mit THF gewaschen. Das Filtrat wird zur Trockene eingedampft. Der Rückstand wird in Essigsäureäthylester gelöst und bei 10° während 17 Stunden gerührt. Das Kristallisat wird abgenutscht, mit -10° kaltem Essigsäureäthylester gewaschen und 17 Stunden bei 40°C getrocknet. Es resultieren 90,54 g rac-(2RS,3RS,5SR)-2-Aethyl-5-heptadecyl-3-hydroxy-δ-valeriolacton, Smp. 101-102°C.

d) Einer Suspension von 191,3 g des δ-Lactons von c) in 1250 ml Toluol werden 138,5 g Benzoesäureanhydrid und anschliessend 2,5 ml Perchlorsäure 70% zugegeben. Nach 2,5 Stunden Rühren wird das Reaktionsgemisch in Toluol mit 1N Natronlauge in 20%-iger Natriumchlorid-Lösung und dann mit gesättigter Natriumchloridlösung extrahiert. Die organischen Phasen werden vereinigt, getrocknet und eingedampft. Es resultieren 243,4 g rac-(2RS,3RS,5SR)-3-Benzoyloxy-2-äthyl -5-heptadecyl-δ-valeriolacton, Smp. 64,5-66°C.

e) Unter Argon werden 243 g des Benzoats von d) in 450 ml Toluol bei 40°C gelöst. Es werden 1000 ml Methanol und danach 2,5 ml konz. Schwefelsäure zugegeben und das Reaktionsgemisch wird während 20 Stunden bei 25°C gerührt. Nach der Neutralisation der Schwefelsäure mit Triäthylamin wird das Lösungsmittel abgedampft. Der Rückstand wird in t-Butylmethyläther gelöst und mit Wasser gewaschen. Die wässrige Phase wird mit t-Butylmethyläther extrahiert und die organischen Phasen werden vereinigt und über Natriumsulfat getrocknet, das Trocknungsmittel wird abgenutscht und mit t-Butylmethyläther gewaschen und anschliessend eingedampft. Es resultieren 257 g rac-(2RS,3RS,5SR)-3-Benzoyloxy-2-äthyl -5-hydroxydocosansäuremethylester.

f) Unter Argon werden 257 g des Hydroxyesters von e) in 1250 ml n-Hexan mit 152 g Benzyl-2,2,2-trichloracetimidat versetzt. Dann werden 3,2 ml Trifluormethansulfonsäure zugegeben. Nach 18 Stunden Rühren wird der Niederschlag abgenutscht und mit n-Hexan gewaschen. Das Filtrat wird mit 5%iger Natriumbicarbonatlösung und Wasser extrahiert. Die vereinigten Hexanphasen werden getrocknet, filtriert und eingeengt. Nach 20 Stunden Rühren bei -20°C wird das Kristallisat abgenutscht, mit n-Hexan gewaschen und verworfen. Das Filtrat wird abgedampft. Es resultieren 239,6 g rac-(2RS,3RS,5SR)-3-Benzoyloxy-5-benzyloxy -2-äthyldocosansäuremethylester.

g) Unter Argon werden 239,6 g des Benzyläthers von f) mit einer Lösung von 140 g Kaliumhydroxid in 1250 ml 95% (v/v) Methanol/Wasser versetzt und während 17 Stunden bei 40°C gerührt. Anschliessend wird bei 40°C eingeengt, die Suspension in t-Butylmethyläther aufgenommen und der Reihe nach mit 10%iger Natriumchloridlösung, 1N Salzsäure und nochmals mit 10%iger Natriumchloridlösung gewaschen. Die organische Phase wird mit Natriumsulfat getrocknet, das Trocknungsmittel abgenuscht und mit t-Butylmethyläther gewaschen. Das Filtrat wird eingedampft. Es resultieren 182,1 g rac-(2RS,3RS,5SR)-5-Benzyloxy-2-äthyl-3-hydroxydocosansäure.

h) Einer Lösung von 182,1 g der β-Hydroxysäure von g) in 1250 ml Essigsäuremethylester werden 33,3 g (S)-(-) -α-Methylbenzylamin zugetropft. Die Lösung wird mit 50 mg Phenäthylaminsalz der (2S,3S,5R)-5-Benzyloxy-2-äthyl-3-hydroxydocosansäure angeimpft und während 20 Stunden stehengelassen. Das Kristallisat wird abgenutscht, mit -20°C kaltem Essigsäuremethylester gewaschen und dann getrocknet. Dieses 1. Kristallisat wird in Essigsäuremethylester heiss gelöst, auf 45°C abgekühlt und mit 50 mg Phenäthylaminsalz der (2S,3S,5R)-5-Benzyloxy-2-äthyl-3-hydroxydocosansäure angeimpft. Die Lösung wird 20 Stunden bei Raumtemperatur stehengelassen. Das Kristallisat wird abgenutscht, mit -20°C kaltem Essigsäuremethylester gewaschen und getrocknet. Die gleiche Arbeitsweise wie mit dem 1. Kristallisat wird mit dem 2. Kristallisat wiederholt. Es resultieren 39,4 g Phenäthylaminsalz der (2S,3S,5R)-5-Benzyloxy-2-äthyl-3-hydroxydocosansäure, Smp. 92-95°C.

i) Es werden 39,4 g des Phenäthylaminsalzes aus h) mit 400 ml t-Butylmethyläther und 80 ml 1N Salzsäure versetzt und unter Rühren gelöst. Die organische Phase wird mit Wasser gewaschen, getrocknet, filtriert und eingeengt. Es resultieren 31,4 g (2S,3S,5R)-5-Benzyloxy-2-äthyl-3-hydroxydocosansäure, Smp. 62-63,5°C.

j) Unter Argon werden einer Lösung von 24,5 g der β-Hydroxysäure von i) in 250 ml Pyridin bei 0°C 17,6 g Benzolsulfochlorid zugetropft. Nach 20 Stunden Rühren bei 0°C werden der Lösung 5 ml Wasser zugetropft. Es wird 1 Stunde bei Raumtemperatur gerührt. Das Pyridin wird abgedampft. Der Kristallbrei wird in t-Butylmethyläther aufgenommen und nacheinander mit 2N Salzsäure, 5%iger Natriumbicarbonatlösung und 10%iger Natriumchloridlösung gewaschen. Die organische Phase wird über Natriumsulfat getrocknet und danach mit Aktivkohle verrührt. Trocknungsmittel und Aktivkohle werden abgenutscht und das Filtrat eingedampft. Es resultieren 23,4 g (3S,4S)-4-[(R)-2-Benzyloxynonadecyl]-3-äthyl-2-oxetanon.

k) Eine Lösung von 23,4 g des Oxetanons von j) in 250 ml THF wird mit 2,3 g Pd/C 10% versetzt. Nach 5 Stunden Hydrierung wird die Hydrierlösung abgenutscht. Nach Waschen mit THF wird das Filtrat eingedampft, der Rückstand in n-Hexan gelöst und mit (3S,4S)-3-Aethyl-4-[(R)-2-hydroxynonadecyl]-2-oxetanon angeimpft. Nach 18 Stunden wird das Kristallisat abgenutscht, mit Hexan gewaschen und getrocknet. Es resultieren 16,1 g (3S,4S)-3-Aethyl-4-[(R)-2-hydroxynonadecyl]-2-oxetanon, Smp. 66,5-68°C, der Ausgangsalkohol von Beispiel 1.

<u>Beispiel B</u>

a) 50 g (R)-3-Hydroxytetradecansäuremethylester, 35 g t-Butyldimethylchlorsilan, 6,1 g 4-Dimethylaminopyridin und 29,4 g Triäthylamin werden in 200 ml Methylenchlorid gelöst und 30 Stunden bei Raumtemperatur sowie 16 Stunden unter Rückfluss gerührt. Hierauf werden weitere 2 g t-Butyldimethylchlorsilan zugesetzt. Nach weiteren 24 Stunden unter Rückfluss wird das ausgefallene Triäthylaminhydrochlorid-Salz abfiltriert, mit Aether gewaschen und das Filtrat eingeengt. Der Rückstand wird in Aether gelöst und nacheinander mit Wasser, 0,5M Zitronensäure, wiederum mit Wasser und gesättigter. Natriumchloridlösung gewaschen, getrocknet, eingeengt und anschliessend bei 50°C im Hochvakuum während 5 Stunden von flüchtigem Material befreit. Man erhält 71,8 g (R)-3-(t-Butyldimethylsiloxy) tetradecansäuremethylester, IR ($cm^{-1}$): 1745, 1254, 895, 776.

b) 18,63 g des Produkts von a), gelöst in 100 ml Aether werden bei einer Temperatur von -70°C bis -75°C mit 65 ml 1M Diisobutylaluminiumhydridlösung in Hexan versetzt und dann 1 Stunde bei dieser Temperatur gerührt. Hierauf werden 2,5 ml Isopropanol, 10 ml Wasser und 50 ml 0,5M Zitronensäurelösung bei max. 10°C zugetropft. Die

Aetherphase wird abgetrennt, die Wasserphase mit Aether extrahiert, die vereinigten Aetherphasen werden mit Sole gewaschen, getrocknet und eingeengt. Der Rückstand wird an Kieselgel mit Pentan/Aether (5:1) chromatographiert und man erhält 14,47 g (R)-3-(t-Butyldimethylsiloxy)tetradecanal, IR ($cm^{-1}$): 1728, 1254, 836, 775.

c) Eine Lösung von 2,55 ml Diisopropylamin in 45 ml THF wird bei 0°C mit 22,5 ml einer Lösung von 1,6M n-Butyllithium in Hexan versetzt und nach 15 Minuten Rühren auf -75°C gekühlt. Dann wird eine Lösung von 2,92 g Pentylthioessigsäure in 9 ml THF zugetropft. Nach 10 Minuten Rühren lässt man das Reaktionsgemisch auf Raumtemperatur aufwärmen, rührt 5 Minuten und kühlt erneut auf -75°C. Bei dieser Temperatur wird eine Lösung von 2,4 g des Aldehyds von b) in 9 ml THF zugetropft. Nach 20 Minuten Rühren wird das Reaktionsgemisch auf gesättigte Ammoniumchloridlösung gegossen und mit Hexan extrahiert. Die Hexanphase wird getrocknet und eingeengt.

Man erhält 3,89 g (2R/S,3R/S,5R)-5-(t-Butyldimethylsiloxy) -3-hydroxy-2-pentylthiohexadecansäure als Gemisch von 4 Diastereomeren.

d) Eine Lösung von 3,89 g des Produkts von c), 3,18 g 2-(1H-Benzotriazol-1-yl)-1,1,3,3 -tetramethyluronium-hexafluorphosphat (HBTU), 2 g 4Å Molekularsieb und 3 ml Triäthylamin in einem Gemisch von 130 ml Methylenchlorid und 6 ml DMF wird 2 Stunden gerührt. Hierauf wird filtriert, das Filtrat eingeengt, der Rückstand in Wasser/Methanol (3:7) gelöst und mit Hexan extrahiert. Die Hexanphase wird getrocknet und eingeengt, Man erhält 3,57 g (3R/S,4R/S)-4-[(R)-2-(t-Butyldimethylsiloxy]tridecyl]-3-pentylthio-2-oxetanon als Gemisch von 4 Diastereomeren.

e) Eine Lösung von 4,59 g des Produkts von d) in 200 ml Acetonitril wird mit 15 ml 40%iger Fluorwasserstoffsäure versetzt und 18 Stunden gerührt. Hierauf wird Natriumbicarbonatlösung zugesetzt, dann mit Hexan extrahiert und die Hexanphase getrocknet und eingeengt. Der Rückstand wird an Kieselgel mit 1-5% Aether in Methylenchlorid chromatographiert. Man erhält 699,9 mg 3R,4R(oder 3S,4S)-4-[(R)-2-Hydroxytridecyl]-3-pentylthio-2-oxetanon, Smp. 43°C und 691,2 mg 3S,4S(oder 3R,4R)-4-[(R)-2-Hydroxytridecyl]-3-pentylthio-2-oxetanon, Smp. 71°C, die Ausgangsalkohole der Beispiele 5 und 6.

Beispiel C

a) 18 ml einer 1M Lösung von Lithium-bis(trimethylsilyl)amid in THF werden unter Argon bei -75°C mit 1,8 ml Essigsäureäthylester versetzt, 30 Minuten bei dieser Temperatur gerührt und anschliessend mit 4,8 g (R)-3-Benzyloxytetradecanal in 15 ml THF versetzt und eine halbe Stunde bei -78°C gerührt. Zum Reaktionsgemisch wird eine Lösung von 3,8 ml konz. Salzsäure in 6 ml Wasser zugetropft. Die erhaltene Lösung wird mit Essigsäureäthylester extrahiert, die vereinigten organischen Phasen werden mit 10%igem Natriumbicarbonat und Wasser gewaschen, getrocknet, filtriert und eingeengt. Man erhält Aethyl (3R, 5R und 3S,5R)-5-benzyloxy-3-hydroxyhexadecanoat (1:1).

b) Eine Lösung von 4 ml Diisopropylamin in 12,5 ml THF wird unter Argon bei 0°C mit 17 ml einer 1,6M Lösung von n-BuLi in n-Hexan versetzt. Nach 15 Minuten Rühren werden 5 g des Produkts von a) in 2,5 ml THF bei -50°C zugetropft. Nach 10 Minuten bei -10°C wird die Temperatur auf -50°C gesenkt und nach Zutropfen einer Lösung von 3,18 g Benzylbromid in 3,1 ml Hexamethylphosphorsäuretriamid bei -50°C 15 Minuten gerührt. Danach wird das Kühlbad entfernt und das Reaktionsgemisch bei Raumtemperatur während 3 Stunden gerührt. Man kühlt das Reaktionsgemisch auf 0°C und gibt 50 ml gesättigte Natriumchloridlösung zu, extrahiert mit t-Butylmethyläther, trocknet die Extrakte, filtriert und dampft das Lösungsmittel ab. Der Rückstand wird an Kieselgel mit n-Hexan/ Essigester (4:1) chromatographiert. Der Rückstand wird getrocknet. Man erhält Aethyl-(2R,3R,5R und 2S,3S,5R)-5-benzyloxy-2-benzyl-3-hydroxyhexadecanoat als 1:1 threo-Diastereomere.

c) Eine Lösung von 3,1 g des Produkts von b) und 26 ml 2,5N Natronlauge in 37,2 ml Aethanol wird während 50 Minuten am Rückfluss erhitzt und anschliessend bei Raumtemperatur mit 26 ml 2,5N Salzsäure neutralisiert. Der Aethanol wird abdestilliert, worauf der Rückstand mit t-Butylmethyläther und Wasser extrahiert wird. Die vereinigten organischen Phasen werden getrocknet und eingeengt. Eine Lösung von 3 g des Rückstands in 109 ml Methylenchlorid wird unter Argon gerührt und mit 2,59 g HBTU und 2,74 g Molekularsieb versetzt. Anschliessend werden 5,5 ml DMF und 2,8 ml Triäthylamin zugegeben und das Reaktionsgemisch wird 1 Stunde gerührt, filtriert und eingeengt. Der Rückstand wird in n-Hexan aufgenommen, darauf die Lösung mit Wasser extrahiert, getrocknet und im Vakuum eingeengt. Chromatographie an Silicagel mit Methylenchlorid ergibt ein 1. trans-Diastereomer, (3S,4S oder 3R,4R)-3-Benzyl-4-[(R)-2-benzyloxytridecyl]-2-oxetanon, Rf-Wert: 0,45 (Dünnschichtchromatographie über Kieselgel 5-40 μ mit Methylenchlorid) und ein 2. trans-Diastereomer, (3R,4R oder 3S,4S)-3-Benzyl-4-[(R)-2-benzyloxytridecyl]-2-oxetanon, Rf-Wert: 0,50 (Dünnschichtchromatographie über Kieselgel 5-40 µ mit Methylenchlorid).

d) Eine Lösung von 646 mg des 2. trans-Diastereomers aus c) in 65 ml THF wird in Gegenwart von 646 mg Pd/C 10% während 1 Stunde hydriert. Das Reaktionsgemisch wird filtriert und eingeengt. Man erhält ein trans-Diastereomer: (3R,4R)-3-Benzyl-4-[(R)-2-hydroxytridecyl]-2-oxetanon, der Ausgangsalkohol in Beispiel 7.

e) Wie unter d) beschrieben erhält man aus dem 1. trans-Diastereomeren aus c) das trans-Diastereomere: (3S,4S) -3-Benzyl-4-[(R)-2-hydroxytridecyl]-2-oxetanon, der Ausgangsalkohol in Beispiel 8.

Beispiel D

Eine Lösung von 3,0 ml Diisopropylamin in 50 ml THF wird bei 0°C mit 12,0 ml einer Lösung von 1,6M n-Butyllithium in Hexan versetzt und nach Rühren auf -75°C gekühlt. Dann wird eine Lösung von 1,26 g Z-Glycin in 10 ml THF zugetropft. Dann lässt man das Reaktionsgemisch auf Raumtemperatur aufwärmen und kühlt erneut auf -75°C. Nun werden 0,70 g (R)-3-(t-Butyldimethylsiloxy)tetradecanal in 5 ml THF bei -75°C zugetropft. Das Reaktionsgemisch wirdl Stunde bei -75°C, 1/2 Stunde bei -40° bis -50°C gerührt, dann auf 5°C aufgewärmt, erneut auf -75°C gekühlt und auf verdünnte Kaliumhydrogensulfatlösung gegossen und mit Aether extrahiert. Die Aetherphase wird getrocknet, eingeengt und mit Methylenchlorid/Methanol an Kieselgel chromatographiert. Es resultieren 540 mg (2R/S,3R/S,5R)-2-[1-(Benzyloxy) formamido]-5-(t-Butyldimethylsiloxy)-3-hydroxyhexadecansäure als Gemisch von 4 Diastereomeren.

In Analogie zu B)d) wird aus obigem Produkt Benzyl-4-[(R)-2-(t-butyldimethylsiloxy)tridecyl]-2-oxo-3-oxetancarbamat als 1:1-Gemisch der beiden trans-Diastereomeren β-Lactone erhalten, MS: 476 ($M^{+\bullet}$-$C_4H_9^{\bullet}$).

In Analogie zu B)e) wird aus obigem Gemisch (3S,4S oder 3R,4R)-Benzyl-4-[(R)-2-hydroxytridecyl]-2-oxo-3-oxetancarbamat, der Ausgangsalkohol in Beispiel 15, Smp. 122-124°C, und (3R,4R oder 3S,4S)-Benzyl-4-[(R)-2-hydroxytridecyl]-2-oxo-3-oxetancarbamat, Smp. 98-99°C, erhalten.

Beispiel E

In Analogie zu Beispiel B erhält man aus Thiophenoxyessigsäure und (R)-3-[(1,1-Dimethyläthyl)dimethylsilyloxy] tetradecanal über

a) (2R/S,3R/S,5R)-5-(t-Butyldimethylsiloxy)-3-hydroxy-2-(phenylthio)-hexadecansäure (Gemisch von 4 Diastereomeren) und

b) (3R/S,4R/S)-4-[(R)-2-(t-Butyldimethylsiloxy)tridecyl]-3-(phenylthio)-2-oxetanon (Gemisch von 4 Diastereomeren), IR ($cm^{-1}$): 2927, 2855, 1833, 1254,

das (3S,4S)-4-[(R)-2-Hydroxytridecyl]-3-(phenylthio)-2-oxetanon, Smp. 79°C (Aether) und (3R,4R)-4-[(R) -2-Hydroxytridecyl]-3-(phenylthio)-2-oxetanon, Smp. 47°C (Aether) die Ausgangsalkohole des Beispiels 16.

Beispiel F

a) Einer Lösung von 117 g Meldrumsäure und 131 ml Pyridin in 1,5 l Methylenchlorid werden 270 ml Stearinsäurechlorid bei maximal 15°C zugetropft. Nach Rühren wird das Reaktionsgemisch mit 4N Salzsäure gewaschen, die wässrige Phase mit Methylenchlorid nachextrahiert, die Methylenchloridphase getrocknet und eingeengt. Der Rückstand wird in Methanol aufgenommen und unter Rückfluss gerührt. Nach dem Abkühlen werden die ausgefallenen Kristalle abfiltriert, in Methylenchlorid gelöst und an Kieselgel mit Methylenchlorid chromatographiert. Man erhält 175 g Methyl-3-oxoeicosanoat, Smp. 52-54°C.

b) Einer Lösung von 9,1 mg [(R)-2,2'-Bis(diphenylphosphino)-6,6'-dimethylbiphenyl]ruthenium-diacetat in 20 ml Methylenchlorid werden 1,84 mg Acetylchlorid in 1,84 ml Methanol zugegeben. Die erhaltene Lösung wird zusammen mit 39,8 g des Ketoesters von a) und 170 ml Methanol bei 35 bar Wasserstoff und 60°C hydriert. Nach Zusatz von Methylenchlorid wird zur Trockene eingedampft. Chromatographie an Kieselgel mit Aether und Umkristallisation aus n-Hexan liefert 35,7 g (R)-3-Hydroxyeicosansäuremethylester, Smp. 64-64,5°C.

c) Analog Beispiel B erhält man aus dem Produkt von b) via

Methyl-(R)-3-(t-butyldimethylsiloxy)eicosanoat, IR ($cm^{-1}$): 1745, 1255, 836,

(R)-3-(t-Butyldimethylsiloxy)eicosanal, IR ($cm^{-1}$): 1728, 1463, 1255, 1104, 836, 775,

(2R/S,3R/S,5R)-5-(t-Butyldimethylsiloxy)-3-hydroxy-2-(methylthio)-docosansäure (Gemisch von 4 Diastereomeren) MS: 533 $(M+H)^+$,

4-[(R)-2-(t-Butyldimethylsiloxy)nonadecyl]-3-(methylthio)-2-oxetanon, (1:1-Gemisch von zwei trans-Diastereomeren), IR ($cm^{-1}$): 1834, 1463, 1256, 1106, 836 und

4-[(R)-2-(t-Butyldimethylsiloxy)nonadecyl]-3-(methylthio)-2-oxetanon (1:1-Gemisch von zwei cis-Diastereomeren), IR ($cm^{-1}$): 1834, 1463, 1256, 1106, 1066, 836,

die folgenden Ausgangsalkohole von Beispiel 17:

(3S,4R oder 3R,4S)-4-[(R)-2-Hydroxynonadecyl]-3-(methylthio)-2-oxetanon, Smp. 65°C (aus Methylenchlorid)
(3R,4S oder 3S,4R)-4-[(R)-2-Hydroxynonadecyl]-3-(methylthio)-2-oxetanon, Smp. 67°C (aus Methylenchlorid)
(3R,4R oder 3S,4S)-4-[(R)-2-Hydroxynonadecyl]-3-(methylthio)-2-oxetanon, Smp. 71°C (aus Aether)
(3S,4S oder 3R,4R)-4-[(R)-2-Hydroxynonadecyl]-3-(methylthio)-2-oxetanon, Smp. 80°C (aus Aether).

Beispiel G

Analog Beispiel B erhält man aus (Benzylthio)-essigsäure und (R)-3-[(1,1-Dimethyläthyl)dimethylsilyloxy]tetradecanal via

(2R/S,3R/S,5R)-2-(Benzylthio)-5-[(1,1-dimethyläthyl)dimethylsilyloxy]-3-hydroxyhexadecansäure (Gemisch von 4 Diastereomeren) und

(3R/S,4R/S)-3-(Benzylthio)-4-[(R)-2-(t-butyldimethylsiloxy)tridecyl]-2-oxetanon (Gemisch von 4 Diastereomeren), MS: 506 $(M^+)$

die folgenden Ausgangsalkohole der Beispiele 19 und 20:

das (3S,4S oder 3R,4R)-3-(Benzylthio)-4-[(R)-2-hydroxytridecyl]-2-oxetanon, Smp. 65°C (Aether),

(3R,4R oder 3S,4S)-3-(Benzylthio)-4-[(R)-2-hydroxytridecyl]-2-oxetanon, MS: 374 $(M^{+\bullet}-H_2O)$ und

das (3R,4S und 3S,4R)-3-(Benzylthio)-4-[(R)-2-hydroxytridecyl]-2-oxetanon (1:1 Diast.), MS: 374 $(M^{+\bullet}-H_2O)$.

Beispiel H

a) 104 g Mutterlauge der 1. Kristallisation im Beispiel Ah) werden in Wasser und Methylenchlorid gelöst. Unter Eiskühlung wird durch Zusatz von konz. HCl auf pH 1 angesäuert, die Methylenchloridphase abgetrennt, die Wasserphase mit Methylenchlorid extrahiert, die Methylenchloridphase mit Wasser gewaschen, getrocknet und eingeengt. Es resultieren 86,7 g angereicherte (2R,3R,5S)-5-Benzyloxy-2-äthyl-3-hydroxydocosansäure, welche in 500 ml Aethylacetat gelöst und unter Kühlung mit 20,6 g (R)-(+)-α-Methylbenzylamin versetzt werden. Nach Zusatz von Aethylacetat wird zum Rückfluss erwärmt, filtriert und auskristallisiert. Die erhaltenen Kristalle werden aus Aethyl- und Methylacetat umkristallisiert. Es resultieren 70,0 g Phenäthylaminsalz der (2R,3R,5S)-5-Benzyloxy-2-äthyl-3-hydroxydocosansäure, Smp. 88-91°C.

b) Analog Beispiel Ai)j)k) wird aus obigem Salz via

(2R,3R,5S)-5-Benyzloxy-2-äthyl-3-hydroxydocosansäure, Smp. 61,5-63°C und

(3R,4R)-4-[(S)-2-Benzyloxynonadecyl]-3-äthyl-2-oxetanon, Smp. 38-40°C

das (3R,4R)-3-Aethyl-4-[(S)-2-hydroxynonadecyl]-2-oxetanon, Smp. 66-68°C erhalten.

c) Eine Lösung von 14,2 g des erhaltenen Produktes und 8,65 g Triphenylphosphin in 250 ml THF wird bei +5°C mit 1,19 ml Ameisensäure und dann mit einer Lösung von 5,12 g Diäthylazodicarboxylat in 20 ml THF versetzt. Dann wird erneut mit 0,4 ml Ameisensäure, 2,9 g Triphenylphosphin und 1,7 ml Diäthylazodicarboxylat versetzt. Das Reaktionsgemisch wird eingeengt und der Rückstand an Kieselgel mit Hexan/Aethylacetat chromatographiert; dabei resultieren 13,1 g (R)-1-[[(2R,3R)-3-Aethyl-4-oxo-2-oxetanyl]methyl]octadecyl-formiat.

Das erhaltene Produkt wird in 150 ml Methanol gelöst und bei 15°C mit 0,114 g p-Toluolsulfonsäuremonohydrat versetzt. Nach Rühren wird das Reaktionsgemisch eingeengt, der Rückstand zwischen Methylenchlorid und wässrigem Natriumbicarbonat verteilt und mit Methylenchlorid extrahiert. Die Methylenchloridphase wird getrocknet, eingeengt und der Rückstand aus Essigester umkristallisiert. Man erhält 9,5 g (3R,4R)-3-Aethyl-4-[(R)-2-hydroxynonadecyl]-2-oxetanon, Smp. 80-82°C, der Ausgangsalkohol im Beispiel 21.

Beispiel I

a) Einer Lösung von 42,5 ml Diisopropylamin in 500 ml THF werden bei -20°C 187,5 ml n-Butyllithiumlösung (1,6M in Hexan) zugetropft. Nach Rühren wird die Lösung bei max. -65°C zu einer Suspension von 39,9 g (S)-(-)-2-Hydroxy-1,2,2-triphenyläthylacetat in 600 ml THF zugetropft. Dann wird das Reaktionsgemisch auf 0°C aufgewärmt, gerührt, auf -70°C gekühlt und mit einer Lösung von 51,2 g (R)-3-[(t-Butyl)dimethylsilyloxy]eicosanal in 400 ml THF versetzt. Nach Rühren werden bei -70°C 500 ml gesättigte Ammoniumchloridlösung zugetropft, dann auf Raumtemperatur aufgewärmt und gerührt. Das Reaktionsgemisch wird eingeengt, zwischen Wasser und Aether verteilt und mit Aether extrahiert, die Aetherphase mit Wasser gewaschen, eingeengt, in 1 l Methylenchlorid aufgenommen, getrocknet und eingeengt. Es resultieren 91,9 g (S)-2-Hydroxy-1,2,2-triphenyläthyl-[3R:3S(4:1),5R]-5-(t-butyldimethylsiloxy)-3-hydroxydocosanoat, IR ($cm^{-1}$): 3525, 1719, 1448, 1250, 1159, 838, 697.

b) Eine Lösung von 90,8 g des oben erhaltenen Produktes in 1 l Methanol wird mit 22,15 ml 5,4M Natriummethylat in Methanol versetzt. Nach Rühren wird die Lösung eingeengt, der Rückstand zwischen Aether und gesättigter Ammoniumchloridlösung verteilt und mit Aether extrahiert. Die Aetherphase wird getrocknet, eingeengt und mit Hexan/Essigester an Kieselgel chromatographiert. Dabei resultieren 42,7 g Methyl-(3R,5R)-5-(t-butyldimethylsiloxy)-3-hydroxydocosanoat, IR ($cm^{-1}$): 3521, 3468, 1738, 1254, 1168, 1137, 1105.

c) Analog Beispiel Be) und Cb),c) wird letztere Verbindung via Methyl-(2R,3R,5R)-5-(t-butyldimethylsiloxy)-3-hydroxy-2-methyldocosanoat, IR ($cm^{-1}$): 3522, 1739, 1464, 1254, 1066 und (3R,4R)-4-[(R)-2-(t-butyldimethylsiloxy)nonodecyl]-3-methyl-2-oxetanon, IR ($cm^{-1}$): 1830, 1464, 1254, 1129, 1071 in das (3R,4R)-4-[(R)-2-Hydroxynonadecyl]-3-methyl-2-oxetanon, Smp. 82,5-84°C (aus EtOAc/Hexan) dem Ausgangsalkohol in Beispiel 22 umgewandelt.

Beispiel J

a) 1,1 g (3S,4S)-3-Hexyl-4-[(R)-2-hydroxytridecyl]-2-oxetanon, 1,6 g Triphenylphosphin, 0,825 g Salicylamid und 3 g Molekularsieb (4Å) werden mit 20 ml THF versetzt und auf 0°C gekühlt. Hierauf werden 1,4 g Azodicarbonsäuredi-t-butylester hinzugefügt. Nach Aufwärmen auf Raumtemperatur und Rühren wird das Reaktionsgemisch eingeengt und der Rückstand zwischen Methanol/ Wasser (70:30) und Hexan verteilt und mit Hexan extrahiert. Die Hexanphase wird getrocknet, eingeengt und der Rückstand mit Hexan/Essigester(4:1) an Kieselgel chromatographiert. Dabei resultieren 0,727 g o-[[(S)-1-[[(2S,3S)-3-Hexyl-4-oxo-2-oxetanyl]methyl]dodecyl]oxy]benzamid, MS: 474 $(M+H)^+$.

b) 972 mg des oben erhaltenen Produktes werden in 12 ml Methanol gelöst und mit 0,2 g Kaliumcarbonat versetzt. Nach Rühren wird das Reaktionsgemisch eingeengt und der Rückstand zwischen Methanol/Wasser (7:3) und Hexan verteilt und mit Hexan extrahiert. Die Hexanphase wird getrocknet und eingeengt. Dabei resultieren 854 mg Methyl-(2S,3S,5S)-5-(o-carbamoylphenoxy)-2-hexyl-3-hydroxyhexadecanoat, MS: 369 ($M^{+\bullet}$-(o-Carbamoylphenoxy).

c) 850 mg des oben erhaltenen Produktes werden in 12 ml Methanol/Wasser (98:2) gelöst, mit 800 mg 5 proz. Rhodium auf Aluminiumoxid versetzt und bei 100°C und 100 bar Wasserstoff hydriert. Das Reaktionsgemisch wird filtriert, eingeengt und an Kieselgel mit Hexan/Essigester (1:1) chomatographiert. Dabei erhält man 213 mg Methyl-(2S,3S,5S)-5-[[(cis)-2-carbamoylcydohexyl]oxy]-2-hexyl-3-hydroxyhexadecanoat (1. Diast.), MS: 367 [M+•-($H_2NCOC_6H_{10}$•+$H_2O$)]; 204 mg Mischfraktion und 142 mg Methyl-(2S,3S,5S)-5-[[(cis)-2-carbamoylcyclohexyl]oxy]-2-hexyl-3-hydroxyhexadecanoat (2. Diast.), MS: 367 [M+•-($H_2NCOC_6H_{10}$•+$H_2O$)].

d) 210 mg des oben erhaltenen 1. Diastereomeren werden in 10 ml Aceton gelöst, mit 3 ml 1N Kaliumhydroxid versetzt. Nach Rühren wird der Reaktionsansatz auf Kaliumhydrogensulfatlösung gegossen und mit Aether extrahiert. Die Aetherphase wird getrocknet und eingedampft. Man erhält 277 mg (2S,3S,5S)-5-[[(cis)-2-Carbamoylcydohexyl]oxyl]-2-hexyl-3-hydroxyhexadecansäure (1. Diast.), die Ausgangssäure in Beispiel 23a).

e) Wie in d) beschrieben wird aus dem 2. Diastereomer von c) die (2S,3S,5S)-5-[[(cis)-2-Carbamoylcyclohexyl]oxy]-2-hexyl-3-hydroxyhexadecansäure (2. Diast.), die Ausgangssäure in Beispiel 23b) erhalten.

Beispiel K

a) Analog Beispiel Hc) erhält man aus (3S,4S)-3-Hexyl-4-[(R)-2-hydroxytridecyl]-2-oxetanon und Ameisensäure via (S)-1-[[(2S,3S)-3-Hexyl-4-oxo-2-oxetanyl]methyl]dodecylformiat, IR ($cm^{-1}$): 1826, 1725, 1177, 1122, das (3S,4S)-3-Hexyl-4-[(S)-2-hydroxytridecyl]-2-oxetanon, Smp. 63-64°C (aus Hexan).

b) 1,8 g des oben hergestellten Hydroxy-β-lactons, 1,3 g Pyridinium-p-toluolsulfonat und 2 g Molekularsieb (4Å) werden in 10 ml 3,3-Dimethoxypropionsäuremethylester bei 100°C unter Argon gerührt, dann das Reaktionsgemisch abfiltriert, der Rückstand eingeengt und mit Aether/Methylenchlorid an Kieselgel chromatographiert. Dabei resultieren

1. 213 mg Methyl-(E)-3-[[(S)-1-[[(2S,3S)-3-hexyl-4-oxo-2-oxetanyl]methyl]-dodecyl]oxy]acrylat, IR ($cm^{-1}$): 1827, 1714, 1643, 1622, 1192, sowie

2. 826 mg Methyl-(R/S)-3-[[(S)-1-[[(2S,3S)-3-hexyl-4-oxo-2-oxetanyl]methyl]dodecyl]oxy]-3-methoxypropionat (1:1 Epimerengemisch), IR ($cm^{-1}$): 1824, 1743, 1438, 1117.

c) 235 mg des Produkts von b)2. werden in 25 ml 0,02N NaOH suspendiert und das Reaktionsgemisch mit Aceton verdünnt. Nach 24 Stunden Rühren wird mit 5 proz. Kaliumhydrogensulfatlösung angesäuert und mit Aether extrahiert. Die Aetherphase wird getrocknet, eingeengt und der Rückstand an Kieselgel mit Methylenchlorid/Methanol chromatographiert. Dabei erhält man 60 mg (2S,3S,5S)-2-Hexyl-3-hydroxy-5-[(R/S)-1-methoxy-2-(methoxycarbonyl)äthoxy]hexadecansäure, MS: 337 (M+•-($H_2O$+•O-($CH_3O$)-$CH_2$-$COOCH_3$).

d) Eine Lösung von 58 mg der obigen Verbindung in 4 ml kondensiertem Ammoniak wird im Autoklaven bei 50°C erwärmt. Anschliessend lässt man das Ammoniakgas entweichen, versetzt dann mit Kaliumhydrogensulfatlösung und extrahiert mit Methylenchlorid. Die Methylenchloridphase wird getrocknet und eingeengt. Es resultieren 42,5 mg (2S,3S,5S)-5-[(R/S)-2-Carbamoyl-1-methoxyäthoxy]-2-hexyl-3-hydroxyhexadecansäure, Ausgangssäure von Beispiel 24.

Beispiel L

Eine Lösung von 200 mg des Produkts von Beispiel Kb)1. in 10 ml THF wird mit 200 mg Pd/C (10%) hydriert. Dann wird filtriert, das Filtrat eingeengt und der Rückstand an Kieselgel mit 1% Aether in Methylenchlorid chromatographiert. Es resultieren 99 mg Methyl-3-[[(S)-1-[[(2S,3S)-3-hexyl-4-oxo-2-oxetanyl]methyl]dodecyl]oxy]propionat, MS: 285 (M+•-($C_{11}H_{23}$•)).

Eine Suspension von 544 mg dieser Verbindung in 49 ml 0,02N Natriumhydroxid wird mit Acetonitril versetzt. Die entstandene Lösung wird mit wässrigem Kaliumhydrogensulfat angesäuert, das Reaktionsgemisch mit Aether extrahiert und die Aetherphase getrocknet und eingeengt. Chromatographie an Kieselgel mit 2% Aether in Methylenchlorid und dann 5% Methanol in Methylenchlorid liefert 43,7 mg 3-[[(S)-1-[[(2S,3S)-3-hexyl-4-oxo-2-oxetanyl]methyl]dodecyl]oxy]propionsäure, IR ($cm^{-1}$): 1823, 1715, 1466, 1105, Ausgangssäure in Beispiel 25.

Beispiel M

Analog Bespiel Cb) und c) wird Methyl-(3R,5R)-5-(t-Butyldimethylsiloxy)-3-hydroxydocosanoat (Beispiel Ib) mit Proparylbromid zu Methyl-(2R,3R,5R)-5-(t-butyldimethylsiloxy)-3-hydroxy-2-(2-propinyl)docosanoat, IR ($cm^{-1}$): 3310, 2120, 1740, 1255, umgesetzt und letzteres zu (2R,3R,5R)-5-(t-Butyldimethylsiloxy)-3-hydroxy-2-(2-propinyl)docosansäure, IR ($cm^{-1}$): 3315, 2120, 1715, 1255 verseift und zu (3R,4R)-4-[(R)-2-(t-Butyldimethylsiloxy)nonadecyl]-3-(2-propinyl)-2-oxetanon, IR ($cm^{-1}$): 3315, 2130, 1830, 1255 cyclisiert.

Nach Abspaltung der Schutzgruppe analog zu Beispiel Be) erhält man das (3R,4R)-4-[(R)-2-Hydroxynonadecyl]-3-(2-propinyl)-2-oxetanon, Smp. 62-63°C (aus Essigester), der Ausgangsalkohol in Beispiel 60.

Beispiel N

Analog Beispiel 1 erhält man aus (3S,4S)-3-Hexyl-4-[(R)-2-hydroxytridecyl]-2-oxetanon und Malonsäuremonobenzylester das Benzyl-(S)-1-[[(2S,3S)-3-hexyl-4-oxo-2-oxetanyl]methyl]dodecylmalonat, IR ($cm^{-1}$): 1824, 1734, 1149, 1125.

Eine Lösung von 430 mg dieses Produktes in 15 ml THF wird mit 100 mg Pd/C versetzt und dann hydriert. Das Reaktionsgemisch wird filtriert und das Filtrat eingeengt. Es resultieren 361 mg (S)-1-[[(2S,3S)-3-Hexyl-4-oxo-2-oxetanyl]-methyl]dodecyl-hydrogenmalonat, IR ($cm^{-1}$): 1824, 1745, die Ausgangssäure des Beispiels 26.

Beispiel O

Eine Lösung von 1,96 g des Alkoholprodukts von Beispiel M in 50 ml Essigester wird mit 0,25 g 10 proz. Pd/C hydriert, dann das Reaktionsgemisch filtriert und der Rückstand an Kieselgel mit Essigester/Hexan chromatographiert. Dabei erhält man 1,44 g (3R,4R)-4-[(R)-2-Hydroxynonadecyl]-3-propyl-2-oxetanon, Smp. 84-85°C (aus Essigester/Hexan), der Ausgangsalkohol im Beispiel 61.

Beispiel P

a) Aus (R)-3-(t-Butyldimethylsiloxy)tetradecanal (Beispiel Bb) erhält man analog Beispiel Ca)b)c) via Aethyl-(3R und 3S,5R)-5-(t-butyldimethylsiloxy)-3-hydroxyhexadecanoat (Epimerengemisch),

Aethyl-(R und S)-2-[(1R und 1S,3R)-3-(t-butyldimethylsiloxy)-1-hydroxytetradecyl]-5-methyl-4-hexenoat (1:1 threo-Diastereomere)

das (3R,4R und 3S,4S)-4-[(R)-2-(t-Butyldimethylsiloxy)tridecyl]-3-(3-methyl-2-butenyl)-2-oxetanon (1:1 trans-Diastereomere).

b) Eine Lösung von 1,87 g des Produktes von a) in 50 ml Acetonitril wird mit 6,2 ml 40%iger Fluorwasserstoffsäure versetzt. Nach Rühren wird Natriumbicarbonatlösung zugesetzt, dann mit Methylenchlorid extrahiert und die Methylenchloridphase getrocknet und eingeengt. Der Rückstand wird an Kieselgel mit 1 Essigester/4,5 Methylenchlorid/4,5 n-Hexan chromatographiert. Die Chromatographie ergibt die Ausgangsalkohole zu den Beispielen 62-65:

ein 1. trans-Diastereomer, (3S,4S)-4-[(R)-2-Hydroxytridecyl]-3-(3-methyl-2-butenyl)-2-oxetanon, Rf-Wert: 0,31 und

ein 2. trans-Diastereomer, (3R,4R)-4-[(R)-2-Hydroxytridecyl]-3-(3-methyl-2-butenyl)-2-oxetanon, Rf-Wert: 0,26 (Dünnschichtchromatographie über Kieselgel 5-40 $\mu$ mit 1 Essigester/4,5 Methylenchlorid/4,5 n-Hexan).

Beispiel Q

Aus Aethyl-(3R,5R und 3S,5R)-5-benzyloxy-3-hydroxyhexadecanoat (1:1) (Beispiel Ca) erhält man analog Beispiel Cb) bis e) via Aethyl-(2R,3R,5R und 2S,3S,5R)-5-benzyl-2-(5-chlorpentyl)-3-hydroxyhexadecanoat (threo-Diastereomere),

ein 1. trans-Diastereomer, (3S,4S oder 3R,4R)-4-[(R)-2-(Benzyloxy)tridecyl]-3-(5-chlorpentyl)-2-oxetanon, Rf-Wert: 0,47,

und ein 2. trans-Diastereomer, (3R,4R oder 3S,4S)-4-[(R)-2-(Benzyloxy)tridecyl]-3-(5-chlorpentyl)-2-oxetanon, Rf-Wert: 0,28 (Dünnschichtchromatographie über Kieselgel 5-40 $\mu$ mit Methylenchlorid), die Ausgangsalkohole zu

den Beispielen 66-69:

(3R,4R oder 3S,4S)-3-(5-Chlorpentyl)-4-[(R)-2-hydroxytridecyl]-2-oxetanon, und

(3S,4S oder 3R,4R)-3-(5-Chlorpentyl)-4-[(R)-2-hydroxytridecyl]-2-oxetanon.

Beispiel R

Aus (R)-3-(t-Butyldimethylsiloxy)tetradecanal (Beispiel Bb) erhält man analog Beispiel Cb) und c) via Aethyl-(R und S,E)-2-[(1R und 1S,3R)-3-(t-butyldimethylsiloxy)tetradecyl]-4-hexenoat (1,1 threo-Diastereomere) und

(3R,4R und 3S,4S)-3-[(E)-2-Butenyl]-4-[(R)-2-(t-butyldimethylsiloxy)- tridecyl]-2-oxetanon (1:1 trans-Diastereomere) die Ausgangsalkohole zu den Beispielen 70-73:

ein 1. trans-Diastereomer, (3S,4S oder 3R,4R)-3-[(E)-2-Butenyl]-4-[(R)-2-hydroxytridecyl]-2-oxetanon, Rf-Wert: 0,475 und

ein 2. trans-Diastereomer, (3R,4R oder 3S,4S)-3-[(E)-2-Butenyl]-4-[(R)-2-hydroxytridecyl]-2-oxetanon, Rf-Wert: 0,44 (Chromatographie und Dünnschichtchromatographie über Kieselgel mit 1 Essigester/2 Methylenchlorid/2 n-Hexan.

Beispiel S

Aus Aethyl-(3R,5R und 3S,5R)-5-benzyloxy-3-hydroxyhexadecanoat (1:1) (Beispiel Ca) erhält man analog Beispiel Cb) und c) via Aethyl-(2R,3R und 2S,3S,5R)-5-(benzyloxy)-3-hydroxy-2-(2,3,4,5,6-pentafluorbenzyl)hexadecanoat (threo-Diastereomere) und

(3R,4R und 3S,4S)-4-[(R)-2-(Benzyloxy)tridecyl]-3-(2,3,4,5,6-pentafluorbenzyl)-2-oxetanon (trans-Diastereomere) die Ausgangsalkohole der Beispiele 74-77:

ein 1. trans-Diastereomer, (3S,4S oder 3R,4R)-4-[(R)-2-Hydroxytridecyl]-3-(2,3,4,5,6-pentafluorbenzyl)-2-oxetanon, Rf-Wert: 0,43 und

ein 2. trans-Diastereomer, (3R,4R oder 3S,4S)-4-[(R)-2-hydroxytridecyl]-3-(2,3,4,5,6-pentafluorbenzyl)-2-oxetanon, Rf-Wert: 0,39 (Chromatographie und Dünnschichtchromatographie an Kieselgel mit 1 Essigester/4,5 Methylenchlorid/4,5 n-Hexan).

Beispiel T

a) Eine Lösung von 0,5 ml Diisopropylamin in 15 ml THF wird bei 0°C mit 2,0 ml einer Lösung von 1,6M n-Butyllithium in Hexan versetzt und nach Rühren auf -75°C gekühlt. Dann wird eine Lösung von 765 mg N-Benzyl-N-phenylglycin-methylester in 3 ml THF zugesetzt. Nach Rühren wird eine Lösung von 700 mg (R)-3-(t-Butyldimethylsiloxy) tetradecanal (Beispiel Bb) in 5 ml THF zugetropft. Nach Rühren bei -75°C wird das Reaktionsgemisch auf wässriges Kaliumhydrogensulfat gegossen und mit Aether extrahiert. Die Aetherphase wird getrocknet, eingeengt, zwischen Hexan und Methanol/Wasser (7:3) verteilt, die Hexanphase getrocknet und eingeengt und der Rückstand an Kieselgel mit Pentan/Aether (5:1) chromatographiert. Man erhält 96,3 mg Methyl-(5R)-2-(N-benzylanilino)-5-(t-butyldimethylsiloxy)-3-hydroxyhexadecanoat, Diastereomer A, MS: 540 ($M^{+}$•-$C_4H_9$•) und 142,8 mg Methyl-(5R)-2-(N-benzylanilino)-5-(t-butyldimethylsiloxy)-3-hydroxyhexadecanoat, Diastereomer B, MS: 540 ($M^{+}$•-$C_4H_9$•) und 313,4 mg Gemisch obiger beider Diastereomeren.

b) 134 mg Diastereomer B werden in 3 ml 0,1N NaOH suspendiert und mit soviel Acetonitril versetzt, dass eine klare Lösung entsteht. Nach Rühren wird auf wässriges Kaliumhydrogensulfat gegossen und mit Aether extrahiert, die Aetherphase getrocknet und eingeengt. Nach Chomatographie an Kieselgel mit Methylenchlorid/Methanol (9:1) erhält man 108 mg (5R)-2-(N-Benzylanilino)-5-(t-butyldimethylsiloxy)-3-hydroxyhexadecansäure, Diastereomer B, MS: 526 ($M^{+}$•$C_4H_9$•).

c) Analog erhält man aus dem Diastereomeren A aus a) (5R)-2-(N-Benzylanilino)-5-(t-butyldimethylsiloxy)-3-hydroxyhexadecansäure, Diastereomer A, MS: 526 ($M^{+\bullet}$-$C_4H_9{}^{\bullet}$).

d) 1,1 g Diastereomer B von b), 1,1 g HBTU, 0,5 g Triäthylamin und 2 g Molekularsieb 4Å werden in 50 ml Acetonitril gerührt. Nach Filtration und Einengen wird das Produkt an Kieselgel mit Methylenchlorid chromatographiert.

Dabei resultieren 1,04 g 3R,4R(oder 3S,4S)-3-(N-Benzylanilino)-4-[(R)-2-(t-butyldimethylsiloxy)tridecyl]-2-oxetanon, Diastereomer B, MS: 566 $(M+H)^+$.

e) Analog erhält man aus dem Diastereomeren A aus c) 3S,4S(oder 3R,4R)-3-(N-Benzylanilino)-4-[(R)-2-(t-butyldimethylsiloxy)tridecyl]-2-oxetanon, Diastereomer A, MS: 566 $(M+H)^+$.

f) 1,0 g Diastereomer B von d) und 0,8 g Pd/C (10%) werden in 30 ml THF hydriert. Hierauf wird filtriert und eingeengt. Dabei erhält man 834 mg 3R,4R(oder 3S,4S)-3-Anilino-4-[(R)-2-(t-butyldimethylsiloxy)tridecyl]-2-oxetanon, Diastereomer B, MS: 475 ($M^{+\bullet}$).

g) Analog erhält man aus dem Diastereomeren A von e) 3S,4S(oder 3R,4R)-3-Anilino-4-[(R)-2-(t-butyldimethylsiloxy)tridecyl]-2-oxetanon, Diastereomer A, MS: 475 ($M^{+\bullet}$).

h) Die Produkte von f) und g) werden einzeln analog Be) in 3R,4R(oder 3S,4S)-3-Anilino-4-[(R)-2-hydroxytridecyl]-2-oxetanon, Diastereomer B, Smp. 104°C bzw.

3S,4S(oder 3R,4R)-3-Anilino-4-[(R)-2-hydroxytridecyl]-2-oxetanon, Diastereomer A, Smp. 60-62°C, die Ausgangsalkohole für Beispiel 79, umgewandelt.

Die Säuren der Formel $Q_q$-OH sind bekannt oder können in Analogie zu den bekannten Säuren hergestellt werden, z.B. durch Verseifung eines entsprechenden Niederalkylesters in einem Lösungsmittel, wie Aceton oder Methanol, mit einem Alkalimetallhydroxid, wie Kaliumhydroxid, in einem Alkohol, wie Aethanol oder Methanol. So kann man die Ausgangssäure der nachfolgenden Beispiele 2d) und 2e) wie folgt herstellen:

Eine Lösung von 3,8 g 2-Propylmalonamidsäureäthylester in 30 ml Aceton wird mit 22 ml 1N KOH in Aethanol versetzt und 4 Stunden gerührt, dann eingeengt, in Natriumbicarbonatlösung aufgenommen und mit Aethylacetat extrahiert. Die Wasserphase wird bei 0° mit Salzsäure auf pH 2 angesäuert und mit Aethylacetat extrahiert. Die Essigesterphase wird mit Sole gewaschen, getrocknet, eingeengt und der Rückstand aus Aethylacetat/Aether umkristallisiert. Man erhält 1,96 g 2-Propylmalonsäuremonoamid, Smp. 137°C.

Analog stellt man aus Aethyl-2-phenäthylmalonamat die 2-Phenäthylmalonamidsäure, Smp. 141,5°C, die Ausgangssäure für Beispiel 58-59 her.

Das (+) und (-) 2-Isopropylmalonsäuremonoamid (Ausgangsamid von Beispiel 11) kann man wie anschliessend beschrieben herstellen:

5,5 g rac-2-Isopropylmalonsäuremonoamid und 12,0 g Chinidin werden in 100 ml kochendem Wasser gelöst, mit einigen Kristallen des Chinidinsalzes des (S)-(+)-2-Isopropylmalonsäuremonoamides angeimpft und dann auskristallisiert. Das Kristallisat wird abgenutscht, mit Wasser und Aether gewaschen und getrocknet; dabei resultieren 8,3 g Chinidinsalz des (S)-(+)-2-Isopropylmalonsäuremonoamides. Dieses Salz wird in 10 proz. Salzsäure gelöst und bei 5°C belassen, die ausgefallenen Kristalle werden abgenutscht, mit Wasser gewaschen, getrocknet und nochmals aus Wasser unter Zusatz einiger Tropfen 1N Salzsäure umkristallisiert. Dabei erhält man 720 mg (S)-(+)-2-Isopropylmalonsäuremonoamid, Smp. 174°C, $[\alpha]_{589}^{20}$ = +45,6° (Aethanol, c=1).

Die bei der Kristallisation des Chinidinsalzes angefallene Mutterlauge wird mit 10 proz. Salzsäure sauer gestellt und bei 5°C belassen, die ausgefallenen Kristalle werden abgenutscht, mit Wasser gewaschen, getrocknet und nochmals aus Wasser unter Zusatz von einigen Tropfen 1N Salzsäure umkristallisiert. Dabei erhält man 850 mg (R)-(-)-2-Isopropylmalonsäuremonoamid, Smp. 176°C, $[\alpha]_{589}^{20}$ = -45,6° (Aethanol, c=1).

Die Ausgangssäure zum Beispiel 42 kann man wie folgt herstellen:

a) Einer Lösung von 13,6 g Malonsäuremethylestermonochlorid in 100 ml Methylenchlorid werden 20,6 g Thiomorpholin zugetropft. Nach Rühren wird das Gemisch mit 200 ml Methylenchlorid verdünnt, im Scheidetrichter mit Wasser gewaschen, dann getrocknet, filtriert und eingedampft. Der Rückstand wird durch Chromatographie an Kieselgel mit Methylenchlorid und dann Methylenchlorid/Aceton (1:1) gereinigt. Man erhält 17,6 g Methyl-tetrahydro-β-oxo-4H-1,4-thiazin-4-propionat.

b) Einer Lösung von 17,3 g des Esters von a) in 170 ml Aceton tropft man 85 ml 1N Kalilauge zu. Nach Rühren und Filtrieren wird eingedampft, der Rückstand in 200 ml Aceton verrührt und dann filtriert. Der Filterkuchen wird mit Aceton gewaschen und getrocknet. Eine wässrige Lösung des entstandenen Kaliumsalzes wird an einer Kationenaustauscher-Säule mit Wasser chromatographiert. Das Eluat wird zur Trockene eingeengt, der Rückstand mit Aether angeteigt und abfiltriert. Man erhält 13 g Tetrahydro-β-oxo-4H-1,4-thiazin-4-propionsäure, Smp. 119-120°C.

Die Ausgangssäure zum Beispiel 44 stellt man wie folgt her:

a) Einer Lösung von 5,6 g Methyl-1-carbamoylcyclopentancarboxylat in 66 ml Aceton werden 33 ml 1N Kalilauge zugetropft. Nach Rühren wird das Gemisch mit 250 ml Aceton versetzt, das ausgefallene Kaliumsalz abfiltriert und dann mit Aceton gewaschen und getrocknet.

b) Einer Lösung der erhaltenen 5,79 g Kaliumsalz in 35 ml Wasser wird bei 0°C mit 4 ml konz. Salzsäure auf pH 1 angesäuert. Der Niederschlag wird abfiltriert, mit Wasser und dann Diäthyläther gewaschen. Nach Trocknen erhält man 3,5 g 1-Carbamoylcyclopentancarbonsäure.

Man kann die Ausgangssäure des Beispiels 46 wie folgt herstellen:

Zu 26 ml 25 proz. wässrigem Ammoniak wird bei -10°C eine Lösung von 10,4 g Methoxymalonsäuremonomethylester in 70 ml Methylenchlorid getropft. Nach Rühren wird das Gemisch eingedampft, der Rückstand in Wasser gelöst und auf einem Kationenaustauscher mit Wasser chromatographiert. Das Eluat wird eingeengt, der Rückstand mit Diäthyläther angeteigt und abfiltriert. Der Niederschlag wird mit Aether gewaschen und getrocknet. Man erhält 8,9 g Methoxymalonamidsäure, Smp. 128-130°C.

Die Ausgangssäure zum Beispiel 49 kann wie folgt hergestellt werden:

Eine Lösung von 1,79 g Carbamoylmethylthioessigsäure in 42 ml Wasser wird mit 3,71 g Monoperoxyphthalsäure-magnesiumsalz-hexahydrat versetzt. Nach Rühren wird abfiltriert, das Filtrat eingeengt und mit 2 ml konz. Salzsäure angesäuert. Nach Abfiltrieren wird das Filtrat über einem Kationenaustauscher perkoliert, mit Wasser eluiert und das Eluat zur Trockene eingedampft. Der Rückstand wird mit Aceton aufgeschlämmt und abfiltriert. Man wäscht mit Aceton und trocknet. Es resultieren 1,65 g rac-[(Carbamoylmethyl)-sulfinyl]essigsäure, Smp. 137-138°C.

Die den Säuren der Formel $Q^a$-OH entsprechenden Niederalkylester sind bekannt oder können in Analogie zu den bekannten Estern hergestellt werden, z.B. wie nachstehend beschrieben ausgehend von dem Monoester der Formel $H-(X)_n-COOR"$, worin R" nieder-Alkyl ist, über den Dicarbonsäuremonoester der Formel $HOCO-(X)_n-COOR"$. So kann man die Ausgangssäure des Beispiels 2f) wie folgt herstellen:

a) Zu 11 ml Diisopropylamin und 5 g 4Å Molekularsieb in 75 ml THF werden bei -15°C 48 ml einer 1.6M n-Butyllithium-Lösung in Hexan zugetropft. Nach 15 Minuten wird das Reaktionsgemisch auf -78°C gekühlt und einer Lösung von 9,5 g Aethyl-1,3-dioxolan-2-carboxylat in 50 ml THF zugetropft. Nach 20 Minuten Rühren wird bei einer Temperatur unter -70°C $CO_2$ eingeleitet. Nach Sättigung wird 20 Minuten bei -75°C gerührt und dann auf Raumtemperatur aufgewärmt. Nach dem Verflüchtigen des $CO_2$-Gases wird das Reaktionsgemisch eingeengt, der Rückstand mit gesättigter Bicarbonatlösung und Essigester versetzt, die Essigesterphase verworfen, die wässrige Phase mit Kaliumhydrogensulfat auf pH 2 angesäuert und mit Essigester extrahiert. Die Essigesterphase wird getrocknet und eingeengt.

b) Zu einer Lösung von 1,08 g des Produkts von a), 1,1 ml Triäthylamin und 3 g Molekularsieb 4Å in 30 ml THF werden bei 0°C 0,93 ml Chlorameisensäureisobutylester in 5 ml THF zugetropft. Nach 40 Minuten Rühren wird während 10 Minuten Ammoniak-Gas eingeleitet und das Reaktionsgemisch anschliessend über Nacht gerührt. Hierauf wird filtriert, das Filtrat eingeengt und der Rückstand an Kieselgel mit Methylenchlorid/Methanol (95:5) chromatographiert. Dabei resultieren 420 mg Aethyl-2-carbamoyl-1,3-dioxolan-2-carboxylat, Smp. 99-100°C.

c) Eine Lösung von 190 mg des Produkts von b) in 10 ml Methanol wird mit 1 ml 2N KOH in Methanol versetzt und 90 Minuten bei Raumtemperatur gerührt. Hierauf wird eine Lösung von 280 mg Kaliumhydrogensulfat in 1 ml Wasser zugesetzt, das Reaktionsgemisch abgenutscht und das Filtrat eingedampft. Dabei erhält man die 2-Carbamoyl-1,3-dioxolan-2-carbonsäure.

Analog erhält man die 2-Carbamoyl-m-dioxan-2-carbonsäure (Ausgangssäure zu Beispiel 3m) aus dem Aethyl-m-dioxan-2-carboxylat.

Die Säuren der Formel $(R^3,R^4)NCO(X)_n-COOH$, worin X eine Gruppe =CHN(R,R°) ist, kann man ausgehend der entsprechenden Dicarbonsäuremonoester der Formel HOCO-X-COOR" über ein entsprechendes Succinimid und den entsprechenden Amidester der Formel $H_2NCO-X-COOR"$ herstellen, z.B. wie nachstehend beschrieben für die Ausgangssäure des Beispiels 9.

a) Zu 54 ml THF werden bei 0°C 4,54 g Dicyclohexylcarbodiimid, 4,16 g Acetamino-monoäthylmalonat und 2,53 g N-Hydroxysuccinimid gegeben. Nach 1 Stunde Rühren wird auf Raumtemperatur erwärmen gelassen und über Nacht gerührt. Dann wird auf 0°C abgekühlt und filtriert. Das Filtrat wird mit 20 ml 25%iger wässriger Ammoniaklösung versetzt, tagsüber bei Raumtemperatur und über Nacht bei 4°C stehen gelassen. Dann wird die Lösung eingedampft, die zurückbleibende wässrige Lösung mit Natriumbicarbonat versetzt. Die wässrige Phase wird abgetrennt, die organische Phase mit gesättigter Kochsalzlösung gewaschen, dann getrocknet und eingeengt. Der Rückstand wird in Aethylacetat enthaltendem Hexan filtriert. Die entstandenen Kristalle werden mit Aether gewaschen und dann getrocknet. Man erhält 1,2 g [D,L]-N-Acetyl-2-carbamoylglycinäthylester, Smp. 126-128°C.

b) Einer Suspension von 1,09 g des Amidesters von a) in 7 ml Aceton wird eine Lösung von 5,8 ml 1N Kalilauge zugetropft. Nach 3 Stunden Rühren wird das Gemisch eingeengt und der Rückstand in wässriger Natriumbicarbonatlösung gelöst. Die Lösung wird mit Aethylacetat extrahiert, die wässrige Phase unter Kühlung mit Salzsäure auf pH 3 angesäuert und dann über einen Ionenaustauscher perkoliert. Das Eluat wird zur Trockene eingeengt und der Rückstand mit Aceton angeteigt. Man erhält 500 mg [D,L]-N-Acetyl-2-carbamoylglycin, Smp. 120°C (Zersetzung).

Ausgangssäuren der Formel $(R^3,R^4)NCO(X)_n$-COOH, worin zumindest eins von $R^3$ und $R^4$ verschieden ist von H, kann man durch Umsetzung des entsprechenden Säureesters der Formel HOC(O)-$(X)_n$-C(O)O-R" mit einem Amin HN $(R^3,R^4)$ herstellen.

So kann man die Ausgangssäure des Beispiels 10d) wie folgt herstellen:

Eine Lösung von 3 g Malonsäuremonomethylester in 15 ml 40%-igem wässrigem Dimethylamin wird nach 18-stündigem Rühren eingeengt, durch einen stark sauren Kationentauscher filtriert, zur Trockene eingeengt und aus Chloroform kristallisiert. Einengen der Mutterlauge und Kristallisieren aus Aether liefern 1,3 g Dimethylcarbamoylessigsäure, Smp. 72-76°C.

Die Oxetanone der Formel I besitzen wertvolle pharmakologische Eigenschaften. Sie hemmen inbesondere die Pankreaslipase und können dementsprechend bei der Bekämpfung oder Verhütung von Obesitas, Hyperlipämie, Atherosklerose und Arteriosklerose verwendet werden.

Die Hemmung der Pankreaslipase durch die Oxetanone der Formel I kann experimentell gezeigt werden, indem man die bei der Spaltung von Triolein durch Schweinepankreaslipase freigesetzte Oelsäure titrimetrisch erfasst. Zu einer Emulsion, welche 1 mM Taurodeoxycholat, 9 mM Taurocholat, 0,1 mM Cholesterin, 1 mM Eilezithin, 15 mg/ml BSA, 2 mM Tris-HCl, 100 mM Natriumchlorid, 1 mM Calciumchlorid und das Substrat Triolein enthält, gibt man die in Aethanol oder Dimethylsulfoxid (10% des Emulsionsvolumens) gelöste Verbindung der Formel I und startet die Reaktion durch Zugabe von 1-3 µg Schweinepankreaslipase. Das pH wird während der Reaktion durch Zugabe von Natronlauge bei 8 gehalten. Aus dem während 10 Minuten ermittelten Verbrauch an Natronlauge wird die $IC_{50}$ berechnet. Die $IC_{50}$ ist diejenige Konzentration, bei der die Lipaseaktivität halbmaximal gehemmt wird. Die nachfolgende Tabelle enthält die für die Verbindungen der Formel I ermittelten $IC_{50}$-Werte in µg/ml.

| | | | | | | | | | |
|---|---|---|---|---|---|---|---|---|---|
| **Beispiel** | 1a,11a | 1b,11b | 2b,12b | 2d | 2e | 2f | | 2g1,13a | 2g2 |
| $IC_{50}$ | 0,032 | 0,025 | 0,063 | 0,12 | 0,051 | 0,47 | | 0,052 | 0,013 |
| **Beispiel** | 3c | 3d | 3e | 3f | 3g | 3h | | 3i | 3j |
| $IC_{50}$ | 0,39 | 0,90 | 1,41 | 0,083 | 0,294 | 2,1 | | 0,42 | 0,16 |
| **Beispiel** | 3k | 3l | 4a | 6b,14c | 7b | 10a | | 10b | 10c |
| $IC_{50}$ | 0,12 | 0,78 | 0,16 | 0,056 | 0,079 | 0,083 | | 0,042 | 0,1 |
| **Beispiel** | 10d | 10e | 10f | 37 | 43 | 45 | 51 | 78c | 78d |
| $IC_{50}$ | 0,47 | 0,027 | 0,32 | 0,034 | 0,12 | 0,047 | 0,36 | 0,28 | 0,051 |

Die akute Toxizität (nach einmaliger oraler Verabreichung an Mäusen) beträgt mehr als 5000 mg/kg für die Produkte der Beispiele 3d, 3h, 3i, 3l, 4a und 10a, b, e und f.

Die Oxetanone der Formel I können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden.

Zur Herstellung von pharmazeutischen Präparaten können die erfindungsgemässen Produkte mit pharmazeutisch inerten, anorganischen oder organischen Trägern verabreicht werden. Als solche Träger kann man für Tabletten, Lacktabletten, Dragees und Hartgelatinekapseln beispielsweise Lactose, Maisstärke oder Derivate davon, Talk, Stearinsäure oder deren Salze verwenden. Für Weichgelatinekapseln eignen sich als Träger beispielsweise pflanzliche Oele, Wachse, Fette, halbfette und flüssige Polyole; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker und Glukose.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Arzneimittel, enthaltend ein Oxetanon der Formel I, ebenfalls Gegenstand der vorliegenden Erfindung, weiterhin auch ein Verfahren zur Herstellung solcher Arzneimittel, welches dadurch gekennzeichnet ist, dass man ein Oxetanon der Formel I und gegebenenfalls einen oder mehrere andere therapeutisch wertvolle Stoffe in eine galenische Darreichungsform bringt. Wie erwähnt, können die Verbindungen der Formel I bei der Bekämpfung oder Verhütung von Krankheiten verwendet werden und zwar insbesondere bei der Bekämpfung oder Verhütung von Obesitas, Hyperlipämien, Atherosklerose und Arteriosklerose. Die Dosierung kann innerhalb weiter Grenzen variieren und ist natürlich in jedem einzelnen Fall den individuellen Gegebenheiten anzupassen. Im allgemeinen dürfte, bei oraler Verabreichung, eine Tagesdosis von etwa 0,1 mg bis 100 mg/kg Körpergewicht angemessen sein.

Die Oxetanone der Formel I können auch industriell gefertigten Lebensmitteln zugegeben werden, wobei insbesondere Fette, Oele, Butter, Margarine, Schokolade und andere Konfektartikel in Frage kommen. Solche industriell gefertigte Lebensmittel, die etwa 0,1 bis 5 Gew.-% eines Oxetanons der Formel I enthalten können, und deren Herstellung sind ebenfalls Gegenstand der vorliegenden Erfindung.

Die nachfolgenden Beispiele sollen die vorliegende Erfindung näher erläutern, ihren Umfang jedoch in keiner Weise beschränken. Sämtliche Temperaturen sind in Celsiusgraden angegeben.

Beispiel 1

Eine Lösung von 574 mg (3S,4S)-3-Aethyl-4-[(R)-2-hydroxynonadecyl]-2-oxetanon, 525 mg Triphenylphosphin, 290 mg 2-Isopropylmalonsäuremonoamid und 2 g Molekularsieb (4Å) in 10 ml THF werden unter Rühren bei 0° mit 0,4 ml Azodicarbonsäurediisopropylester versetzt. Nach 30 Minuten Rühren bei 0° und 1 Stunde bei Raumtemperatur wird das Reaktionsgemisch abfiltriert, das Molekularsieb mit Aether gewaschen und die Lösungsmittel abgedampft. Der Rückstand wird in Hexan gelöst und mit Methanol/Wasser (7:3) extrahiert. Die Hexanphase wird mit Aether verdünnt, getrocknet und eingedampft. Der Rückstand wird mit Methylenchlorid/ Aether (9:1) an Kieselgel chromatographiert. Man erhält

a) 239 mg (S)-[[(2S,3S)-3-Aethyl-4-oxo-2-oxetanyl]methyl]octadecyl (R oder S)-2-isopropylmalonamat, Smp. 115° und

b) 266 mg (S)-[[(2S,3S)-3-Aethyl-4-oxo-2-oxetanyl]methyl]octadecyl (S oder R)-2-isopropylmalonamat, Smp. 118°.

Beispiel 2

Analog Beispiel 1 erhält man aus (3S,4S)-3-Hexyl-4-[(R)-2-hydroxytridecyl]-2-oxetanon und 2-Isopropylmalonsäuremonoamid

a) (S)-1-[[(2S,3S)-3-Hexyl-4-oxo-2-oxetanyl]methyl]dodecyl -(R oder S)-2-isopropylmalonamat, Smp. 136° und

b) (S)-1-[[(2S,3S)-3-Hexyl-4-oxo-2-oxetanyl]methyl]dodecyl -(S oder R)-2-isopropylmalonamat, Smp. 82°;

c) aus (3S,4S)-3-Hexyl-4-[(R)-2-hydroxytridecyl]-2-oxetanon und Isopropylidenmalonsäuremonoamid (S)-1-[[(2S,3S)-3-Hexyl-4-oxo-2-oxetanyl]methyl]-dodecyl -2-carbamoyl-3-methylcrotonat, Smp. 108-111°;

d) aus (3S,4S)-3-Hexyl-4-[(R)-2-hydroxytridecyl]-2-oxetanon und 2-Propylmalonsäuremonoamid das (S)-1-[[(2S,3S)-3-Hexyl-4-oxo-2-oxetanyl]methyl]-dodecyl-(RS) -2-carbamoylvalerat (Epimere 1:1), Smp. 92-94°;

e) aus (3S,4S)-3-Aethyl-4-[(R)-2-hydroxynonadecyl] -2-oxetanon und 2-Propylmalonsäuremonoamid das (S)-1-[[(2S,3S)-3-Aethyl-4-oxo-2-oxetanyl]-methyl]octadecyl -(RS)-2-carbamoylvalerat (Epimere 1:1), Smp. 78-80°;

f) aus (3S,4S)-Aethyl-4-[(R)-2-hydroxynonadecyl]-2-oxetanon und 2-Carbamoyl-1,3-dioxolan-2-carbonsäure das (S)-1-[[(2S,3S)-3-Aethyl-4-oxo-2-oxetanyl]-methyl]octadecyl -2-carbamoyl-1,3-dioxolan-2-carboxylat, Smp. 95°;

g) aus (3S,4S)-3-Aethyl-4-[(R,10Z,13Z)-2-hydroxy -10,13-nonadecadienyl]-2-oxetanon und 2-Isopropylmalonsäuremonoamid

1. (all Z,S)-1-[[(2S,3S)-3-Aethyl-4-oxo-2-oxetanyl]methyl] -9,12-octadecadienyl (R oder S)-2-isopropylmalonamat, Smp. 87-88° (aus Aether) und

2. (all Z,S)-1-[[(2S,3S)-3-Aethyl-4-oxo-2-oxetanyl]methyl] -9,12-octadecadienyl-(S oder R)-2-isopropylmalonamat, IR: 3393, 1840, 1716, 1647, 1185 $cm^{-1}$.

Beispiel 3

Analog Beispiel 1 erhält man durch Reaktion des (3S,4S)-3-Hexyl-4-[(R)-2-hydroxytridecyl]-2-oxetanons mit den folgenden Amiden folgende Esteramide:

a) mit der 4-Carbamoylbuttersäure den 4-Carbamoylbuttersäure-(S)-1-[[(2S,3S)-3-hexyl-4-oxo -2-oxetanyl]methyl] dodecylester, Smp. 67-68°

b) mit der 3-Carbamoylpropionsäure den 3-Carbamoylpropionsäure-(S)-1-[[(2S,3S)-3-hexyl-4-oxo -2-oxetanyl] methyl]dodecylester, Smp. 50,5-51°

c) mit der 2-Carbamoylessigsäure den 2-Carbamoylessigsäure-(S)-1-[[(2S,3S)-3-hexyl-4-oxo -2-oxetanyl]methyl] dodecylester, Smp. 86,5-87°

d) mit dem Oxalsäuremonoamid das (S)-1-[[(2S,3S)-3-Hexyl-4-oxo -2-oxetanyl]methyl]dodecyloxamat, Smp. 77-78°

e) mit der Methylcarbamoylessigsäure das (S)-1-[[(2S,3S)-3-Hexyl-4-oxo-2-oxetanyl]methyl]dodecyl -N-methylmalonamat, Smp. 63-67°

f) mit der rac-2-Carbamoyl-4-methylvaleriansäure das (S)-1-[[(2S,3S)-3-Hexyl-4-oxo-2-oxetanyl]methyl]dodecyl -(RS)-2-carbamoyl-4-methylvalerat (Epimere 1:1), Smp. 102-104°

g) mit der 1-Carbamoylcydohexancarbonsäure das (S)-1-[[(2S,3S)-3-Hexyl-4-oxo-2-oxetanyl]methyl]dodecyl -1-carbamoylcydohexancarboxylat, Smp. 50-52°

h) mit der 2,2-Dimethylmalonamidsäure das (S)-1-[[(2S,3S)-3-Hexyl-4-oxo-2-oxetanyl]methyl]dodecyl -2,2-dimethylmalonamat, $[\alpha]_D^{20}$ = -23,8° ($CHCl_3$, c = 0,9%)

i) mit der rac-2-Methylmalonamidsäure das (S)-1-[[(2S,3S)-3-Hexyl-4-oxo-2-oxetanyl]methyl]dodecyl -(RS) -2-methylmalonamat (Epimere 1:1), Smp. 107-108°

j) mit der rac-2-Aethylmalonamidsäure das (S)-1-[[(2S,3S)-3-Hexyl-4-oxo-2-oxetanyl]methyl]dodecyl -(RS)-2-äthylmalonamat (Epimere 1:1), Smp. 87-90°

k) mit der rac-2-Butylmalonamidsäure das (S)-1-[[(2S,3S)-3-Hexyl-4-oxo-2-oxetanyl]methyl]dodecyl -(RS)-2-butylmalonamat (Epimere 1:1), Smp. 96-98°

l) mit der 2,2-Diäthylmalonamidsäure das (S)-1-[[(2S,3S)-3-Hexyl-4-oxo-2-oxetanyl]methyl]dodecyl -2,2-diäthylma-

lonamat, $[\alpha]_D^{20}$ = -21,1° ($CHCl_3$, c = 1%)

m) mit der 2-Carbamoyl-m-dioxan-2-carbonsäure das (S)-1-[[(2S,3S)-3-Hexyl-4-oxo-2-oxetanyl]methyl]dodecyl -2-carbamoyl-m-dioxan-2-carboxylat, Smp. 51°.

Beispiel 4

Analog Beispiel 1 erhält man aus dem (3S,4S)-3-Aethyl-4-[(R)-2-hydroxynonadecyl]-2-oxetanon und

a) aus der 1-Carbamoylcydohexancarbonsäure das (S)-1-[[(2S,3S)-3-Aethyl-4-oxo-2-oxetanyl]methyl]octadecyl -1-carbamoylcyclohexancarboxylat, Smp. 78-79° und

b) aus der 2-Carbamoyl-m-dioxan-2-carbonsäure das (S)-1-[[(2S,3S)-3-Aethyl-4-oxo-2-oxetanyl]methyl]octadecyl -2-carbamoyl-m-dioxan-2-carboxylat, Smp. 79°.

Beispiel 5

Analog Beispiel 1 erhält man aus (3R,4R oder 3S,4S)-4-[(R)-2-Hydroxytridecyl]-3-pentylthio-2-oxetanon und 2-Isopropylmalonsäureamid

a) das (S)-1-[[(2R,3R oder 2S,3S)-4-Oxo-3-pentylthio-2-oxetanyl]methyl]-dodecyl-(R oder S)-2-isopropylmalonamat, MS: 354 [$M^{+\bullet}$-(2-Isopropylmalonsäureamid)]; IR ($cm^{-1}$): 3397, 2924, 1829, 1731, 1657, 1120 und

b) das (S)-1-[[(2R,3R oder 2S,3S)-4-Oxo-3-pentylthio-2-oxetanyl]methyl]-dodecyl-(S oder R)-2-isopropylmalonamat, Smp. 77-78° (Diäthyläther).

Beispiel 6

Analog Beispiel 1 erhält man aus (3S,4S oder 3R,4R)-4-[(R)-2-Hydroxytridecyl]-3-pentylthio-2-oxetanon und 2-Isopropylmalonsäureamid

a) das (S)-1-[[(2S,3S oder 2R,3R)-4-Oxo-3-pentylthio-2-oxetanyl]methyl]dodecyl-(R oder S)-2-isopropylmalonamat, Smp. 133° (Aethylacetat) und

b) das (S)-1-[[(2S,3S oder 2R,3R)-4-Oxo-3-pentylthio-2-oxetanyl]methyl]-dodecyl-[S:R oder R:S(2:1)]-2-isopropylmalonamat, Smp. 102-104° (Aethylacetat).

Beispiel 7

Analog Beispiel 1 erhält man aus (3R,4R)-3-Benzyl-4-[(R)-2-hydroxytridecyl]-2-oxetanon und 2-Isopropylmalonsäuremonoamid ein Epimerengemisch, das durch Chromatographie an Silicagel mit Aethylacetat/Hexan/ Methylenchlorid (1:2:2) aufgetrennt wird in

a) (S)-1-[[(2R,3R)-3-Benzyl-4-oxo-2-oxetanyl]methyl]dodecyl-(R oder S)-2-isopropylmalonamat, Smp. 85-87° (Methylenchlorid) und

b) (S)-1-[[(2R,3R)-3-Benzyl-4-oxo-2-oxetanyl]methyl]dodecyl-(S oder R)-2-isopropylmalonamat, Smp. 108-110° (Methylenchlorid).

Beispiel 8

Analog Beispiel 1 erhält man aus (3S,4S)-3-Benzyl-4-[(R)-2-hydroxytridecyl]-2-oxetanon und 2-Isopropylmalonsäuremonoamid ein Epimerengemisch, das durch Chromatographie an Silicagel mit Aethylacetat/Hexan/ Methylenchlorid (1:2:2) aufgetrennt wird in

a) (S)-1-[[(2S,3S)-3-Benzyl-4-oxo-2-oxetanyl]methyl]dodecyl-(R oder S)-2-isopropylmalonamat, Smp. 107-108° (Methylenchlorid) und

b) (S)-1-[[(2S,3S)-3-Benzyl-4-oxo-2-oxetanyl]methyl]dodecyl-(S oder R)-2-isopropylmalonamat, Smp. 148-149° (Methylenchlorid).

Beispiel 9

Einer auf -10°C abgekühlten Suspension von 1,06 g (3S,4S)-3-Hexyl-4-[(R)-2-hydroxytridecyl]-2-oxetanon, 480 mg [D,L]-N-Acetyl-2-carbamoylglycin, 1,1 g Triphenylphosphin und 1,2 g Molekularsieb 4Å in 12 ml THF werden 1,03 g Azodicarbonsäure-di-t-butylester zugegeben. Nach 1 Stunde Rühren bei 0°C und über Nacht bei Zimmertemperatur wird das Reaktionsgemisch ähnlich wie in Beispiel 1 beschrieben aufgearbeitet. Man erhält

a) 190 mg (S)-1-[[(2S,3S)-3-Hexyl-4-oxo-2-oxetanyl]methyl]dodecyl-(R oder S)-2-acetamidomalonamat, Smp. 125-126° und

b) 100 mg (S)-1-[[(2S,3S)-3-Hexyl-4-oxo-2-oxetanyl]methyl]dodecyl-(RS)-2-acetamidomalonamat (Epimere 1:1), Smp. 110-116°, $[\alpha]_D^{25} = -8°$ (c = 0,5, $CHCl_3$).

Beispiel 10

Analog Beispiel 1 jedoch unter Verwendung folgender Amiden erhält man folgende Esteramide:

a) aus dem Oxalsäuremonoamid das (S)-1-[[(2S,3S)-3-Aethyl-4-oxo-2-oxetanyl]methyl]octadecyloxamat, Smp. 99-100°

b) aus der 2-Carbamoylessigsäure das (S)-1-[[(2S,3S)-3-Aethyl-4-oxo-2-oxetanyl]methyl]octadecylmalonamat, Smp. 90,5-91,5°

c) aus der Methylcarbamoylessigsäure das (S)-1-[[(2S,3S)-3-Aethyl-4-oxo-2-oxetanyl]methyl]octadecyl-N-methylmalonamat, Smp. 84-85°

d) aus der Dimethylcarbamoylessigsäure das (S)-1-[[(2S,3S)-3-Aethyl-4-oxo-2-oxetanyl]methyl]octadecyl-N,N-dimethylmalonamat, Smp. 66-67°

e) aus der rac-2-Methylmalonamidsäure das (S)-1-[[(2S,3S)-3-Aethyl-4-oxo-2-oxetanyl]methyl]octadecyl-(RS)-2-methylmalonamat (Epimere 1:1), Smp. 91,5-92°

f) aus der 3-Carbamoylpropionsäure das (S)-1-[[(2S,3S)-3-Aethyl-4-oxo-2-oxetanyl]methyl]octadecyl-succinamat, Smp. 74,5-75,7°.

Beispiel 11

Analog Beispiel 1, jedoch unter Verwendung von (S)-(+)- bzw. (±)-2-Isopropylmalonsäuremonoamid, erhält man

a) (S)-[[(2S,3S)-3-Aethyl-4-oxo-2-oxetanyl]methyl]octadecyl-(R)-2-isopropylmalonamat, Smp. 115° und

b) (S)-[[(2S,3S)-3-Aethyl-4-oxo-2-oxetanyl]methyl]octadecyl-(S)-2-isopropylmalonamat, Smp. 118°.

Beispiel 12

Analog Beispiel 1, 2a) b) und 11 erhält man aus (3S,4S)-3-Hexyl-4-[(R)-2hydroxytridecyl]-2-oxetanon und (±)- bzw. (S)-(+)-2-Isopropylmalonsäuremonoamid

a) (S)-1-[[(2S,3S)-3-Hexyl-4-oxo-2-oxetanyl]methyl]dodecyl-(R)-2-isopropylmalonamat, Smp. 136° bzw.

b) (S)-1-[[(2S,3S)-3-Hexyl-4-oxo-2-oxetanyl]methyl]dodecyl-(S)-2-isopropylmalonamat, Smp. 82°

c) aus (3S,4S)-3-Hexyl-4-[(R)-2-hydroxytridecyl]-2-oxetanon und 2-Propylmalonsäuremonoamid nach chromatographischer Auftrennung an Kieselgel mit Methylenchlorid/Acetonitril (85:15)

1. das (S)-1-[[(2S,3S)-3-Hexyl-4-oxo-2-oxetanyl]methyl]dodecyl-(R oder S)-2-carbamoylvalerat, Smp. 113° (aus Methanol/Wasser) und

2. das (S)-1-[[(2S,3S)-3-Hexyl-4-oxo-2-oxetanyl]methyl]dodecyl-(S oder R)-2-carbamoylvalerat, Smp. 85°.

Beispiel 13

Analog Beispiel 1, 2g) und 11 erhält man aus (3S,4S)-3-Aethyl-4-[(R,10Z,13Z)-2-hydroxy-10,13-nonadecadienyl]-2-oxetanon und (±)- bzw. (S)-(+)-2-Isopropylmalonsäuremonoamid

a) (all Z,S)-1-[[(2S,3S)-3-Aethyl-4-oxo-2-oxetanyl]methyl]-9,12-octadecadienyl-(R)-2-isopropylmalonamat, Smp. 87-88° (aus Aether) und

b) (all Z,S)-1-[[(2S,3S)-3-Aethyl-4-oxo-2-oxetanyl]methyl]-9,12-octadecadienyl-(S)-2-isopropylmalonamat, Smp. 109° (aus wässrigem Methanol).

Beispiel 14

Analog Beispiel 1, 6 und 11 erhält man aus (3S,4S oder 3R,4R)-4-[(R)-2-Hydroxytridecyl]-3-pentylthio-2-oxetanon und (±)- bzw. (S)-(+)-2-Isopropylmalonsäureamid

a) das (S)-1-[[(2S,3S oder 2R,3R)-4-Oxo-3-pentylthio-2-oxetanyl]methyl] dodecyl-(R)-2-isopropylmalonamat, Smp. 133° (Aethylacetat)

b) das (S)-1-[[(2S,3S oder 2R,3R)-4-Oxo-3-pentylthio-2-oxetanyl]methyl] dodecyl-(S)-2-isopropylmalonamat, Smp. 93° (Diäthyläther/Hexan) und

c) das (S)-1-[[(2S,3S oder 2R,3R)-4-Oxo-3-pentylthio-2-oxetanyl]methyl] dodecyl-[S:R (2:1)]-2-isopropylmalonamat, Smp. 102-104° (Aethylacetat).

Beispiel 15

Analog Beispiel 1 und 11 erhält man aus (3S,4S oder 3R,4R)-Benzyl-4-[(R)-2-hydroxytridecyl]-2-oxo-3-oxetancarbamat und (S)-(+)-2-Isopropylmalonsäuremonoamid das (S)-1-[[(2S,3S oder 2R,3R)-3-[1-(Benzyloxy)formamido]-4-oxo-2-oxetanyl]methyl]dodecyl-(S)-2-isopropylmalonamat, Smp. 133° (aus Aether/ Hexan).

Beispiel 16

Analog Beispiel 1 und 11 erhält man

a) aus (3R,4R)-4-[(R)-2-Hydroxytridecyl]-3-(phenylthio)-2-oxetanon und (S)-(+)-2-Isopropylmalonsäuremonoamid das (S)-1-[[(2R,3R)-4-Oxo-3-(phenylthio)-2-oxetanyl]methyl]dodecyl-(S)-2-isopropylmalonamat, Smp. 88° (Aether)

b) aus (3S,4S)-4-[(R)-2-Hydroxytridecyl]-3-(phenylthio)-2-oxetanon und 2-Isopropylmalonsäuremonoamid das (S)-1-[[(2S,3S)-4-Oxo-3-(phenylthio)-2-oxetanyl]methyl]dodecyl-(RS)-2-isopropylmalonamat (1:1 Epimere), Smp. 109° (Aether)

c) aus (3R,4R)-4-[(R)-2-Hydroxytridecyl]-3-(phenylthio)-2-oxetanon und 2-Isopropylmalonsäuremonoamid das gleiche Produkt wie unter a) und das (S)-1-[[(2R,3R)-4-Oxo-3-(phenylthio)-2-oxetanyl]methyl]dodecyl-2-isopropylmalonamat (R:S = 7:1), Smp. 85° (Aether).

Beispiel 17

Analog Beispiel 1 und 11 erhält man aus (S)-(+)-2-Isopropylmalonsäuremonoamid und aus folgenden Alkoholen folgende Ester:

a) aus (3S,4S oder 3R,4R)-4-[(R)-2-Hydroxynonadecyl]-3-(methylthio)-2-oxetanon das (S)-1-[[(2S,3S oder 2R,3R)-3-(Methylthio)-4-oxo-2-oxetanyl] methyl]octadecyl-(S)-2-isopropylmalonamat, Smp. 133° (aus Aether)

b) aus (3R,4R oder 3S,4S)-4-[(R)-2-Hydroxynonadecyl]-3-(methylthio)-2-oxetanon das (S)-1-[[(2R,3R oder 2S,3S) -3-(Methylthio)-4-oxo-2-oxetanyl] methyl]octadecyl-(S)-2-isopropylmalonamat, Smp. 103° (aus Aether) und

c) aus (3S,4R oder 3R,4S)-4-[(R)-2-Hydroxynonadecyl]-3-(methylthio)-2-oxetanon das (S)-1-[[(2R,3S oder 2S,3R) -3-(Methylthio)-4-oxo-2-oxetanyl] methyl]octadecyl-(S)-2-isopropylmalonamat, Smp. 96° (aus Aether/Hexan)

d) aus (3R,4S oder 3S,4R)-4-[(R)-2-Hydroxynonadecyl]-3-(methylthio]-2-oxetanon das (S)-1-[[(2S,3R oder 2R,3S) -3-(Methylthio)-4-oxo-2-oxetanyl] methyl]octadecyl-(S)-2-isopropylmalonamat, Smp. 120° (aus Aether/Hexan).

Beispiel 18

Analog Beispiel 1 und 11 erhält man aus (R)-(-)-2-Isopropylmalonsäuremonoamid und

a) aus (3S,4S oder 3R,4R)-4-[(R)-2-Hydroxynonadecyl]-3-(methylthio)-2-oxetanon das (S)-1-[[(2S,3S oder 2R,3R) -3-(Methylthio)-4-oxo-2-oxetanyl] methyl]octadecyl-(R)-2-isopropylmalonamat, Smp. 96° (aus Aether)

b) aus (3R,4R oder 3S,4S)-4-[(R)-2-Hydroxynonadecyl]-3-(methylthio)-2-oxetanon das (S)-1-[[(2R,3R oder 2S,3S) -3-(Methylthio)-4-oxo-2-oxetanyl] methyl]octadecyl-(R)-2-isopropylmalonamat, Smp. 87° (aus Aether/Hexan).

Beispiel 19

In Analogie zu Beispiel 1 und 11 erhält man aus (S)-(+)-2-Isopropylmalonsäuremonoamid und aus folgenden Alkoholen folgende Ester:

a) aus (3S,4S oder 3R,4R)-3-(Benzylthio)-4-[(R)-2-hydroxytridecyl]-2-oxetanon das (S)-1-[[(2S,3S oder 2R,3R)-3-(Benzylthio)-4-oxo-2-oxetanyl]methyl]dodecyl-(S)-2-isopropylmalonamat, Smp. 84° (aus Pentan)

b) aus (3R,4R oder 3S,4S)-3-(Benzylthio)-4-[(R)-2-hydroxytridecyl]-2-oxetanon das (S)-1-[[(2R,3R oder 2S,3S)-3-(Benzylthio)-4-oxo-2-oxetanyl]methyl]dodecyl-(S)-2-isopropylmalonamat, Smp. 65° (aus Aether/Pentan)

c) aus (3S,4R und 3R,4S)-3-(Benzylthio)-4-[(R)-2-hydroxytridecyl]-2-oxetanon

1. das (S)-1-[[(2S,3R oder 2R,3S)-3-(Benzylthio)-4-oxo-2-oxetanyl]methyl] dodecyl-(S)-2-isopropylmalonamat, Smp. 69° (aus Pentan)

2. das (S)-1-[[(2R,3S oder 2S,3R)-3-(Benzylthio)-4-oxo-2-oxetanyl]methyl] dodecyl-(S)-2-isopropylmalonamat, Smp. 106° (aus Hexan).

Beispiel 20

In Analogie zu Beispiel 1 und 11 erhält man aus (R)-(-)-2-Isopropylmalonsäuremonoamid und aus folgenden Alkoholen folgende Ester:

a) aus (3S,4S oder 3R,4R)-3-(Benzylthio)-4-[(R)-2-hydroxytridecyl]-2-oxetanon das (S)-1-[[(2S,3S oder 2R,3R)-3-(Benzylthio)-4-oxo-2-oxetanyl]methyl]dodecyl-(R)-2-isopropylmalonamat, Smp. 130° (aus Aether/Pentan)

b) aus (3R,4R oder 3S,4S)-3-(Benzylthio)-4-[(R)-2-hydroxytridecyl]-2-oxetanon das (S)-1-[[(2R,3R oder 2S,3S)-3-(Benzylthio)-4-oxo-2-oxetanyl]methyl]dodecyl-(R)-2-isopropylmalonamat, Smp. 119° (aus Hexan/Pentan)

c) aus (3S,4R und 3R,4S)-3-(Benzylthio)-4-[(R)-2-hydroxytridecyl]-2-oxetanon

1. das (S)-1-[[(2S,3R oder 2R,3S)-3-(Benzylthio)-4-oxo-2-oxetanyl]methyl] dodecyl-(R)-2-isopropylmalonamat, Smp. 132° (aus Aether/Pentan)

2. das (S)-1-[[(2R,3S oder 2S,3R)-3-(Benzylthio)-4-oxo-2-oxetanyl]methyl] dodecyl-(R)-2-isopropylmalonamat, Smp. 102° (aus Aether/Pentan).

Beispiel 21

Analog Beispiel 1 und 11 erhält man durch Reaktion von (3R,4R)-3-Aethyl-4-[(R)-2-hydroxynonadecyl]-2-oxetanon

a) mit (S)-(+)-2-Isopropylmalonsäuremonoamid das (S)-1-[[(2R,3R)-3-Aethyl-4-oxo-2-oxetanyl]methyl]octadecyl-(S)-2-isopropylmalonamat, Smp. 116-118° (Methylenchlorid),

b) mit 1-Carbamoylcyclohexancarbonsäure das (S)-1-[[(2R,3R)-3-Aethyl-4-oxo-2-oxetanyl]methyl]octadecyl-1-carbamoylcydohexancarboxylat, Smp. 71-74°.

Beispiel 22

In Analogie zu Beispiel 1 und 11 erhält man

a) aus (S)-(+)-2-Isopropylmalonsäuremonoamid und (3R,4R)-4-[(R)-2-Hydroxynonadecyl]-3-methyl-2-oxetanon das (S)-1-[[(2R,3R)-3-Methyl-4-oxo-2-oxetanyl]methyl]octadecyl-(S)-2-isopropylmalonamat, Smp. 124-126° (aus Essigester/Hexan)

b) aus rac-2-t-Butylmalonsäuremonoamid und (3R,4R)-4-[(R)-2-Hydroxynonadecyl]-3-methyl-2-oxetanon das (S) -1-[[(2R,3R)-3-Methyl-4-oxo-2-oxetanyl] methyl]octadecyl-(RS)-2-t-butylmalonamat (Epimere 1:1), Smp. 51-54° (Essigester/Hexan).

Beispiel 23

Eine Lösung von 277 mg (2S,3S,5S)-5-[[(cis)-2-Carbamoylcydohexyl]oxy]-2-hexyl-3-hydroxyhexadecansäure (Beispiel Jd) in 24 ml Methylenchlorid und 3 ml DMF wird mit 2 g Molekularsieb (4Å), 240 mg HBTU und 240 mg Triäthylamin versetzt. Nach Rühren werden 300 mg HBTU und 300 mg Triäthylamin hinzugefügt. Dann wird filtriert und das Filtrat eingeengt. Der Rückstand wird zwischen Methanol/Wasser (7:3) und Hexan verteilt und mit Hexan extrahiert. Die Hexanphase wird mit Methylenchlorid verdünnt, dann getrocknet und eingeengt und der Rückstand aus Aether/Hexan umkristallisiert. Dabei resultieren

a) 96 mg (1R oder S,2S oder R)-2-[[(S)-1-[[(2S,3S)-3-Hexyl-4-oxo-2-oxetanyl] methyl]dodecyl]oxy]cydohexancarboxamid (1. cis-Diastereomer), Smp. 102° (aus Aether/Hexan)

b) analog wird aus (2S,3S,5S)-5-[[(cis)-2-Carbamoylcydohexyl]oxy]-2-hexyl-3-hydroxyhexadecansäure (2. diast. Säure) in Beispiel Je) das (1S oder R,2R oder S)-2-[[(S)-1-[[(2S,3S)-3-Hexyl-4-oxo-2-oxetanyl]methyl]dodecyl]oxy] cyclohexancarboxamid (2. cis-Diastereomer), Smp. 94° (aus Aether/Hexan) erhalten.

Beispiel 24

Eine Lösung von 42,5 mg (2S,3S,5S)-5-[(R/S)-2-Carbamoyl-1-methoxyäthoxy]-2-hexyl-3-hydroxydecansäure (Beispiel Kd) in 10 ml Methylenchlorid/Acetonitril (1:1) wird mit 1 g Molekularsieb (4Å), 0,1 ml Triäthylamin und 50 mg HBTU versetzt. Nach Rühren wird das Reaktionsgemisch filtriert, eingeengt und der Rückstand zwischen Hexan und Methanol/Wasser (1:1) verteilt; die wässrig methanolische Phase wird mit Hexan extrahiert, die Hexanphase getrocknet und eingeengt; anschliessend wird der Rückstand an Kieselgel mit 5 proz. Methanol in Methylenchlorid chromatographiert. Dabei resultieren 21 mg (R/S)-3-[[(S)-1-[[(2S,3S)-3-Hexyl-4-oxo-2-oxetanyl]methyl] dodecyl]oxy]-3-methoxypropionamid (Epimere 3:1), Smp. 54°.

Beispiel 25

In eine Lösung von 40 mg 3-[[(S)-1-[[(2S,3S)-3-hexyl-4-oxo-2-oxetanyl] methyl]dodecyl]oxy]propionsäure (Beispiel L) in 2,5 ml Acetonitril wird Ammoniagas bis zur Sättigung eingeblasen und anschliessend 50 mg HBTU hinzugefügt. Dann wird filtriert und eingedampft und der Rückstand an Kieselgel mit 5 proz. Methanol in Methylenchlorid chromatographiert. Es resultieren 32,5 mg (3S,4S)-4-[(S)-2-(2-Carbamoyläthoxy)tridecyl]-3-hexyl-2-oxetanon, Smp. 35°.

Beispiel 26

Eine Lösung von 33,5 mg (S)-1-[[(2S,3S)-3-Hexyl-4-oxo-2-oxetanyl]methyl] dodecyl-hydrogenmalonat (Beispiel N)

in 2 ml Acetonitril wird mit 60 mg HBTU und 75 mg Isopropylamin versetzt. Nach Rühren wird das Reaktionsgemisch abfiltriert, das Filtrat eingeengt und der Rückstand an Kieselgel mit Hexan/Methylenchlorid/Aethylacetat (2:2:1) chromatographiert. Es resultieren 31,1 mg (S)-1-[[(2S,3S)-3-Hexyl-4-oxo-2-oxetanyl]methyl]dodecyl-N-isopropylmalonamat, Smp. 59°.

In zu Beispiel 1 analoger Weise werden folgende Verbindungen hergestellt:

Beispiel 27: (S)-1 -[[(2S,3S)-3-Hexyl-oxo-2-oxetanyl]methyl]dodecyl-5-carbamoylvalerianat, Smp. 50-51°,
Beispiel 28: (S)-1-[[(2S,3S)-3-Hexyl-4-oxo-2-oxetanyl]methyl] dodecyl-6-carbamoylhexanoat, Smp. 52-53°,
Beispiel 29: (S)-1-[[(2S,3S)-3-Hexyl-4-oxo-2-oxetanyl]methyl]dodecyl-7-carbamoylheptanoat, Smp. 40-43°,
Beispiel 30: (S)-1-[[(2S,3S)-3-Hexyl-4-oxo-2-oxetanyl]methyl]dodecyl-9-carbamoylnonanoat, Smp. 29-30°,
Beispiel 31: (S)-1-[[(2S,3S)-3-Aethyl-4-oxo-2-oxetanyl]methyl]octadecyl-4-carbamoylbutyrat, Smp. 74-75°,
Beispiel 32: (S)-1-[[(2S,3S)-3-Aethyl-4-oxo-2-oxetanyl]methyl]octadecyladipamat, Smp. 63,5-64,5°,
Beispiel 33: (S)-1-[[(2S,3S)-3-Aethyl-4-oxo-2-oxetanyl]methyl]octadecyl-6-carbamoylhexanoat, Smp. 71,5-72,5°,
Beispiel 34: (S)-1-[[(2S,3S)-3-Hexyl-4-oxo-oxetanyl]methyl]dodecyl (R oder S)-2-t-butylmalonamat, Smp. 53-54°,
Beispiel 35: (S)-1-[[(2S,3S)-3-Hexyl-4-oxo-2-oxetanyl]methyl]dodecyl-(S oder R)-2-t-butylmalonamat, $[\alpha]_D^{20}$= -16,4° (c = 0,8, $CHCl_3$),
Beispiel 36: (S)-1-[[(2S,3S)-3-Aethyl-4-oxo-2-oxetanyl]methyl]octadecyl-(R oder S)-2-t-butylmalonamat, Smp. 48-49°,
Beispiel 37: (S)-1-[[(2S,3S)-3-Aethyl-4-oxo-2-oxetanyl]methyl]octadecyl-(S oder R)-2-t-butylmalonamat, Smp. 81-82°,
Beispiel 38:(S)-1-[[(2S,3S)-3-Hexyl-4-oxo-2-oxetanyl]methyl]dodecyl-(S)-3-[1-(benzyloxy)formamido]succinamat, Smp. 72-73°,
Beispiel 39: (S)-1-[[(2S,3S)-3-Hexyl-4-oxo-2-oxetanyl]methyl]dodecyl-cis-6-carbamoyl-3-cyclohexen-1-carboxylat, Smp. 55-59°,
Beispiel 40:(S)-1-[[(2S,3S)-3-Hexyl-4-oxo-2-oxetanyl]methyl]dodecyl-cis-2-carbamoylcyclohexancarboxylat, Smp. 76-77°,
Beispiel 41: (S)-1-[[(2S,3S)-3-Hexyl-4-oxo-2-oxetanyl]methyl]dodecyl-β-oxo-4-morpholinpropionat, Smp. 49-51°,
Beispiel 42: (S)-1-[[(2S,3S)-3-Hexyl-4-oxo-2-oxetanyl]methyl]dodecyl-tetrahydro-β-oxo-4H-1,4-thiazin-4-propionat, Smp. 59-61°,
Beispiel 43:(S)-1-[[(2S,3S)-3-Aethyl-4-oxo-2-oxetanyl]methyl]octadecyl-1-carbamoylcyclopentancarboxylat, Smp. 62-63°,
Beispiel 44: (S)-1-[[(2S,2S)-3-Hexyl-4-oxo-2-oxetanyl]methyl]dodecyl-1-carbamoylcyclopentancarboxylat, Smp. 40-41°,
Beispiel 45: (S)-1-[[(2S,3S)-3-Hexyl-4-oxo-2-oxetanyl]methyl]dodecyl-(RS)-2-benzylmalonomat (Epimere 1:1), Smp. 86-92°,
Beispiel 46: (S)-1-[[(2S,3S)-3-Hexyl-4-oxo-2-oxetanyl]methyl]dodecyl-(RS)-2-methoxymalonamat (Epimere 1:1), Smp. 65-67°,
Beispiel 47:(S)-1-[[(2S,3S)-3-Hexyl-4-oxo-2-oxetanyl]methyl]dodecyl-[(carbamoyl)thio]acetat, Smp. 58-60°,
Beispiel 48: (S)-1-[[(2S,3S)-3-Aethyl-4-oxo-2-oxetanyl]methyl]octadecyl-[(carbamoylmethyl)thio]acetat, Smp. 83-84°C,
Beispiel 49:(S)-1-[[(2S,3S)-3-Hexyl-4-oxo-2-oxetanyl]methyl]dodecyl-[(RS)-(carbamoylmethyl)sulfinyl]acetat (Epimere 1:1), Smp. 55-59°,
Beispiel 50:(S)-1-[[(2S,3S)-3-Aethyl-4-oxo-2-oxetanyl]methyl]octadecyl-[(RS)-(carbamoylmethyl)thio]acetat-S-oxid, Smp. 80-82°,
Beispiel 51:(S)-1-[[(2S,3S)-3-Hexyl-4-oxo-2-oxetanyl]methyl]dodecyl-3-[(2-carbamoyläthyl)thio]propionat, Smp. 73-74°,
Beispiel 52: (S)-1-[[(2S,3S)-3-Hexyl-4-oxo-2-oxetanyl]methyl]dodecyl-3-[(RS)-(2-carbamoyläthyl)sulfinyl]propionat (Epimere 1:1), Smp. 48-51°,
Beispiel 53: (S)-1-[[(2S,3S)-3-Hexyl-4-oxo-2-oxetanyl]methyl]dodecyl-(carbamoylmethoxy)acetat, Smp. 52-53°,
Beispiel 54:(S)-1-[[(2S,3S)-3-Aethyl-4-oxo-2-oxetanyl]methyl]octadecyl-(carbamoylmethoxy)acetat, Smp. 75-76°,
Beispiel 55: (S)-1-[[(2S,3S)-3-Hexyl-4-oxo-2-oxetanyl]methyl]dodecyl-(RS)-2-[1-(benzyloxy)formamido]malonamat (Epimere 1:1), Smp. 94-95°,
Beispiel 56: (S)-1-[[(2S,3S)-3-Aethyl-4-oxo-2-oxetanyl]methyl]octadecyl-(RS)-2-isobutylmalonamat (Epimere 1:1), Smp. 96-99°,
Beispiel 57a): (S)-1-[[(2S,3S)-3-Aethyl-4-oxo-2-oxetanyl]methyl]octadecyl-(RS)-2-benzylmalonamat (Epimere 1:1), Smp. 96-103°,
Beispiel 57b): (S)-1-[[(2S,3S)-3-Aethyl-4-oxo-2-oxetanyl]methyl]octadecyl-(R oder S)-2-benzylmalonamat, Smp.

118-120°,

Beispiel 58:(S)-1-[[(2S,3S)-3-Hexyl-4-oxo-2-oxetanyl]methyl]dodecyl-(RS)-2-phenäthylmalonamat (Epimere 1:1), Smp. 92-93°,

Beispiel 59:(S)-1-[[(2S,3S)-3-Aethyl-4-oxo-2-oxetanyl]methyl]octadecyl-(RS)-2-phenäthylmalonamat (Epimere 1:1)), Smp. 97-98°.

Beispiel 60

Analog Beispiel 1 und 11 erhält man aus (S)-(+)-2-Isopropylmalonsäuremonoamid und (3R,4R)-4-[(R)-2-Hydroxynonadecyl]-3-(2-propinyl)-2-oxetanon (Beispiel M) das (S)-1-[[(2R,3R)-4-Oxo-3-(2-propinyl)-2-oxetanyl]methyl]octadecyl-(S)-2-isopropylmalonamat, Smp. 92-95°C (aus Essigester/Hexan).

Beispiel 61

Analog Beispiel 1 und 11 erhält man aus (S)-(+)-2-Isopropylmalonsäuremonoamid und (3R,4R)-4-[(R)-2-Hydroxynonadecyl]-3-propyl-2-oxetanon (Beispiel O) das (S)-1-[[(2R,3R)-4-Oxo-3-propyl-2-oxetanyl]methyl]octadecyl-(S)-2-isopropylmalonamat, Smp. 90-93° (aus Essigester/Hexan).

In zu Beispiel 1 und 11 analoger Weise werden folgende Verbindungen hergestellt:

Beispiel 62: (S)-1-[[(2S,3S)-3-(3-Methyl-2-butenyl)-4-oxo-2-oxetanyl]methyl] dodecyl-(R oder S)-2-isopropylmalonamat, Smp. 100-102°,

Beispiel 63:(S)-1-[[(2S,3S)-3-(3-Methyl-2-butenyl)-4-oxo-2-oxetanyl]methyl] dodecyl-(S oder R)-2-isopropylmalonamat, Smp. 120-121°,

Beispiel 64: (S)-1-[[(2R,3R)-3-(3-Methyl-2-butenyl)-4-oxo-2-oxetanyl] methyl]dodecyl-(R oder S)-2-isopropylmalonamat, Smp. 60-62°,

Beispiel 65: (S)-1-[[(2R,3R)-3-(3-Methyl-2-butenyl)-4-oxo-2-oxetanyl] methyl]dodecyl-(S oder R)-2-isopropylmalonamat, Smp. 76-78°,

Beispiel 66: (S)-1-[[(2S,3S oder 2R,3R)-3-(5-Chlorpentyl)-4-oxo-2-oxetanyl] methyl]dodecyl-(S)-2-isopropylmalonamat, Smp. 66-72°,

Beispiel 67: (S)-1-[[(2S,3S oder 2R,3R)-3-(5-Chlorpentyl)-4-oxo-2-oxetanyl] methyl]dodecyl-(R)-2-isopropylmalonamat, Smp. 130°,

Beispiel 68: (S)-1-[[(2R,3R oder 2S,3S)-3-(5-Chlorpentyl)-4-oxo-2-oxetanyl] methyl]dodecyl-(S)-2-isopropylmalonamat, Smp. 44-50°,

Beispiel 69: (S)-1-[[(2R,3R oder 2S,3S)-3-(5-Chlorpentyl)-4-oxo-2-oxetanyl] methyl]dodecyl-(R)-2-isopropylmalonamat, Smp. 85-87°,

Beispiel 70: (S)-1-(2S,3S oder 2R,3R)-[[3-[(E)-2-Butenyl]-4-oxo-2-oxetanyl] methyl]dodecyl-(R oder S)-2-isopropylmalonamat, Smp. 105-107°,

Beispiel 71: (S)-1-(2S,3S oder 2R,3R)-[[3-[(E)-2-Butenyl]-4-oxo-2-oxetanyl] methyl]dodecyl-(S oder R)-2-isopropylmalonamat, Smp. 96-98°,

Beispiel 72: (S)-1-(2R,3R oder 2S,3S)-[[3-[(E)-2-Butenyl]-4-oxo-2-oxetanyl] methyl]dodecyl-(S)-2-isopropylmalonamat, Smp. 89-90°,

Beispiel 73: (S)-1-(2R,3R oder 2S,3S)-[[3-[(E)-2-Butenyl]-4-oxo-2-oxetanyl] methyl]dodecyl-(R)-2-isopropylmalonamat, Smp. 60-63°,

Beispiel 74: (S)-1-[[(2R,3R oder 2S,3S)-3-(2,3,4,5,6-Pentafluorbenzyl)-2-oxetanyl]methyl]dodecyl-(R)-2-isopropylmalonamat, Smp. 107-109°,

Beispiel 75: (S)-1-[[(2R,3R oder 2S,3S)-3-(2,3,4,5,6-Pentafluorbenzyl)-2-oxetanyl]methyl]dodecyl-(S)-2-isopropylmalonamat, Smp. 115-118°,

Beispiel 76: (S)-1-[[(2S,3S oder 2R,3R)-3-(2,3,4,5,6-Pentafluorbenzyl)-2-oxetanyl]methyl]dodecyl-(R)-2-isopropylmalonamat, Smp. 88-90°,

Beispiel 77: (S)-1-[[(2S,3S oder 2R,3R)-3-(2,3,4,5,6-Pentafluorbenzyl)-2-oxetanyl]methyl]dodecyl-(S)-2-isopropylmalonamat, Smp. 52-55°.

Beispiel 78

Analog Beispiel 1 und 2 jedoch unter Verwendung von (R)- oder (S)-2-Pyrrolidon-5-carbonsäure anstatt 2-Isopropylmalonsäuremonoamid erhält man

a) 5-Oxo-D-prolin-(S)-1-[[(2S,3S)-3-hexyl-4-oxo-2-oxetanyl]methyl]dodecyl-ester, $^1$H-NMR ($CDCl_3$): 0,88 (m,6H);

1,26 (m,26H); 1,55-1,9 (m,4H); 1,95-2,55 (m,6H); 3,21 (m,1H); 4,25 (m,1H); 4,31 (m,1H); 5,14 (m,1H); 5,80 (s,1H) ppm.

b) 5-Oxo-L-prolin-(S)-1-[[(2S,3S)-3-hexyl-4-oxo-2-oxetanyl]methyl]dodecyl-ester, Smp. 51-52°

c) 5-Oxo-D-prolin-(S)-1-[[(2S,3S)-3-äthyl-4-oxo-2-oxetanyl]methyl]octadecyl-ester, Smp. 55° (Diäthyläther)

d) 5-Oxo-L-prolin-(S)-1-[[(2S,3S)-3-äthyl-4-oxetanyl]methyl]octadecyl-ester, Smp. 57-58°.

Beispiel 79

Analog Beispiel 1 und 11 erhält man aus (S)-(+)-2-Isopropylmalonsäuremonoamid und

a) aus 3R,4R(oder 3S,4S)-3-Anilino-4-[(R)-2-hydroxytridecyl]-2-oxetanon, Diastereomer B (Beispiel T) das (S)-1-[[(2R,3R oder 2S,3S)-3-Anilino-4-oxo-2-oxetanyl]methyl]dodecyl-(S)-2-isopropylmalonamat (Diastereomer B), Smp. 92°, und

b) aus 3S,4S(oder 3R,4R)-3-Anilino-4-[(R)-2-hydroxytridecyl]-2-oxetanon, Diastereomer A (Beispiel T) das (S)-1-[[(2S,3S oder 2R,3R)-3-Anilino-4-oxo-2-oxetanyl]methyl]dodecyl-(S)-2-isopropylmalonamat (Diastereomer A), Smp. 77°.

In an sich bekannter Weise werden pharmazeutische Präparat folgender Zusammensetzung hergestellt:

Beispiel A

| Weichgelatinekapseln: | |
|---|---|
| | Menge pro Kapsel |
| Ein Oxetanon der Formel I | 50 mg |
| Mittelkettiges Triglycerid | 450 µl |

Beispiel B

| Hartgelatinekapsel: | |
|---|---|
| Ein Oxetanon der Formel I | 20,0 mg |
| Milchzucker krist. | 37,0 mg |
| Mikrokristalline Cellulose | 20,0 mg |
| Polyvinylpolypyrrolidon | 8,5 mg |
| Natriumsalz des Carboxymethyläthers von Stärke | 8,5 mg |
| Talk | 4,5 mg |
| Magnesiumstearat | 1,5 mg |
| Kapselfüllgewicht | 100,0 mg |

Beispiel C

| Tablette: | |
|---|---|
| Ein Oxetanon der Formel I | 30,0 g |
| Milchzucker wasserfrei | 118,8 |
| Mikrokristalline Cellulose | 30,0 |
| Polyvinylpyrrolidon | 10,0 |
| Polymer von Carboxymethylcellulose | 10,0 |
| Magnesiumstearat | 1,2 |
| Tablettengewicht | 200,0 mg |

Beispiel D

| Tablette mit kontrollierter Wirkstoffabgabe und erhöhter Verweilzeit im Magen: | |
|---|---|
| Ein Oxetanon der Formel I | 60,0 mg |
| Milchzucker pulv. | 70,0 mg |
| Hydroxypropylmethylcellulose | 52,5 mg |
| Polyvinylpyrrolidon | 7,5 mg |
| Talk | 8,0 mg |
| Magnesiumstearat | 1,0 mg |
| kolloidale Kieselsäure | 1,0 mg |
| Kerngewicht | 200,0 mg |
| Hydroxypropylmethylcellulose | 2,5 mg |
| Talk | 1,25 mg |
| Titandioxid | 1,25 mg |
| Gewicht des Filmüberzuges | 5,0 mg |

Beispiel E

| Rekonstituierbares Pulver: | |
|---|---|
| Ein Oxetanon der Formel I | 200,0 mg |
| Aethylvanillin | 10,0 mg |
| Aspartam | 30,0 mg |
| Sprühmagermilchpulver | 4'760,0 mg |
| Total | 5'000,0 mg |

**Patentansprüche**

**Patentansprüche für folgende Vertragsstaaten : AT, BE, CH, DE, DK, FR, GB, IT, LI, LU, NL, SE**

**1.** Oxetanone der Formel

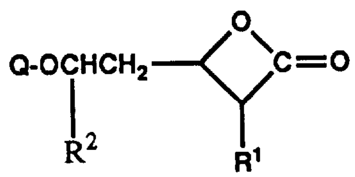

I

worin Q eine Gruppe der Formel

$$(R^3,R^4)NCO(X)n\text{-}CO\text{-} \qquad Q1$$

$$(R^3,R^4)NCO\text{-}X'\text{-} \qquad Q2$$

oder

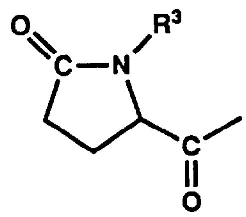

$Q^3$

ist und

$R^1$ und $R^2$ durch 1 bis 3 Halogenatome substituiertes Alkyl mit bis zu 18 C-Atomen oder gegebenenfalls durch eine 1,4-Arylengruppe unterbrochene, gegebenenfalls durch eine Arylgruppe in ω-Stellung und gegebenenfalls durch eine Aryl-$C_{1-4}$-alkylgruppe substituierte Alkyl-, Alkenyl-, Alkinyl- oder Alkadienylgruppen mit bis zu 20 C-Atomen sind, wobei $R^1$ in einer anderen als der α-Stellung zu einem ungesättigten C-Atom durch ein O- oder S-Atom oder durch eine Sulfinyl- oder Sulfonylgruppe unterbrochen sein kann, oder $R^1$ eine Gruppe Aryl-NH- oder Aryl-$C_{1-4}$-alkyl-OCONH-,

$R^3$ und $R^4$ Wasserstoff oder $C_{1-4}$-Alkyl sind oder zusammen mit dem N-Atom, an dem sie gebunden sind, einen gesättigten 3- bis 6-gliedrigen, gegebenenfalls ein O- oder S-Atom in einer anderen als der α-Stellung zum N-Atom enthaltenden Ring bilden,

n die Zahl 1 oder 0,

X eine gegebenenfalls durch ein O- oder S-Atom oder durch eine Sulfinyl- oder Sulfonylgruppe unterbrochene Alkylengruppe ist, die bis zu 6 C-Atome enthält und gegebenenfalls durch eine Hydroxy-, Mercapto-, Aryl-, Aryloxy-, Arylthio-, Aryl-$C_{1-4}$-alkyl-, Aryl-$C_{1-4}$-alkoxy-, Aryl-$C_{1-4}$-alkylthio-, Aryl-$C_{1-4}$-alkyliden-, $C_{3-7}$-Cycloalkyliden- oder $C_{1-6}$-Alkylidengruppe oder durch eine oder zwei $C_{1-6}$-Alkyl-, $C_{1-6}$-Alkoxy- oder $C_{1-6}$-Alkylthiogruppen substituiert ist, wobei zwei am gleichen C-Atom oder an zwei benachbarten C-Atomen gebundene $C_{1-6}$-Alkyl-, $C_{1-6}$-Alkoxy- oder $C_{1-6}$-Alkylthiogruppen einen gegebenenfalls monoungesättigten 3- bis 7-gliedrigen Ring bilden können und eine gegebenenfalls vorhandene Hydroxy- oder Mercaptogruppe oder ein gegebenenfalls vorhandenes ungesättigtes C-Atom in einer anderen als der α-Stellung zu einem gegebenenfalls vorhandenen O- oder S-Atom oder einer gegebenenfalls vorhandenen Sulfinyl- oder Sulfonylgruppe liegen muss, oder

X eine Gruppe der Formel

$$=CHN(R,R^{\circ}) \text{ oder } -CHN(R,R^{\circ})CH_2-$$

R und R° Wasserstoff, $C_{1-4}$-Alkyl, $C_{1-4}$-Alkyl(CO oder OCO)-, Aryl, Aryl(CO oder OCO)-, Aryl-$C_{1-4}$-alkyl oder Aryl-$C_{1-4}$-alkyl(CO oder OCO)- sind und

X' eine bis zu 6 C-Atome enthaltende Alkylengruppe ist, die durch eine $C_{1-4}$-Alkoxy-, Aryl-, Aryloxy-, Arylthio-, Aryl-$C_{1-4}$-alkyl-, Aryl-$C_{1-4}$-alkoxy- oder Aryl-$C_{1-4}$-alkylthiogruppe oder durch eine oder zwei $C_{1-6}$-Alkylgruppe(n) substituiert sein kann, wobei zwei an benachbarten C-Atome gebundene $C_{1-6}$-Alkygruppen einen 3- bis 7-gliedrigen Ring bilden können.

**2.** Oxetanone der Formel I nach Anspruch 1, worin Q eine Gruppe der Formel $Q^1$,

$R^1$ und $R^2$ gegebenenfalls durch eine 1,4-Phenylengruppe unterbrochene, gegebenenfalls durch eine Phenylgruppe in ω-Stellung und gegebenenfalls durch eine Phenyl-$C_{1-4}$-alkylgruppe substituierte Alkyl-, Alkenyl- oder Alkadienylgruppen mit bis zu 20 C-Atomen sind, wobei $R^1$ in einer anderen als der ω-Stellung zu einem ungesättigten C-Atom durch ein O- oder S-Atom unterbrochen sein kann,

X eine gegebenenfalls durch ein O- oder S-Atom unterbrochene Alkylengruppe ist, die bis zu 6 C-Atome enthält

und gegebenenfalls durch eine Hydroxy-, Mercapto-, Phenyl-, Phenoxy-, Phenylthio-, Phenyl-$C_{1-4}$-alkyl-, Phenyl-$C_{1-4}$-alkoxy-, Phenyl-$C_{1-4}$-alkylthio-, Phenyl-$C_{1-4}$-alkyliden-, $C_{3-7}$-Cycloalkyliden- oder $C_{1-6}$-Alkylidengruppe oder durch eine oder zwei $C_{1-6}$-Alkyl-, $C_{1-6}$-Alkoxy- oder $C_{1-6}$-Alkylthiogruppen substituiert ist, wobei zwei am gleichen C-Atom gebundene $C_{1-6}$-Alkyl-, $C_{1-6}$-Alkoxy- oder $C_{1-6}$-Alkyl thiogruppen einen 3- bis 7-gliedrigen Ring bilden können und eine gegebenenfalls vorhandene Hydroxy- oder Mercaptogruppe in einer anderen als der α-Stellung zu einem gegebenenfalls vorhandenen O- oder S-Atom vorliegen muss, oder
X eine Gruppe =CHN(R,R°),
R und R° Wasserstoff, $C_{1-4}$-Alkyl, $C_{1-4}$-Alkyl-(CO oder OCO)-, Phenyl oder Phenyl-(CO oder OCO)- sind und
n, $R^3$ und $R^4$ die in Anspruch 1 angegebene Bedeutung haben.

3. Oxetanone der Formel I nach Anspruch 1, worin Q eine Gruppe $Q^1$, $R^1$ und $R^2$ gegebenenfalls durch eine Arylgruppe in ω-Stellung substituierte Alkyl-, Alkenyl-, Alkinyl- oder Alkadienylgruppen mit bis zu 20 C-Atome sind, wobei $R^1$ in einer anderen als der α-Stellung zu einem ungesättigten C-Atom durch ein S-Atom unterbrochen sein kann, oder $R^1$ Anilino, durch ein Halogenatom substituiertes Alkyl mit bis zu 18 C-Atomen oder eine Gruppe Phenyl-$C_{1-4}$-alkyl-OCONH-,
$R^3$ und $R^4$ Wasserstoff oder $C_{1-4}$-Alkyl sind oder zusammen mit dem N-Atom, an dem sie gebunden sind, einen gesättigten ein O- oder S-Atom in einer anderen als der α-Stellung zum N-Atom enthaltenden 6-gliedrigen Ring bilden,
n die Zahl 1 oder 0,
X eine gegebenenfalls durch ein O- oder S-Atom oder durch eine Sulfinylgruppe unterbrochene Alkylengruppe ist, die bis zu 6 C-Atome enthält und gegebenenfalls durch eine oder zwei $C_{1-6}$-Alkyl- oder $C_{1-6}$-Alkoxygruppen substituiert ist, wobei zwei am gleichen C-Atom oder an zwei benachbarten C-Atome gebundene $C_{1-6}$-Alkyl- oder $C_{1-6}$-Alkoxygruppen einen gegebenenfalls monoungesättigten 3- bis 7-gliedrigen Ring bilden können, oder X eine Gruppe =CHN(R,R°) oder -CHN(R,R°)$CH_2$-, und
R und R° Wasserstoff, $C_{2-5}$-Alkanoyl oder Benzyloxycarbonyl sind.

4. Oxetanone nach Anspruch 3, worin $R^1$ Methyl, Aethyl, Propyl, Hexyl, 2-Butenyl, 3-Methyl-2-butenyl, 2-Propinyl, Methylthio, Pentylthio, 5-Chlorpentyl, Benzyl, Phenylthio, Benzylthio, Pentafluorbenzyl, Anilino oder Benzyloxycarbonylamino, $R^2$ Undecyl, Heptadecyl oder 8,11-Heptadecadienyl, $R^3$ und $R^4$ Wasserstoff, Methyl oder Isopropyl sind oder zusammen mit dem N-Atom eine Morpholino- oder Thiomorpholinogruppe bilden, n die Zahl 1 oder 0, und X die Gruppe $(CH2)_{1-8}$, Aethyliden, Propyliden, Isopropyliden, Butyliden, Isobutyliden, Pentyliden, Isopentyliden, t-Butylmethylen, Dimethylvinyliden, Cyclopentyliden, Cyclohexyliden, Phenäthyliden, Phenylpropyliden, 1,2-Cyclohexylen, Cyclohex-3-en-1,6-ylen, Acetamidomethylen, Benzyloxycarbonylaminomethylen, 1-Benzyloxycarbonylamino-1,2-äthylen, Methylenoxymethylen, Methylenthiomethylen, Methylensulfinylmethylen, Aethylenthioäthylen, Aethylensulfinyläthylen, Methoxymethylen oder Aethylen- oder Propylendioxymethylen sind.

5. Oxetanone der Formel I nach Anspruch 1, worin Q eine Gruppe $Q^2$; $R^1$ und $R^2$ $C_{1-20}$-Alkyl, $R^3$ und $R^4$ Wasserstoff und X' eine bis zu 6 C-Atome enthaltende Alkylengruppe, die durch eine $C_{1-4}$-Alkoxy oder durch eine oder zwei $C_{1-6}$-Alkylgruppe(n) substituiert sein kann, wobei zwei an benachbarten C-Atome gebundene $C_{1-16}$-Alkylgruppen einen 3- bis 7-gliedrigen Ring bilden können.

6. Oxetanone nach Anspruch 5, worin $R^1$ Hexyl, $R^2$ Undecyl und X' Aethylen, 1-Methoxy-1,2-äthylen oder 1,2-Cyclohexylen ist.

7. Oxetanone der Formel I nach Anspruch 1, worin Q eine Gruppe $Q^3$; $R^3$ Wasserstoff und $R^1$ und $R^2$ $C_{1-20}$-Alkyl, insbesondere Hexyl bzw. Undecyl sind.

8. Oxetanone nach Ansspruch 1 aus der Gruppe der folgenden:

(S)-1-[[(2S,3S)-3-Hexyl-4-oxo-2-oxetanyl]methyl]dodecyl-(S)-2-isopropylmalonamat,
(S)-1-[[(2S,3S)-3-Hexyl-4-oxo-2-oxetanyl]methyl]dodecyl-(S oder R)-2-carbamoylvalerat,
(all Z,S)-1-[[(2S,3S)-3-Aethyl-4-oxo-2-oxetanyl]methyl]-9,12-octadecadienyl-(S)-2-isopropylmalonamat,
(S)-1-[[(2S,3S oder 2R,3R)-4-Oxo-3-pentylthio-2-oxetanyl]methyl]dodecyl-(S)-2-isopropylmalonamat,
(S)-1-[(2S,3S) oder 2R,3R)-4-Oxo-3-pentylthio-2-oxetanyl]methyl]dodecyl-[S:R(2:1)]-2-isopropylmalonamat,
(S)-1-[[(2S,3S)-3-Aethyl-4-oxo-2-oxetanyl]methyl]octadecyl-(S oder R)-2-t-butylmalonamat,
(S)-1-[[(2S,3S)-3-Aethyl-4-oxo-2-oxetanyl]methyl]octadecyl-1-carbamoylcydopentancarboxylat,
(S)-1-[[(2S,3S)-3-Hexyl-4-oxo-2-oxetanyl]methyl]dodecyl-(RS)-2-benzylmalonamat,
(S)-1-[[(2S,3S)-3-Hexyl-4-oxo-2-oxetanyl]methyl]dodecyl-3-[(2-carbamoyl äthyl)thio]propionat,

5-Oxo-D-prolin-(S)-1-[[(2S,3S)-3-äthyl-4-oxo-2-oxetanyl]methyl]octadecyl-ester,
5-Oxo-L-prolin-(S)-1-[[(2S,3S)-3-äthyl-4-oxo-2-oxetanyl]methyl]octadecyl-ester.

**9.** Oxetanone nach Anspruch 2 aus der Gruppe der folgenden:

(S)-1-[[(2S,3S)-3-Hexyl-4-oxo-2-oxetanyl]methyl]dodecyl-(S oder R)-2-isopropylmalonamat,
(S)-1-[[(2S,3S)-3-Hexyl-4-oxo-2-oxetanyl]methyl]dodecyl-(RS)-2-carbamoylvalerat (Epimere 1:1),
(all Z,S)-1-[[(2S,3S)-3-Aethyl-4-oxo-2-oxetanyl]methyl]-9,12-octadecadienyl-(S oder R)-2-isopropylmalonamat,
(S)-1-[[(2S,3S)-3-Hexyl-4-oxo-2-oxetanyl]methyl]dodecyl-(RS)-2-carbamoyl-4-methylvalerat (Epimere 1:1),
(S)-1-[[(2S,3S)-3-Hexyl-4-oxo-2-oxetanyl]methyl]dodecyl-1-carbamoylcyclohexancarboxylat,
(S)-1-[[(2S,3S)-3-Hexyl-4-oxo-2-oxetanyl]methyl]dodecyl-(RS)-2-methylmalonamat (Epimere 1:1),
(S)-1-[[(2S,3S)-3-Hexyl-4-oxo-2-oxetanyl]methyl]dodecyl-(RS)-2-äthylmalonamat (Epimere 1:1),
(S)-1-[[(2S,3S)-3-Hexyl-4-oxo-2-oxetanyl]methyl]dodecyl-(RS)-2-butylmalonamat (Epimere 1:1),
(S)-1-[[(2S,3S)-3-Aethyl-4-oxo-2-oxetanyl]methyl]octadecyl-1-carbamoylcyclohexancarboxylat,
(S)-1-[[(2S,3S oder 2R,3R)-4-Oxo-3-pentylthio-2-oxetanyl]methyl]dodecyl-[S:R oder R:S(2:1)]-2-isopropylmalonamat,
(S)-1-[[(2R,3R)-3-Benzyl-4-oxo-2-oxetanyl]methyl]dodecyl-(S oder R)-2-isopropylmalonamat.

**10.** Oxetanone nach Anspruch 1 zur Verwendung als pharmazeutische Wirkstoffe.

**11.** Verfahren zur Herstellung der Oxetanone von Anspruch 1, dadurch gekennzeichnet, dass man

a) einen Alkohol der Formel

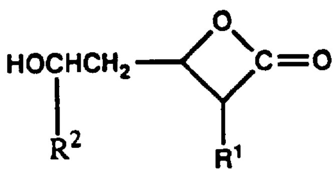

IIa

mit einer Säure der Formel $Q^a$-OH, worin $Q^a$ eine Gruppe der Formel $Q^1$ oder $Q^3$ ist, verestert oder

b) eine Säure der Formel

$$(Q\text{-}O,R^2)CHCH_2CH(OH)CH(R^1)\text{-}COOH \qquad \text{IIb}$$

cyclisiert oder

c) in einer Säure der Formel

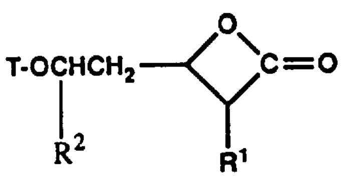

IIc

worin T eine Gruppe der Formel

$$HOCO(X)_n\text{-}CO\text{-} \qquad T^1$$

oder

$$HOCO\text{-}X'\text{-} \qquad T^2$$

ist, die Carboxygruppe in der Gruppe T in eine Amidgruppe $(R^3,R^4)$NCO-umwandelt und

d) gewünschtenfalls ein Epimerengemisch der Formel I in die einzelnen Epimeren auftrennt.

**12.** Arzneimittel, enthaltend ein Oxetanon gemäß einem der Ansprüche 1-9 und ein therapeutisch inertes Trägermaterial.

**13.** Verwendung eines Oxetanons nach einem der Ansprüche 1-9 bei der Herstellung eines Arzneimittels zur Bekämpfung oder Verhütung von Obesitas, Hyperlipämie, Atherosklerose und Arteriosklerose.

**Patentansprüche für folgenden Vertragsstaat : ES**

1. Verfahren zur Herstellung der Oxetanone der Formel

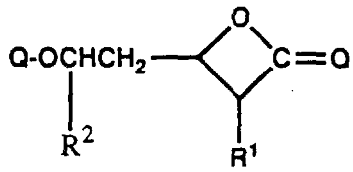

I

worin Q eine Gruppe der Formel

$$(R^3,R^4)NCO(X)n\text{-}CO\text{-} \qquad Q1$$

$$(R^3,R^4)NCO\text{-}X'\text{-} \qquad Q2$$

oder

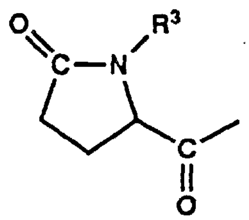

$Q^3$

ist und

$R^1$ und $R^2$ durch 1 bis 3 Halogenatome substituiertes Alkyl mit bis zu 18 C-Atomen oder gegebenenfalls durch eine 1,4-Arylengruppe unterbrochene, gegebenenfalls durch eine Arylgruppe in ω-Stellung und gegebenenfalls durch eine Aryl-$C_{1-4}$-alkylgruppe substituierte Alkyl-, Alkenyl-, Alkinyl- oder Alkadienylgruppen mit bis zu 20 C-Atomen sind, wobei $R^1$ in einer anderen als der α-Stellung zu einem ungesättigten C-Atom durch ein O- oder S-Atom oder durch eine Sulfinyl- oder Sulfonylgruppe unterbrochen sein kann, oder $R^1$ eine Gruppe Aryl-NH- oder Aryl-$C_{1-4}$-alkyl-OCONH-,

$R^3$ und $R^4$ Wasserstoff oder $C_{1-4}$-Alkyl sind oder zusammen mit dem N-Atom, an dem sie gebunden sind, einen gesättigten 3- bis 6-gliedrigen, gegebenenfalls ein O- oder S-Atom in einer anderen als der α-Stellung zum N-Atom enthaltenden Ring bilden,

n die Zahl 1 oder 0,

X eine gegebenenfalls durch ein O- oder S-Atom oder durch eine Sulfinyl- oder Sulfonylgruppe unterbrochene Alkylengruppe ist, die bis zu 6 C-Atome enthält und gegebenenfalls durch eine Hydroxy-, Mercapto-, Aryl-, Aryloxy-, Arylthio-, Aryl-$C_{1-4}$-alkyl-, Aryl-$C_{1-4}$-alkoxy-, Aryl-$C_{1-4}$-alkylthio-, Aryl-$C_{1-4}$-alkyliden-, $C_{3-7}$-Cycloalkyliden- oder $C_{1-6}$-Alkydengruppe oder durch eine oder zwei $C_{1-6}$-Alkyl-, $C_{1-6}$-Alkoxy- oder $C_{1-6}$-Alkythiogruppen substituiert ist, wobei zwei am gleichen C-Atom oder an zwei benachbarten C-Atomen gebundene $C_{1-6}$-Alkyl-, $C_{1-6}$-Alkoxy- oder $C_{1-6}$-Alkylthiogruppen einen gegebenenfalls monoungesättigten 3- bis 7-gliedrigen Ring bilden können und eine gegebenenfalls vorhandene Hydroxy- oder Mercaptogruppe oder ein gegebenenfalls vorhandenes ungesättigtes C-Atom in einer anderen als der α-Stellung zu einem gegebenenfalls vorhandenen O- oder S-Atom oder einer gegebenenfalls vorhandenen Sulfinyl- oder Sulfonylgruppe liegen muss, oder

X eine Gruppe der Formel

$$=CHN(R,R°) \text{ oder } -CHN(R,R^{o})CH_2\text{-}$$

R und R° Wasserstoff, $C_{1-4}$-Alkyl, $C_{1-4}$-Alkyl(CO oder OCO)-, Aryl, Aryl(CO oder OCO)-, Aryl-$C_{1-4}$-alkyl oder Aryl-$C_{1-4}$-alkyl(CO oder OCO)- sind und

X' eine bis zu 6 C-Atome enthaltende Alkylengruppe ist, die durch eine $C_{1-4}$-Alkoxy-, Aryl-, Aryloxy-, Arylthio-, Aryl-$C_{1-4}$-alkyl-, Aryl-$C_{1-4}$-alkoxy- oder Aryl-$C_{1-4}$-alkylthiogruppe oder durch eine oder zwei $C_{1-6}$-Alkylgruppe(n) substituiert sein kann, wobei zwei an benachbarten C-Atome gebundene $C_{1-6}$-Alkylgruppen einen 3- bis 7-gliedrigen Ring bilden können,

dadurch gekennzeichnet, dass man

a) einen Alkohol der Formel

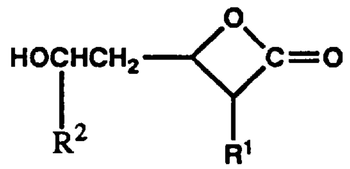

IIa

mit einer Säure der Formel $Q^a$-OH, worin $Q^a$ eine Gruppe der Formel $Q^1$ oder $Q^3$ ist, verestert oder

b) eine Säure der Formel

$$(Q\text{-}O,R^2)CHCH_2CH(OH)CH(R^1)\text{-}COOH \qquad IIb$$

cyclisiert oder

c) eine Säure der Formel

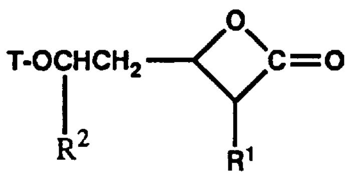

IIc

worin T eine Gruppe der Formel

$$HOCO(X)_n\text{-}CO\text{-} \qquad T^1$$

oder

$$HOCO\text{-}X'\text{-} \qquad T^2$$

ist, die Carboxygruppe in der Gruppe T in eine Amidgruppe $(R^3,R^4)NCO$-umwandelt und

d) gewünschtenfalls ein Epimerengemisch der Formel I in die einzelnen Epimeren auftrennt.

**2.** Verfahren nach Anspruch 1, worin Q eine Gruppe der Formel $Q^1$, $R^1$ und $R^2$ gegebenenfalls durch eine 1,4-Phenylengruppe unterbrochene, gegebenenfalls durch eine Phenylgruppe in ω-Stellung und gegebenenfalls durch eine Phenyl-$C_{1-4}$-alkylgruppe substituierte Alkyl-, Alkenyl- oder Alkadienylgruppen mit bis zu 20 C-Atomen sind, wobei $R^1$ in einer anderen als der α-Stellung zu einem ungesättigten C-Atom durch ein O- oder S-Atom unterbrochen sein kann,
X eine gegebenenfalls durch ein O- oder S-Atom unterbrochene Alkylengruppe ist, die bis zu 6 C-Atome enthält und gegebenenfalls durch eine Hydroxy-, Mercapto-, Phenyl-, Phenoxy-, Phenylthio-, Phenyl-$C_{1-4}$-alkyl-, Phenyl-$C_{1-4}$-alkoxy-, Phenyl-$C_{1-4}$-alkylthio-, Phenyl-$C_{1-4}$-alkyliden-, $C_{3-7}$-Cycloalkyliden- oder $C_{1-6}$-Alkylidengruppe oder durch eine oder zwei $C_{1-6}$-Alkyl-, $C_{1-6}$-Alkoxy- oder $C_{1-6}$-Alkylthiogruppen substituiert ist, wobei zwei am gleichen C-Atom gebundene $C_{1-6}$-Alkyl-, $C_{1-6}$-Alkoxy- oder $C_{1-6}$-Alkylthiogruppen einen 3- bis 7-gliedrigen Ring bilden können und eine gegebenenfalls vorhandene Hydroxy- oder Mercaptogruppe in einer anderen als der α-Stellung zu einem gegebenenfalls vorhandenen O- oder S-Atom vorliegen muss, oder
X eine Gruppe $=CHN(R,R^o)$,
R und $R^o$ Wasserstoff, $C_{1-4}$-Alkyl, $C_{1-4}$-Alkyl-(CO oder OCO)-, Phenyl oder Phenyl-(CO oder OCO)- sind und
n, $R^3$ und $R^4$ die in Anspruch 1 angegebene Bedeutung haben.

**3.** Verfahren nach Anspruch 1, worin Q eine Gruppe $Q^1$,
$R^1$ und $R^2$ gegebenenfalls durch eine Arylgruppe in ω-Stellung substituierte Alkyl-, Alkenyl-, Alkinyl- oder Alkadienylgruppen mit bis zu 20 C-Atomen sind, wobei $R^1$ in einer anderen als der α-Stellung zu einem ungesättigten C-Atom durch ein S-Atom unterbrochen sein kann, oder
$R^1$ Anilino, durch ein Halogenatom substituiertes Alkyl mit bis zu 18 C-Atomen oder eine Gruppe Phenyl-$C_{1-4}$-alkyl-OCONH-,
$R^3$ und $R^4$ Wasserstoff oder $C_{1-4}$-Alkyl sind oder zusammen mit dem N-Atom, an dem sie gebunden sind, einen gesättigten ein O- oder S-Atom in einer anderen als der α-Stellung zum N-Atom enthaltenden 6-gliedrigen Ring bilden,
n die Zahl 1 oder 0,

X eine gegebenenfalls durch ein O- oder S-Atom oder durch eine Sulfinylgruppe unterbrochene Alkylengruppe ist, die bis zu 6 C-Atome enthält und gegebenenfalls durch eine oder zwei $C_{1-6}$-Alkyl- oder $C_{1-6}$-Alkoxygruppen substituiert ist, wobei zwei am gleichen C-Atom oder an zwei benachbarten C-Atome gebundene $C_{1-6}$-Alkyl- oder $C_{1-6}$-Alkoxygruppen einen gegebenenfalls monoungesättigten 3- bis 7-gliedrigen Ring bilden können, oder X eine Gruppe =CHN(R,R°) oder -CHN(R,R°)$CH_2$-, und
R und R° Wasserstoff, $C_{2-5}$-Alkanoyl oder Benzyloxycarbonyl sind.

**4.** Verfahren nach Anspruch 3, worin $R^1$ Methyl, Aethyl, Propyl, Hexyl, 2-Butenyl, 3-Methyl-2-butenyl, 2-Propinyl, Methylthio, Pentylthio, 5-Chlorpentyl, Benzyl, Phenylthio, Benzylthio, Pentafluorbenzyl, Anilino oder Benzyloxycarbonylamino, $R^2$ Undecyl, Heptadecyl oder 8,11-Heptadecadienyl, $R^3$ und $R^4$ Wasserstoff, Methyl oder Isopropyl sind oder zusammen mit dem N-Atom eine Morpholino- oder Thiomorpholinogruppe bilden, n die Zahl 1 oder 0, und X die Gruppe $(CH_2)_{1-8}$, Aethyliden, Propyliden, Isopropyliden, Butyliden, Isobutyliden, Pentyliden, Isopentyliden, t-Butylmethylen, Dimethylvinyliden, Cyclopentyliden, Cyclohexyliden, Phenäthyliden, Phenylpropyliden, 1,2-Cyclohexylen, Cydohex-3,4-en-1,6-ylen, Acetamidomethylen, Benzyloxycarbonylaminomethylen, 1-Benzyloxycarbonylamino-1,2-äthylen, Methylenoxymethylen, Methylenthiomethylen, Methylensulfinylmethylen, Aethylenthioäthylen, Aethylensulfinyläthylen, Methoxymethylen oder Aethylen- oder Propylendioxymethylen sind.

**5.** Verfahren nach Anspruch 1, worin Q eine Gruppe $Q^2$; $R^1$ und $R^2$ $C_{1-20}$-Alkyl, $R^3$ und $R^4$ Wasserstoff und X' eine bis zu 6 C-Atome enthaltende Alkylengruppe, die durch eine $C_{1-4}$-Alkoxy oder durch eine oder zwei $C_{1-6}$-Alkylgruppe(n) substituiert sein kann, wobei zwei an benachbarten C-Atome gebundene $C_{1-6}$-Alkylgruppen einen 3- bis 7-gliedrigen Ring bilden können.

**6.** Verfahren nach Anspruch 5, worin $R^1$ Hexyl, $R^2$ Undecyl und X' Aethylen, 1-Methoxy-1,2-äthylen oder 1,2-Cydohexylen ist.

**7.** Verfahren nach Anspruch 1, worin Q eine Gruppe $Q^3$; $R^3$ Wasserstoff und $R^1$ und $R^2$ $C_{1-20}$-Alkyl, insbesondere Hexyl bzw. Undecyl sind.

**8.** Verfahren nach Anspruch 1, dadurch gekennzeichnet, dass man Verbindungen aus der Gruppe der folgenden herstellt:

(S)-1-[[(2S,3S)-3-Hexyl-4-oxo-2-oxetanyl]methyl]dodecyl-(S)-2-isopropylmalonamat,
(S)-1-[[(2S,3S)-3-Hexyl-4-oxo-2-oxetanyl]methyl]dodecyl-(S oder R)-2-carbamoylvalerat,
(all Z,S)-1-[[(2S,3S)-3-Aethyl-4-oxo-2-oxetanyl]methyl]-9,12-octadecadienyl. (S)-2-isopropylmalonamat,
(S)-1-[[(2S,3S oder 2R,3R)-4-Oxo-3-pentylthio-2-oxetanyl]methyl]dodecyl-(S)-2-isopropylmalonamat,
(S)-1-[(2S,3S) oder 2R,3R)-4-Oxo-3-pentylthio-2-oxetanyl]methyl]dodecyl-[S:R(2:1)]-2-isopropylmalonamat,
(S)-1-[[(2S,3S)-3-Aethyl-4-oxo-2-oxetanyl]methyl]octadecyl-(S oder R)-2-t-butylmalonamat,
(S)-1-[[(2S,3S)-3-Aethyl-4-oxo-2-oxetanyl]methyl]octadecyl-1-carbamoylcyclopentancarboxylat,
(S)-1-[[(2S,3S)-3-Hexyl-4-oxo-2-oxetanyl]methyl]dodecyl-(RS)-2-benzylmalonamat,
(S)-1-[[(2S,3S)-3-Hexyl-4-oxo-2-oxetanyl]methyl]dodecyl-3-[(2-carbamoyläthyl)thio]propionat,
5-Oxo-D-prolin-(S)-1-[[(2S,3S)-3-äthyl-4-oxo-2-oxetanyl]methyl]octadecylester,
5-Oxo-L-prolin-(S)-1-[[(2S,3S)-3-äthyl-4-oxo-2-oxetanyl]methyl]octadecylester.

**9.** Verfahren nach Anspruch 2, dadurch gekennzeichnet, dass man aus der Gruppe der folgenden herstellt:

(S)-1-[[(2S,3S)-3-Hexyl-4-oxo-2-oxetanyl]methyl]dodecyl-(S oder R)-2-isopropylmalonamat,
(S)-1-[[(2S,3S)-3-Hexyl-4-oxo-2-oxetanyl]methyl]dodecyl-(RS)-2-carbamoylvalerat (Epimere 1:1),
(all Z,S)-1-[[(2S,3S)-3-Aethyl-4-oxo-2-oxetanyl]methyl]-9,12-octadecadienyl-(S oder R)-2-isopropylmalonamat,
(S)-1-[[(2S,3S)-3-Hexyl-4-oxo-2-oxetanyl]methyl]dodecyl-(RS)-2-carbamoyl-4-methylvalerat (Epimere 1:1),
(S)-1-[[(2S,3S)-3-Hexyl-4-oxo-2-oxetanyl]methyl]dodecyl-1-carbamoylcyclohexancarboxylat,
(S)-1-[[(2S,3S)-3-Hexyl-4-oxo-2-oxetanyl]methyl]dodecyl-(RS)-2-methylmalonamat (Epimere 1:1),
(S)-1-[[(2S,3S)-3-Hexyl-4-oxo-2-oxetanyl]methyl]dodecyl-(RS)-2-äthylmalonamat (Epimere 1:1),
(S)-1-[[(2S,3S)-3-Hexyl-4-oxo-2-oxetanyl]methyl]dodecyl-(RS)-2-butylmalonamat (Epimere 1:1),
(S)-1-[[(2S,3S)-3-Aethyl-4-oxo-2-oxetanyl]methyl]octadecyl-1-carbamoylcydohexancarboxylat,
(S)-1-[[(2S,3S oder 2R,3R)-4-Oxo-3-pentylthio-2-oxetanyl]methyl]dodecyl-[S:R oder R:S(2:1)]-2-isopropylmalonamat,
(S)-1-[[(2R,3R)-3-Benzyl-4-oxo-2-oxetanyl]methyl]dodecyl-(S oder R)-2-isopropylmalonamat.

**10.** Verwendung eines Oxetanons nach Anspruch 1 bei der Herstellung eines Arzneimittels zur Bekämpfung oder Verhütung von Obesitas, Hyperlipämie, Atherosklerose und Arteriosklerose.

**Patentansprüche für folgenden Vertragsstaat : GR**

**1.** Verfahren zur Herstellung der Oxetanone der Formel

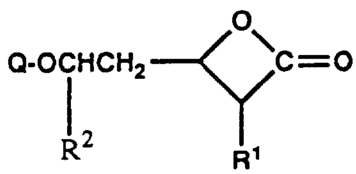

I

worin Q eine Gruppe der Formel

$(R^3,R^4)NCO(X)n\text{-}CO\text{-}$ Q1

$(R^3,R^4)NCO\text{-}X'\text{-}$ Q2

oder

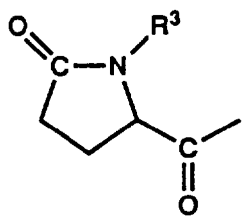

$Q^3$

ist und

$R^1$ und $R^2$ durch 1 bis 3 Halogenatome substituiertes Alkyl mit bis zu 18 C-Atomen oder gegebenenfalls durch eine 1,4-Arylengruppe unterbrochene, gegebenenfalls durch eine Arylgruppe in ω-Stellung und gegebenenfalls durch eine Aryl-$C_{1-4}$-alkygruppe substituierte Alkyl-, Alkenyl-, Alkinyl- oder Alkadienylgruppen mit bis zu 20 C-Atomen sind, wobei $R^1$ in einer anderen als der α-Stellung zu einem ungesättigten C-Atom durch ein O- oder S-Atom oder durch eine Sulfinyl- oder Sulfonylgruppe unterbrochen sein kann, oder $R^1$ eine Gruppe Aryl-NH- oder Aryl-$C_{1-4}$-alkyl-OCONH-,

$R^3$ und $R^4$ Wasserstoff oder $C_{1-4}$-Alkyl sind oder zusammen mit dem N-Atom, an dem sie gebunden sind, einen gesättigten 3- bis 6-gliedrigen, gegebenenfalls ein O- oder S-Atom in einer anderen als der α-Stellung zum N-Atom enthaltenden Ring bilden, n die Zahl 1 oder 0,

X eine gegebenenfalls durch ein O- oder S-Atom oder durch eine Sulfinyl- oder Sulfonylgruppe unterbrochene Alkylengruppe ist, die bis zu 6 C-Atome enthält und gegebenenfalls durch eine Hydroxy-, Mercapto-, Aryl-, Aryloxy-, Arylthio-, Aryl-$C_{1-4}$-alkyl-, Aryl-$C_{1-4}$-alkoxy-, Aryl-$C_{1-4}$-alkylthio-, Aryl-$C_{1-4}$-alkyliden-, $C_{3-7}$-Cycloalkyliden- oder $C_{1-6}$-Alkylidengruppe oder durch eine oder zwei $C_{1-6}$-Alkyl-, $C_{1-6}$-Alkoxy- oder $C_{1-6}$-Alkylthiogruppen substituiert ist,

wobei zwei am gleichen C-Atom oder an zwei benachbarten C-Atomen gebundene $C_{1-6}$-Alkyl-, $C_{1-6}$-Alkoxy- oder $C_{1-6}$-Alkylthiogruppen einen gegebenenfalls monoungesättigten 3- bis 7-gliedrigen Ring bilden können und eine gegebenenfalls vorhandene Hydroxy- oder Mercaptogruppe oder ein gegebenenfalls vorhandenes ungesättigtes C-Atom in einer anderen als der α-Stellung zu einem gegebenenfalls vorhandenen O- oder S-Atom oder einer gegebenenfalls vorhandenen Sulfinyl- oder Sulfonylgruppe liegen muss, oder

X eine Gruppe der Formel

$$=CHN(R,R^\circ) \text{ oder } -CHN(R,R^\circ)CH_2-$$

R und R° Wasserstoff, $C_{1-4}$-Alkyl, $C_{1-4}$-Alkyl(CO oder OCO)-, Aryl, Aryl(CO oder OCO)-, Aryl-$C_{1-4}$-alkyl oder Aryl-$C_{1-4}$-alkyl(CO oder OCO)- sind und

X' eine bis zu 6 C-Atome enthaltende Alkylengruppe ist, die durch eine $C_{1-4}$-Alkoxy-, Aryl-, Aryloxy-, Arylthio-, Aryl-$C_{1-4}$-alkyl-, Aryl-$C_{1-4}$-alkoxy- oder Aryl-$C_{1-4}$-alkylthiogruppe oder durch eine oder zwei $C_{1-6}$-Alkylgruppe(n) substituiert sein kann, wobei zwei an benachbarten C-Atome gebundene $C_{1-6}$-Alkygruppen einen 3- bis 7-gliedrigen Ring bilden können,

dadurch gekennzeichnet, dass man

a) einen Alkohol der Formel

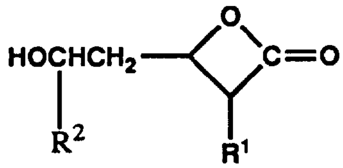

IIa

mit einer Säure der Formel $Q^a$-OH, worin $Q^a$ eine Gruppe der Formel $Q^1$ oder $Q^3$ ist, verestert oder

b) eine Säure der Formel

$$(Q\text{-}O,R^2)CHCH_2CH(OH)CH(R^1)\text{-}COOH \qquad IIb$$

cyclisiert oder

c) eine Säure der Formel

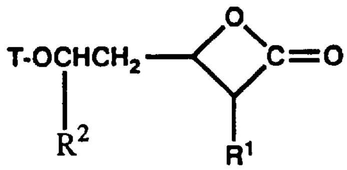

IIc

worin T eine Gruppe der Formel

$$HOCO(X)_n\text{-}CO\text{-} \qquad T^1$$

oder

$$HOCO\text{-}X'\text{-} \qquad T^2$$

ist, die Carboxygruppe in der Gruppe T in eine Amidgruppe ($R^3$,$R^4$)NCO-umwandelt und

d) gewünschtenfalls ein Epimerengemisch der Formel I in die einzelnen Epimeren auftrennt.

**2.** Verfahren nach Anspruch 1, worin Q eine Gruppe der Formel $Q^1$, $R^1$ und $R^2$ gegebenenfalls durch eine 1,4-Phenylengruppe unterbrochene, gegebenenfalls durch eine Phenylgruppe in ω-Stellung und gegebenenfalls durch eine Phenyl-$C_{1-4}$-alkylgruppe substituierte Alkyl-, Alkenyl- oder Alkadienylgruppen mit bis zu 20 C-Atomen sind, wobei $R^1$ in einer anderen als der ω-Stellung zu einem ungesättigten C-Atom durch ein O- oder S-Atom unterbrochen sein kann,
X eine gegebenenfalls durch ein O- oder S-Atom unterbrochene Alkylengruppe ist, die bis zu 6 C-Atome enthält und gegebenenfalls durch eine Hydroxy-, Mercapto-, Phenyl-, Phenoxy-, Phenylthio-, Phenyl-$C_{1-4}$-alkyl-, Phenyl-$C_{1-4}$-alkoxy-, Phenyl-$C_{1-4}$-alkylthio-, Phenyl-$C_{1-4}$-alkyliden-, $C_{3-7}$-Cycloalkyliden- oder $C_{1-6}$-Alkylidengruppe oder durch eine oder zwei $C_{1-6}$-Alkyl-, $C_{1-6}$-Alkoxy- oder $C_{1-6}$-Alkylthiogruppen substituiert ist, wobei zwei am gleichen C-Atom gebundene $C_{1-6}$-Alkyl-, $C_{1-6}$-Alkoxy- oder $C_{1-6}$-Alkylthiogruppen einen 3- bis 7-gliedrigen Ring bilden können und eine gegebenenfalls vorhandene Hydroxy- oder Mercaptogruppe in einer anderen als der α-Stellung zu einem gegebenenfalls vorhandenen O- oder S-Atom vorliegen muss, oder
X eine Gruppe =CHN(R,R°),
R und R° Wasserstoff, $C_{1-4}$-Alkyl, $C_{1-4}$-Alkyl-(CO oder OCO)-, Phenyl oder Phenyl-(CO oder OCO)- sind und
n, $R^3$ und $R^4$ die in Anspruch 1 angegebene Bedeutung haben.

**3.** Verfahren nach Anspruch 1, worin Q eine Gruppe $Q^1$,
$R^1$ und $R^2$ gegebenenfalls durch eine Arylgruppe in ω-Stellung substituierte Alkyl-, Alkenyl-, Alkinyl- oder Alkadienylgruppen mit bis zu 20 C-Atomen sind, wobei $R^1$ in einer anderen als der α-Stellung zu einem ungesättigten C-Atom durch ein S-Atom unterbrochen sein kann, oder
$R^1$ Anilino, durch ein Halogenatom substituiertes Alkyl mit bis zu 18 C-Atomen oder eine Gruppe Phenyl-$C_{1-4}$-alkyl-OCONH-,
$R^3$ und $R^4$ Wasserstoff oder $C_{1-4}$-Alkyl sind oder zusammen mit dem N-Atom, an dem sie gebunden sind, einen gesättigten ein O- oder S-Atom in einer anderen als der α-Stellung zum N-Atom enthaltenden 6-gliedrigen Ring bilden,
n die Zahl 1 oder 0,
X eine gegebenenfalls durch ein O- oder S-Atom oder durch eine Sulfinylgruppe unterbrochene Alkylengruppe ist,

die bis zu 6 C-Atome enthält und gegebenenfalls durch eine oder zwei $C_{1-6}$-Alkyl- oder $C_{1-6}$-Alkoxygruppen substituiert ist, wobei zwei am gleichen C-Atom oder an zwei benachbarten C-Atome gebundene $C_{1-6}$-Alkyl- oder $C_{1-6}$-Alkoxygruppen einen gegebenenfalls monoungesättigten 3- bis 7-gliedrigen Ring bilden können, oder
X eine Gruppe =CHN(R,R°) oder -CHN(R,R°)$CH_2$-, und
R und R° Wasserstoff, $C_{2-5}$-Alkanoyl oder Benzyloxycarbonyl sind.

**4.** Verfahren nach Anspruch 3, worin $R^1$ Methyl, Aethyl, Propyl, Hexyl, 2-Butenyl, 3-Methyl-2-butenyl, 2-Propinyl, Methylthio, Pentylthio, 5-Chlorpentyl, Benzyl, Phenylthio, Benzylthio, Pentafluorbenzyl, Anilino oder Benzyloxycarbonylamino, $R^2$ Undecyl, Heptadecyl oder 8,11-Heptadecadienyl, $R^3$ und $R^4$ Wasserstoff, Methyl oder Isopropyl sind oder zusammen mit dem N-Atom eine Morpholino- oder Thiomorpholinogruppe bilden, n die Zahl 1 oder 0, und X die Gruppe $(CH_2)_{1-8}$, Aethyliden, Propyliden, Isopropyliden, Butyliden, Isobutyliden, Pentyliden, Isopentyliden, t-Butylmethylen, Dimethylvinyliden, Cyclopentyliden, Cyclohexyliden, Phenäthyliden, Phenylpropyliden, 1,2-Cyclohexylen, Cyclohex-3,4-en-1,6-ylen, Acetamidomethylen, Benzyloxycarbonylaminomethylen, 1-Benzyloxycarbonylamino-1,2-äthylen, Methylenoxymethylen, Methylenthiomethylen, Methylensulfinylmethylen, Aethylenthioäthylen, Aethylensulfinyläthylen, Methoxymethylen oder Aethylen- oder Propylendioxymethylen sind.

**5.** Verfahren nach Anspruch 1, worin Q eine Gruppe $Q^2$; $R^1$ und $R^2$ $C_{1-20}$-Alkyl, $R^3$ und $R^4$ Wasserstoff und X' eine bis zu 6 C-Atome enthaltende Alkylengruppe, die durch eine $C_{1-4}$-Alkoxy oder durch eine oder zwei $C_{1-6}$-Alkylgruppe(n) substituiert sein kann, wobei zwei an benachbarten C-Atome gebundene $C_{1-6}$-Alkylgruppen einen 3- bis 7-gliedrigen Ring bilden können.

**6.** Verfahren nach Anspruch 5, worin $R^1$ Hexyl, $R^2$ Undecyl und X' Aethylen, 1-Methoxy-1,2-äthylen oder 1,2-Cyclohexylen ist.

**7.** Verfahren nach Anspruch 1, worin Q eine Gruppe $Q^3$; $R^3$ Wasserstoff und $R^1$ und $R^2$ $C_{1-20}$-Alkyl, insbesondere Hexyl bzw. Undecyl sind.

**8.** Verfahren nach Anspruch 1, dadurch gekennzeichnet, dass man Verbindungen aus der Gruppe der folgenden herstellt:

(S)-1-[[(2S,3S)-3-Hexyl-4-oxo-2-oxetanyl]methyl]dodecyl-(S)-2-isopropylmalonamat,
(S)-1-[[(2S,3S)-3-Hexyl-4-oxo-2-oxetanyl]methyl]dodecyl-(S oder R)-2-carbamoylvalerat,
(all Z,S)-1-[[(2S,3S)-3-Aethyl-4-oxo-2-oxetanyl]methyl]-9,12-octadecadienyl-(S)-2-isopropylmalonamat,
(S)-1-[[(2S,3S oder 2R,3R)-4-Oxo-3-penty1thio-2-oxetanyl]methyl]dodecyl-(S)-2-isopropylmalonamat,
(S)-1-[(2S,3S) oder 2R,3R)-4-Oxo-3-pentylthio-2-oxetanyl]methyl]dodecyl-[S:R(2:1)]-2-isopropylmalonamat,
(S)-1-[[(2S,3S)-3-Aethyl-4-oxo-2-oxetanyl]methyl]octadecyl-(S oder R)-2-t-butylmalonamat,
(S)-1-[[(2S,3S)-3-Aethyl-4-oxo-2-oxetanyl]methyl]octadecyl-1-carbamoylcyclopentancarboxylat,
(S)-1-1[(2S,3S)-3-Hexyl-4-oxo-2-oxetanyl]methyl]dodecyl-(RS)-2-benzylmalonamat,
(S)-1-[[(2S,3S)-3-Hexyl-4-oxo-2-oxetanyl]methyl]dodecyl-3-[(2-carbamoyläthyl)thio]propionat,
5-Oxo-D-prolin-(S)-1-[[(2S,3S)-3-äthyl-4-oxo-2-oxetanyl]methyl]octadecylester,
5-Oxo-L-prolin-(S)-1-[[(2S,3S)-3-äthyl-4-oxo-2-oxetanyl]methyl]octadecylester.

**9.** Verfahren nach Anspruch 2, dadurch gekennzeichnet, dass man aus der Gruppe der folgenden herstellt:

(S)-1-[[(2S,3S)-3-Hexyl-4-oxo-2-oxetanyl]methyl]dodecyl-(S oder R)-2-isopropylmalonamat,
(S)-1-[[(2S,3S)-3-Hexyl-4-oxo-2-oxetanyl]methyl]dodecyl-(RS)-2-carbamoylvalerat (Epimere 1:1),
(all Z,S)-1-[[(2S,3S)-3-Aethyl-4-oxo-2-oxetanyl]methyl]-9,12-octadecadienyl-(S oder R)-2-isopropylmalonamat,
(S)-1-[[(2S,3S)-3-Hexyl-4-oxo-2-oxetanyl]methyl]dodecyl-(RS)-2-carbamoyl-4-methylvalerat (Epimere 1:1),
(S)-1-[[(2S,3S)-3-Hexyl-4-oxo-2-oxetanyl]methyl]dodecyl-1-carbamoylcyclohexancarboxylat,
(S)-1-[[(2S,3S)-3-Hexyl-4-oxo-2-oxetanyl]methyl]dodecyl-(RS)-2-methylmalonamat (Epimere 1:1),
(S)-1-[[(2S,3S)-3-Hexyl-4-oxo-2-oxetanyl]methyl]dodecyl-(RS)-2-äthylmalonamat (Epimere 1:1),
(S)-1-[[(2S,3S)-3-Hexyl-4-oxo-2-oxetanyl]methyl]dodecyl-(RS)-2-butylmalonamat (Epimere 1:1),
(S)-1-[[(2S,3S)-3-Aethyl-4-oxo-2-oxetanyl]methyl]octadecyl-1-carbamoylcyclohexancarboxylat,
(S)-1-[[(2S,3S oder 2R,3R)-4-Oxo-3-pentylthio-2-oxetanyl]methyl]dodecyl-[S:R oder R:S(2:1)]-2-isopropylmalonamat,
(S)-1-[[(2R,3R)-3-Benzyl-4-oxo-2-oxetanyl]methyl]dodecyl-(S oder R)-2-isopropylmalonamat.

**10.** Verfahren zur Herstellung von Arzneimitteln, insbesondere zur Bekämpfung oder Verhütung von Obesitas, Hyperlipämie, Atherosklerose und Arteriosklerose, dadurch gekennzeichnet, dass man ein Oxetanon der Formel I und erwünschtenfalls eine oder mehrere andere therapeutisch wertvolle Stoffe in eine galenische Darreichungsform bringt.

**11.** Verwendung eines Oxetanons nach Anspruch 1 bei der Herstellung eines Arzneimittels zur Bekämpfung oder Verhütung von Obesitas, Hyperlipämie, Atherosklerose und Arteriosklerose.

## Claims

**Claims for the following Contracting States : AT, BE, CH, DE, DK, FR, GB, IT, LI, LU, NL, SE**

**1.** Oxetanones of the formula

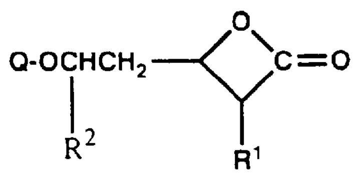

I

wherein Q is a group of the formula

$(R^3,R^4)NCO(X)_n$-CO- $Q^1$

$(R^3,R^4)$NCO-X'- $Q^2$

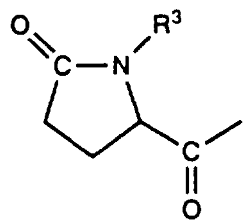

$Q^3$

and
$R^1$ and $R^2$ are alkyl with up to 18 C atoms substituted by 1 to 3 halogen atoms or alkyl, alkenyl, alkynyl or alkadienyl groups with up to 20 C atoms optionally interrupted by a 1,4-arylene group, optionally substituted by an aryl group in the ω-position and optionally substituted by an aryl-$C_{1-4}$-alkyl group, whereby $R^1$ can be interrupted by an O or S atom or by a sulphinyl or sulphonyl group in a position other than the α-position to an unsaturated C atom, or $R^1$ is an aryl-NH- or aryl-$C_{1-4}$-alkyl-OCONH- group,
$R^3$ and $R^4$ are hydrogen or $C_{1-4}$-alkyl or together with the N atom to which they are attached form a saturated 3- to 6-membered ring optionally containing an O or S atom in a position other than the α-position to the N atom,
n is the number 1 or 0,
X is an alkylene group, which contains up to 6 C atoms, which is optionally interrupted by an O or S atom or by a sulphinyl or sulphonyl group and which is optionally substituted by a hydroxy, mercapto, aryl, aryloxy, arylthio, aryl-$C_{1-4}$-alkyl, aryl-$C_{1-4}$-alkoxy, aryl-$C_{1-4}$-alkylthio, aryl-$C_{1-4}$-alkylidene, $C_{3-7}$-cycloalkylidene or $C_{1-6}$-alkylidene group or by one or two $C_{1-6}$-alkyl, $C_{1-6}$-alkoxy or $C_{1-6}$-alkylthio groups, whereby two $C_{1-6}$-alkyl, $C_{1-6}$-alkoxy or $C_{1-6}$-alkylthio groups on the same C atom or on two adjacent C atoms can form an optionally mono-unsaturated 3- to 7-membered ring and an optionally present hydroxy or mercapto group or an optionally present unsaturated C atom must be in a position other than the α-position to an optionally present O or S atom or to an optionally present sulphinyl or sulphonyl group, or
X is a group of the formula

$$=CHN(R,R^o)\text{or}-CHN(R,R^o)CH_2-$$

R and R° are hydrogen, $C_{1-4}$-alkyl, $C_{1-4}$-alkyl(CO or OCO)-, aryl, aryl(CO or OCO)-, aryl-$C_{1-4}$-alkyl or aryl-$C_{1-4}$-alkyl (CO or OCO)- and
X' is an alkylene group containing up to 6 C atoms which can be substituted by a $C_{1-4}$-alkoxy, aryl, aryloxy, arylthio,

aryl-$C_{1-4}$-alkyl, aryl-$C_{1-4}$-alkoxy or aryl-$C_{1-4}$-alkylthio group or by one or two $C_{1-6}$-alkyl groups, whereby two $C_{1-6}$-alkyl groups attached to adjacent C atoms can form a 3- to 7-membered ring.

**2.** Oxetanones of formula I according to claim 1, wherein Q is a group of the formula $Q^1$,
$R^1$ and $R^2$ are alkyl, alkenyl or alkadienyl groups with up to 20 C atoms optionally interrupted by a 1,4-phenylene group, optionally substituted by a phenyl group in the ω-position and optionally substituted by a phenyl-$C_{1-4}$-alkyl group, whereby $R^1$ can be interrupted by an O or S atom in a position other than the α-position to an unsaturated C atom,
X is an alkylene group, which contains up to 6 C atoms, which is optionally interrupted by an O or S atom and which is optionally substituted by a hydroxy, mercapto, phenyl, phenoxy, phenylthio, phenyl-$C_{1-4}$-alkyl, phenyl-$C_{1-4}$-alkoxy, phenyl-$C_{1-4}$-alkylthio, phenyl-$C_{1-4}$-alkylidene, $C_{3-7}$-cycloaykyoidene or $C_{1-6}$-alkylidene group or by one or two $C_{1-6}$-alkyl, $C_{1-6}$-alkoxy or $C_{1-6}$-alkylthio groups, whereby two $C_{1-6}$-alkyl, $C_{1-6}$-alkoxy or $C_{1-6}$-alkylthio groups attached to the same C atom can form a 3- to 7-membered ring and an optionally present hydroxy or mercapto group must be in a position other than the α-position to an optionally present O or S atom, or
X is a group =CHN(R,R°),
R and R° are hydrogen, $C_{1-4}$-alkyl, $C_{1-4}$-alkyl-(CO or OCO)-, phenyl or phenyl-(CO or OCO)- and
n, $R^3$ and $R^4$ have the significance given in claim 1.

**3.** Oxetanones of formula I according to claim 1, wherein Q is a group $Q^1$,
$R^1$ and $R^2$ are alkyl, alkenyl, alkynyl or alkadienyl groups with up to 20 C atoms optionally substituted by an aryl group in the ω-position, whereby $R^1$ can be interrupted by a S atom in a position other than the α-position to an unsaturated C atom, or
$R^1$ is anilino, alkyl with up to 20 C atoms substituted by a halogen atom or a phenyl-$C_{1-4}$-alkyl-OCONH- group,
$R^3$ and $R^4$ are hydrogen or $C_{1-4}$-alkyl or together with the N atoms to which they are attached form a saturated 6-membered ring containing an O or S atom in a position other than the α-position to the N atom, n is the number 1 or 0,
X is an alkylene group, which contains up to 6 C atoms, which is optionally interrupted by an O or S atom or by a sulphinyl group and which is optionally substituted by one or two $C_{1-6}$-alkyl or $C_{1-6}$-alkoxy groups, whereby two $C_{1-6}$-alkyl or $C_{1-6}$-alkoxy groups attached to the same C atom or to two adjacent C atoms can form an optionally mono-unsaturated 3- to 7-membered ring,
or
X is a group =CHN(R,R°) or -CHN(R,R°)$CH_2$- and
R and R° are hydrogen, $C_{2-5}$-alkanoyl or benzyloxycarbonyl.

**4.** Oxetanones according to claim 3, wherein $R^1$ is methyl, ethyl, propyl, hexyl, 2-butenyl, 3-methyl-2-butenyl, 2-propynyl, methylthio, pentylthio, 5-chloropentyl, benzyl, phenyl thio, benzylthio, pentafluorobenzyl, anilino or benzyloxycarbonylamino, $R^2$ is undecyl, heptadecyl or 8,11-heptadecadienyl, $R^3$ and $R^4$ are hydrogen, methyl or isopropyl or together with the N atom form a morpholino or thiomorpholino group, n is the number 1 or 0 and X is the group $(CH_2)_{1-8}$, ethylidene, propylidene, isopropylidene, butylidene, isobutylidene, pentylidene, isopentylidene, t-butylmethylene dimethylvinylidene, cyclopentylidene, cyclohexylidene, phenethylidene, phenylpropylidene, 1,2-cyclohexylene, cyclohex-3-en-1,6-ylene, acetamidomethylene, benzyloxycarbonylaminomethylene, 1-benzyloxycarbonylamino-1,2-ethylene, methyleneoxymethylene, methylenethiomethylene, methylenesulphinylmethylene, ethylenethioethylene, ethylenesulphinylethylene, methoxymethylene or ethylene- or propylenedioxymethylene.

**5.** Oxetanones of formula I according to claim 1, wherein Q is a group $Q^2$; $R^1$ and $R^2$ are $C_{1-20}$-alkyl, $R^3$ and $R^4$ are hydrogen and X' is an alkylene group containing up to 6 C atoms which can be substituted by a $C_{1-4}$-alkoxy group or by one or two $C_{1-6}$-alkyl groups, whereby two $C_{1-6}$-alkyl groups attached to adjacent C atoms can form a 3- to 7-membered ring.

**6.** Oxetanones according to claim 5, wherein $R^1$ is hexyl, $R^2$ is undecyl and X' is ethylene, 1-methoxy-1,2-ethylene or 1,2-cyclohexylene.

**7.** Oxetanones of formula I according to claim 1, wherein Q is a group $Q^3$; $R^3$ is hydrogen and $R^1$ and $R^2$ are $C_{1-20}$-alkyl, especially hexyl or undecyl.

**8.** Oxetanones according to claim 1 from the following group:

(S)-1-[[(2S,3S)-3-Hexyl-4-oxo-2-oxetanyl]methyl]dodecyl (S)-2-isopropylmalonamate,
(S)-1-[[(2S,3S)-3-hexyl-4-oxo-2-oxetanyl]methyl]dodecyl (S or R)-2-carbamoylvalerate,
(all Z,S)-1-[[(2S,3S)-3-ethyl-4-oxo-2-oxetanyl]methyl]-9,12-octadecadienyl (S)-2-isopropylmalonamate,
(S)-1-[[(2S,3S or 2R,3R)-4-oxo-3-pentylthio-2-oxetanyl]-methyl]dodecyl (S)-2-isopropylmalonamate,
(S)-1-[(2S,3S) or 2R,3R)-4-oxo-3-pentylthio-2-oxetanyl]-methyl]dodecyl [S:R(2:1)]-2-isopropylmalonamate,
(S)-1[[(2S,3S)-3-ethyl-4-oxo-2-oxetanyl]methyl]octadecyl (S or R)-2-t-butylmalonamate,
(S)-1[[(2S,3S)-3-ethyl-4-oxo-2-oxetanyl]methyl]octadecyl 1-carbamoylcyclopentanecarboxylate,
(S)-1-[[(2S,3S)-3-hexyl-4-oxo-2-oxetanyl]methyl]dodecyl (RS)-2-benzylmalonamate,
(S)-1-[[(2S,3S)-3-hexyl-4-oxo-2-oxetanyl]methyl]dodecyl 3-[(2-carbamoylethyl)thio]propionate,
5-oxo-D-proline (S)-1-[[(2S,3S)-3-ethyl-4-oxo-2-oxetanyl]methyl]octadecyl ester,
5-oxo-L-proline (S)-1-[[(2S,3S)-3-ethyl-4-oxo-2-oxetanyl]methyl]octadecyl ester.

**9.** Oxetanones according to claim 2 from the following group:

(S)-1-[[(2S,3S)-3-Hexyl-4-oxo-2-oxetanyl]methyl]dodecyl (S or R)-2-isopropylmalonamate,
(S)-1-[[(2S,3S)-3-hexyl-4-oxo-2-oxetanyl]methyl]dodecyl (RS)-2-carbamoylvalerate (epimers 1:1),
(all Z,S)-1-[[(2S,3S)-3-ethyl-4-oxo-2-oxetanyl]methyl]-9,12-octadecadienyl (S or R)-2-isopropylmalonamate
(S)-1-[[(2S,3S)-3-hexyl-4-oxo-2-oxetanyl]methyl]dodecyl (RS)-2-carbamoyl-4-methylvalerate (epimers 1:1),
(S)-1-[[(2S,3S)-3-hexyl-4-oxo-2-oxetanyl]methyl]dodecyl 1-carbamoylcyclohexanecarboxylate,
(S)-1-[[(2S,3S)-3-hexyl-4-oxo-2-oxetanyl]methyl]dodecyl (RS)-2-methylmalonamate (epimers 1:1),
(S)-1-[[(2S,3S)-3-hexyl-4-oxo-2-oxetanyl]methyl]dodecyl (RS)-2-ethylmalonamate (epimers 1:1),
(S)-1-[[(2S,3S)-3-hexyl-4-oxo-2-oxetanyl]methyl]dodecyl (RS)-2-butylmalonamate (epimers 1:1),
(S)-1-[[(2S,3S)-3-ethyl-4-oxo-2-oxetanyl]methyl]octadecyl 1-carbamoylcyclohexanecarboxylate,
(S)-1-[(2S,3S or 2R,3R)-4-oxo-3-pentylthio-2-oxetanyl]-methyl]dodecyl [S:R or R:S(2:1)]-2-isopropylmalonamate,
(S)-1-[[(2R,3R)-3-benzyl-4-oxo-2-oxetanyl]methyl]dodecyl (S or R)-2-isopropylmalonamate.

**10.** Oxetanones according to claim 1 for use as pharmaceutically active substances.

**11.** A process for the manufacture of the oxetanones of claim 1, characterized by

a) esterifying an alcohol of the formula

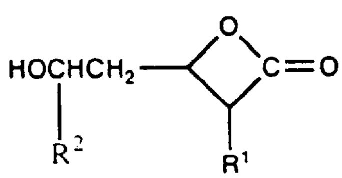

IIa

with an acid of the formula $Q^a$-OH, wherein $Q^a$ is a group of formula $Q^1$ or $Q^3$, or

b) cyclizing an acid of the formula

$$(Q\text{-}O,R^2)CHCH_2CH(OH)CH(R^1)\text{-}COOH \qquad IIb$$

or

c) converting the carboxy group in the group T in an acid of the formula

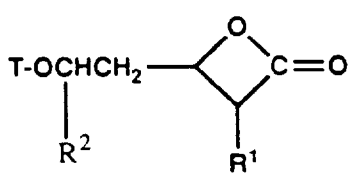

IIc

wherein T is a group of the formula

$$HOCO(X)_n\text{-}CO\text{-} \qquad T^1$$

or

$$HOCO\text{-}X'\text{-} \qquad T^{2,}$$

into an amide group ($R^3$, $R^4$)NCO-, and

d) if desired, separating a mixture of epimers of formula I into the individual epimers.

**12.** A medicament containing an oxetanone according to any one of claims 1-9 and a therapeutically inert carrier material.

**13.** The use of an oxetanone according to any one of claims 1-9 for the manufacture of a medicament for the control or prevention of obesity, hyperlipemia, atherosclerosis and arteriosclerosis.

**Claims for the following Contracting State : ES**

**1.** A process for the manufacture of oxetanones of the formula

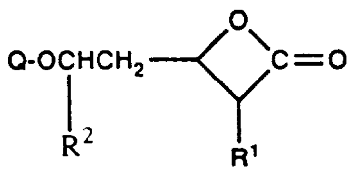

I

wherein Q is a group of the formula

$$(R^3,R^4)NCO(X)_n\text{-CO-} \qquad Q^1$$

$$(R^3,R^4)NCO\text{-}X'\text{-} \qquad Q^2$$

or

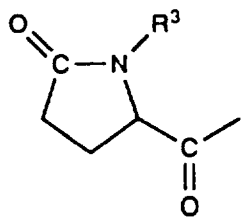

$Q^3$

and

$R^1$ and $R^2$ are alkyl with up to 18 C atoms substituted by 1 to 3 halogen atoms or alkyl, alkenyl, alkynyl or alkadienyl groups with up to 20 C atoms optionally interrupted by a 1,4-arylene group, optionally substituted by an aryl group in the ω-position and optionally substituted by an aryl-$C_{1-4}$-alkyl group, whereby $R^1$ can be interrupted by an O or S atom or by a sulphinyl or sulphonyl group in a position other than the α-position to an unsaturated C atom, or

$R^1$ is an aryl-NH- or aryl-$C_{1-4}$-alkyl-OCONH- group,

$R^3$ and $R^4$ are hydrogen or $C_{1-4}$-alkyl or together with the N atom to which they are attached form a saturated 3- to 6-membered ring optionally containing an O or S atom in a position other than the α-position to the N atom,

n is the number 1 or 0,

X is an alkylene group, which contains up to 6 C atoms, which is optionally interrupted by an O or S atom or by a sulphinyl or sulphonyl group and which is optionally substituted by a hydroxy, mercapto, aryl, aryloxy, arylthio, aryl-$C_{1-4}$-alkyl, aryl-$C_{1-4}$-alkoxy, aryl-$C_{1-4}$-alkylthio, aryl-$C_{1-4}$-alkylidene, $C_{3-7}$-cycloalkylidene or $C_{1-6}$-alkylidene group or by one or two $C_{1-6}$-alkyl, $C_{1-6}$-alkoxy or $C_{1-6}$-alkylthio groups, whereby two $C_{1-6}$-alkyl, $C_{1-6}$-alkoxy or $C_{1-6}$-alkylthio groups on the same C atom or on two adjacent C atoms can form an optionally mono-unsaturated 3- to 7-membered ring and an optionally present hydroxy or mercapto group or an optionally present unsaturated C atom must be in a position other than the α-position to an optionally present O or S atom or to an optionally present sulphinyl or sulphonyl group, or

X is a group of the formula

$$=CHN(R,R°) \text{ or } -CHN(R,R°)CH_2-$$

R and R° are hydrogen, $C_{1-4}$-alkyl, $C_{1-4}$-alkyl(CO or OCO)-, aryl, aryl(CO or OCO)-, aryl-$C_{1-4}$-alkyl or aryl-$C_{1-4}$-alkyl(CO or OCO)- and

X' is an alkylene group containing up to 6 C atoms which can be substituted by a $C_{1-4}$-alkoxy, aryl, aryloxy, arylthio, aryl-$C_{1-4}$-alkyl, aryl-$C_{1-4}$-alkoxy or aryl-$C_{1-4}$-alkylthio group or by one or two $C_{1-6}$-alkyl groups, whereby two $C_{1-6}$-alkyl groups attached to adjacent C atoms can form a 3- to 7-membered ring,

characterized by

a) esterifying an alcohol of the formula

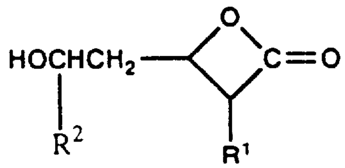

IIa

with an acid of the formula $Q^a$-OH, wherein $Q^a$ is a group of formula $Q^1$ or $Q^3$, or

b) cyclizing an acid of the formula

$$(Q\text{-}O,R^2)CHCH_2CH(OH)CH(R^1)\text{-}COOH \qquad IIb$$

or

c) converting the carboxy group in the group T in an acid of the formula

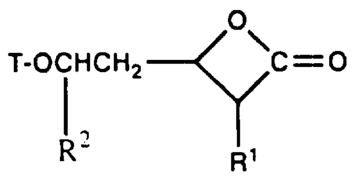

IIc

wherein T is a group of the formula

$$HOCO(X)_n\text{-}CO\text{-} \qquad T^1$$

or

$$HOCO\text{-}X'\text{-} \qquad T^2,$$

into an amide group ($R^3$, $R^4$)NCO-, and

d) if desired, separating a mixture of epimers of formula I into the individual epimers.

**2.** A process according to claim 1, wherein Q is a group of the formula $Q^1$,
$R^1$ and $R^2$ are alkyl, alkenyl or alkadienyl groups with up to 20 C atoms optionally interrupted by a 1,4-phenylene group, optionally substituted by a phenyl group in the ω-position and optionally substituted by a phenyl-$C_{1-4}$-alkyl group, whereby $R^1$ can be interrupted by an O or S atom in a position other than the α-position to an unsaturated C atom,
X is an alkylene group, which contains up to 6 C atoms, which is optionally interrupted by an O or S atom and which is optionally substituted by a hydroxy, mercapto, phenyl, phenoxy, phenylthio, phenyl-$C_{1-4}$-alkyl, phenyl-$C_{1-4}$-alkoxy, phenyl-$C_{1-4}$-alkylthio, phenyl-$C_{1-4}$-alkylidene, $C_{3-7}$-cycloalkylidene or $C_{1-6}$-alkylidene group or by one or two $C_{1-6}$-alkyl, $C_{1-6}$-alkoxy or $C_{1-6}$-alkylthio groups, whereby two $C_{1-6}$-alkyl, $C_{1-6}$-alkoxy or $C_{1-6}$-alkylthio groups attached to the same C atom can form a 3- to 7-membered ring and an optionally present hydroxy or mercapto group must be in a position other than the α-position to an optionally present O or S atom, or
X is a group =CHN(R,R°),
R and R° are hydrogen, $C_{1-4}$-alkyl, $C_{1-4}$-alkyl-(CO or OCO)-, phenyl or phenyl-(CO or OCO)- and
n, $R^3$ and $R^4$ have the significance given in claim 1.

**3.** A process according to claim 1, wherein Q is a group $Q^1$,
$R^1$ and $R^2$ are alkyl, alkenyl, alkynyl or alkadienyl groups with up to 20 C atoms optionally substituted by an aryl group in the ω-position, whereby $R^1$can be interrupted by a S atom in a position other than the α-position to an unsaturated C atom, or

$R^1$ is anilino, alkyl with up to 20 C atoms substituted by a halogen atom or a phenyl-$C_{1-4}$-alkyl-OCONH- group, $R^3$ and $R^4$ are hydrogen or $C_{1-4}$-alkyl or together with the N atoms to which they are attached form a saturated 6-membered ring containing an O or S atom in a position other than the $\alpha$-position to the N atom, n is the number 1 or 0,
X is an alkylene group, which contains up to 6 C atoms, which is optionally interrupted by an O or S atom or by a sulphinyl group and which is optionally substituted by one or two $C_{1-6}$-alkyl or $C_{1-6}$-alkoxy groups, whereby two $C_{1-6}$-alkyl or $C_{1-6}$-alkoxy groups attached to the same C atom or to two adjacent C atoms can form an optionally mono-unsaturated 3- to 7-membered ring, or
X is a group =CHN(R,R°) or -CHN(R,R°)$CH_2$- and
R and R° are hydrogen, $C_{2-5}$-alkanoyl or benzyloxycarbonyl.

**4.** A process according to claim 3, wherein $R^1$ is methyl, ethyl, propyl, hexyl, 2-butenyl, 3-methyl-2-butenyl, 2-propynyl, methylthio, pentylthio, 5-chloropentyl, benzyl, phenyl- thio, benzylthio, pentafluorobenzyl, anilino or benzyloxycarbonylamino, $R^2$ is undecyl, heptadecyl or 8,11-heptadecadienyl, $R^3$ and $R^4$ are hydrogen, methyl or isopropyl or together with the N atom form a morpholino or thiomorpholino group, n is the number 1 or 0 and X is the group $(CH_2)_{1-8}$, ethylidene, propylidene, isopropylidene, butylidene, isobutylidene, pentylidene, isopentylidene, t-butylmethylene dimethylvinylidene, cyclopentylidene, cyclohexylidene, phenethylidene, phenylpropylidene, 1,2-cyclohexylene, cyclohex-3-en-1,6-ylene, acetamidomethylene, benzyloxycarbonylaminomethylene, 1-benzyloxycarbonylamino-1,2-ethylene, methyleneoxymethylene, methylenethiomethylene, methylenesulphinylmethylene, ethylenethioethylene, ethylene-sulphinylethylene, methoxymethylene or ethylene- or propylene-dioxymethylene.

**5.** A process according to claim 1, wherein Q is a group $Q^2$; $R^1$ and $R^2$ are $C_{1-20}$-alkyl, $R^3$ and $R^4$ are hydrogen and X' is an alkylene group containing up to 6 C atoms which can be substituted by a $C_{1-4}$-alkoxy group or by one or two $C_{1-6}$-alkyl groups, whereby two $C_{1-6}$-alkyl groups attached to adjacent C atoms can form a 3- to 7-membered ring.

**6.** A process according to claim 5, wherein $R^1$ is hexyl, $R^2$ is undecyl and X' is ethylene, 1-methoxy-1,2-ethylene or 1,2-cyclohexylene.

**7.** A process according to claim 1, wherein Q is a group $Q^3$; $R^3$ is hydrogen and $R^1$ and $R^2$ are $C_{1-20}$-alkyl, especially hexyl or undecyl.

**8.** A process according to claim 1, characterized in that compounds from the following group are manufactured:

(S)-1-[[(2S,3S)-3-Hexyl-4-oxo-2-oxetanyl]methyl]dodecyl (S)-2-isopropylmalonamate,
(S)-1-[[(2S,3S)-3-hexyl-4-oxo-2-oxetanyl]methyl]dodecyl (S or R)-2-carbamoylvalerate,
(all Z,S)-1-[[(2S,3S)-3-ethyl-4-oxo-2-oxetanyl]methyl]-9,12-octadecadienyl (S)-2-isopropylmalonamate,
(S)-1-[[(2S,3S or 2R,3R)-4-oxo-3-pentylthio-2-oxetanyl] methyl]dodecyl (S)-2-isopropylmalonamate,
(S)-1-[(2S,3S) or 2R,3R)-4-oxo-3-pentylthio-2-oxetanyl] methyl]dodecyl [S:R(2:1)]-2-isopropylmalonamate,
(S)-1-[[(2S,3S)-3-ethyl-4-oxo-2-oxetanyl]methyl]octadecyl (S or R)-2-t-butylmalonamate
(S)-1-[[(2S,3S)-3-ethyl-4-oxo-2-oxetanyl]methyl]octadecyl 1 -carbamoylcyclopentanecarboxylate,
(S)-1-[[(2S,3S)-3-hexyl-4-oxo-2-oxetanyl]methyl]dodecyl (RS)-2-benzylmalonamate,
(S)-1-[[(2S,3S)-3-hexyl-4-oxo-2-oxetanyl]methyl]dodecyl 3-[(2-carbamoylethyl)thio]propionate,
5-oxo-D-proline (S)-1-[[(2S,3S)-3-ethyl-4-oxo-2-8-oxetanyl]methyl]octadecyl ester,
5-oxo-L-proline (S)-1-[[(2S,3S)-3-ethyl-4-oxo-2-oxetanyl]methyl]octadecyl ester.

**9.** A process according to claim 2, characterized in that compounds from the following group are manufactured:

(S)-1-[[(2S,3S)-3-Hexyl-4-oxo-2-oxetanyl]methyl]dodecyl (S or R)-2-isopropylmalonamate,
(S)-1-[[(2S,3S)-3-hexyl-4-oxo-2-oxetanyl]methyl]dodecyl (RS)-2-carbamoylvalerate (epimers 1:1),
(all Z,S)-1-[[(2S,3S)-3-ethyl-4-oxo-2-oxetanyl]methyl]-9,12-octadecadienyl (S or R)-2-isopropylmalonamate
(S)-1-[[(2S,3S)-3-hexyl-4-oxo-2-oxetanyl]methyl]dodecyl (RS)-2-carbamoyl-4-methylvalerate (epimers 1:1),
(S)-1-[[(2S,3S)-3-hexyl-4-oxo-2-oxetanyl]methyl]dodecyl 1 -carbamoylcyclohexanecarboxylate,
(S)-1-[[(2S,3S)-3-hexyl-4-oxo-2-oxetanyl]methyl]dodecyl (RS)-2-methylmalonamate (epimers 1:1),
(S)-1-[[(2S,3S)-3-hexyl-4-oxo-2-oxetanyl]methyl]dodecyl (RS)-2-ethylmalonamate (epimers 1:1),
(S)-1-[[(2S,3S)-3-hexyl-4-oxo-2-oxetanyl]methyl]dodecyl (RS)-2-butylmalonamate (epimers 1:1),
(S)-1-[[(2S,3S)-3-ethyl-4-oxo-2-oxetanyl]methyl]octadecyl 1-carbamoylcyclohexanecarboxylate,

(S)-1-[(2S,3S or 2R,3R)-4-oxo-3-pentylthio-2-oxetanyl] methyl]dodecyl [S:R or R:S(2:1)]-2-isopropylmalonamate,
(S)-1-[[(2R,3R)-3-benzyl-4-oxo-2-oxetanyl]methyl]dodecyl (S or R)-2-isopropylmalonamate.

**10.** The use of an oxetanone according to claim 1 for the manufacture of a medicament for the control or prevention of obesity, hyperlipemia, atherosclerosis and arteriosclerosis.

**Claims for the following Contracting State : GR**

**1.** A process for the manufacture of oxetanones of the formula

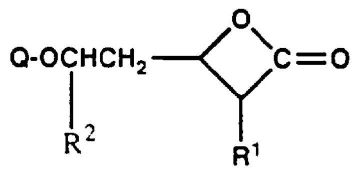

I

wherein Q is a group of the formula

$$(R^3,R^4)NCO(X)_n\text{-}CO\text{-} \qquad Q^1$$

$$(R^3,R^4)NCO\text{-}X'\text{-} \qquad Q^2$$

or

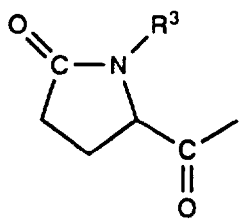

$Q^3$

and

$R^1$ and $R^2$ are alkyl with up to 18 C atoms substituted by 1 to 3 halogen atoms or alkyl, alkenyl, alkynyl or alkadienyl groups with up to 20 C atoms optionally interrupted by a 1,4-arylene group, optionally substituted by an aryl group in the ω-position and optionally substituted by an aryl-$C_{1-4}$-alkyl group, whereby $R^1$ can be interrupted by an O or S atom or by a sulphinyl or sulphonyl group in a position other than the α-position to an unsaturated C atom, or

$R^1$ is an aryl-NH- or aryl-$C_{1-4}$-alkyl-OCONH- group,

$R^3$ and $R^4$ are hydrogen or $C_{1-4}$-alkyl or together with the N atom to which they are attached form a saturated 3- to 6-membered ring optionally containing an O or S atom in a position other than the α-position to the N atom,

n is the number 1 or 0,

X is an alkylene group, which contains up to 6 C atoms, which is optionally interrupted by an O or S atom or by a sulphinyl or sulphonyl group and which is optionally substituted by a hydroxy, mercapto, aryl, aryloxy, arylthio, aryl-$C_{1-4}$-alkyl, aryl-$C_{1-4}$-alkoxy, aryl-$C_{1-4}$-alkylthio, aryl-$C_{1-4}$-alkylidene, $C_{3-7}$-cycloalkylidene or $C_{1-6}$-alkylidene group or by one or two $C_{1-6}$-alkyl, $C_{1-6}$-alkoxy or $C_{1-6}$-alkylthio groups, whereby two $C_{1-6}$-alkyl, $C_{1-6}$-alkoxy or $C_{1-6}$-alkylthio groups on the same C atom or on two adjacent C atoms can form an optionally mono-unsaturated 3- to 7-membered ring and an optionally present hydroxy or mercapto group or an optionally present unsaturated C atom must be in a position other than the α-position to an optionally present O or S atom or to an optionally present sulphinyl or sulphonyl group, or X is a group of the formula

$$=CHN(R,R°) \text{ or } -CHN(R,R°)CH_2-$$

R and R° are hydrogen, $C_{1-4}$-alkyl, $C_{1-4}$-alkyl(CO or OCO)-, aryl, aryl(CO or OCO)-, aryl-$C_{1-4}$-alkyl or aryl-$C_{1-4}$-alkyl(CO or OCO)- and

X' is an alkylene group containing up to 6 C atoms which can be substituted by a $C_{1-4}$-alkoxy, aryl, aryloxy, arylthio, aryl-$C_{1-4}$-alkyl, aryl-$C_{1-4}$-alkoxy or aryl-$C_{1-4}$-alkylthio group or by one or two $C_{1-6}$-alkyl groups, whereby two $C_{1-6}$-alkyl groups attached to adjacent C atoms can form a 3- to 7-membered ring,

characterized by

a) esterifying an alcohol of the formula

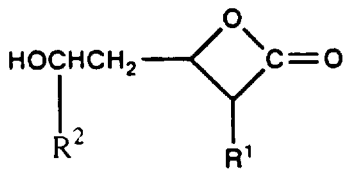

IIa

with an acid of the formula $Q^a$-OH, wherein $Q^a$ is a group of formula $Q^1$ or $Q^3$, or

b) cyclizing an acid of the formula

$$(Q\text{-}O,R^2)CHCH_2CH(OH)CH(R^1)\text{-}COOH \qquad IIb$$

or

c) converting the carboxy group in the group T in an acid of the formula

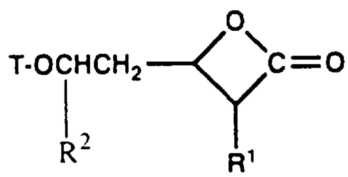

IIc

wherein T is a group of the formula

$$HOCO(X)_n\text{-}CO\text{-} \qquad T^1$$

or

$$HOCO\text{-}X'\text{-} \qquad T^{2,}$$

into an amide group ($R^3$, $R^4$)NCO-, and

d) if desired, separating a mixture of epimers of formula I into the individual epimers.

**2.** A process according to claim 1, wherein Q is a group of the formula $Q^1$,
$R^1$ and $R^2$ are alkyl, alkenyl or alkadienyl groups with up to 20 C atoms optionally interrupted by a 1,4-phenylene group, optionally substituted by a phenyl group in the ω-position and optionally substituted by a phenyl-$C_{1-4}$-alkyl group, whereby $R^1$ can be interrupted by an O or S atom in a position other than the α-position to an unsaturated C atom,
X is an alkylene group, which contains up to 6 C atoms, which is optionally interrupted by an O or S atom and which is optionally substituted by a hydroxy, mercapto, phenyl, phenoxy, phenylthio, phenyl-$C_{1-4}$-alkyl, phenyl-$C_{1-4}$-alkoxy, phenyl-$C_{1-4}$-alkylthio, phenyl-$C_{1-4}$-alkylidene, $C_{3-7}$-cycloalkylidene or $C_{1-6}$-alkylidene group or by one or two $C_{1-6}$-alkyl, $C_{1-6}$-alkoxy or $C_{1-6}$-alkylthio groups, whereby two $C_{1-6}$-alkyl, $C_{1-6}$-alkoxy or $C_{1-6}$-alkylthio groups attached to the same C atom can form a 3- to 7-membered ring and an optionally present hydroxy or mercapto group must be in a position other than the α-position to an optionally present O or S atom, or
X is a group =CHN(R,R°),
R and R° are hydrogen, $C_{1-4}$-alkyl, $C_{1-4}$-alkyl-(CO or OCO)-, phenyl or phenyl-(CO or OCO)- and
n, $R^3$ and $R^4$ have the significance given in claim 1.

**3.** A process according to claim 1, wherein Q is a group $Q^1$,
$R^1$ and $R^2$ are alkyl, alkenyl, alkynyl or alkadienyl groups with up to 20 C atoms optionally substituted by an aryl group in the ω-position, whereby $R^1$ can be interrupted by a S atom in a position other than the α-position to an unsaturated C atom, or
$R^1$ is anilino, alkyl with up to 20 C atoms substituted by a halogen atom or a phenyl-$C_{1-4}$-alkyl-OCONH- group,
$R^3$ and $R^4$ are hydrogen or $C_{1-4}$-alkyl or together with the N atoms to which they are attached form a saturated 6-membered ring containing an O or S atom in a position other than the α-position to the N atom, n is the number 1 or 0,

X is an alkylene group, which contains up to 6 C atoms, which is optionally interrupted by an O or S atom or by a sulphinyl group and which is optionally substituted by one or two $C_{1-6}$-alkyl or $C_{1-6}$-alkoxy groups, whereby two $C_{1-6}$-alkyl or $C_{1-6}$-alkoxy groups attached to the same C atom or to two adjacent C atoms can form an optionally mono-unsaturated 3- to 7-membered ring, or
X is a group =CHN(R,R°) or -CHN(R,R°)$CH_2$- and
R and R° are hydrogen, $C_{2-5}$-alkanoyl or benzyloxycarbonyl.

**4.** A process according to claim 3, wherein $R^1$ is methyl, ethyl, propyl, hexyl, 2-butenyl, 3-methyl-2-butenyl, 2-propynyl, methylthio, pentylthio, 5-chloropentyl, benzyl, phenyl- thio, benzylthio, pentafluorobenzyl, anilino or benzyloxycarbonylamino, $R^2$ is undecyl, heptadecyl or 8,11-heptadecadienyl, $R^3$ and $R^4$ are hydrogen, methyl or isopropyl or together with the N atom form a morpholino or thiomorpholino group, n is the number 1 or 0 and X is the group $(CH_2)_{1-8}$, ethylidene, propylidene, isopropylidene, butylidene, isobutylidene, pentylidene, isopentylidene, t-butylmethylene dimethylvinylidene, cyclopentylidene, cyclohexylidene, phenethylidene, phenylpropylidene, 1,2-cyclohexylene, cyclohex-3-en-1,6-ylene, acetamidomethylene, benzyloxycarbonylaminomethylene, 1-benzyloxycarbonylamino-1,2-ethylene, methyleneoxymethylene, methylenethiomethylene, methylenesulphinylmethylene, ethylenethioethylene, ethylenesulphinylethylene, methoxymethylene or ethylene- or propylenedioxymethylene.

**5.** A process according to claim 1, wherein Q is a group $Q^2$; $R^1$ and $R^2$ are $C_{1-20}$-alkyl, $R^3$ and $R^4$ are hydrogen and X' is an alkylene group containing up to 6 C atoms which can be substituted by a $C_{1-4}$-alkoxy group or by one or two $C_{1-6}$-alkyl groups, whereby two $C_{1-6}$-alkyl groups attached to adjacent C atoms can form a 3- to 7-membered ring.

**6.** A process according to claim 5, wherein $R^1$ is hexyl, $R^2$ is undecyl and X' is ethylene, 1-methoxy-1,2-ethylene or 1,2-cyclohexylene.

**7.** A process according to claim 1, wherein Q is a group $Q^3$; $R^3$ is hydrogen and $R^1$ and $R^2$ are $C_{1-20}$-alkyl, especially hexyl or undecyl.

**8.** A process according to claim 1, characterized in that compounds from the following group are manufactured:

(S)-1-[[(2S,3S)-3-Hexyl-4-oxo-2-oxetanyl]methyl]dodecyl (S)-2-isopropylmalonamate,
(S)-1-[[(2S,3S)-3-hexyl-4-oxo-2-oxetanyl]methyl]dodecyl (S or R)-2-carbamoylvalerate,
(all Z,S)-1-[[(2S,3S)-3-ethyl-4-oxo-2-oxetanyl]methyl]-9,12-octadecadienyl (S)-2-isopropylmalonamate,
(S)-1-[[(2S,3S or 2R,3R)-4-oxo-3-pentylthio-2-oxetanyl] methyl]dodecyl (S)-2-isopropylmalonamate,
(S)-1-[(2S,3S) or 2R,3R)-4-oxo-3-pentylthio-2-oxetanyl] methyl]dodecyl [S:R(2:1)]-2-isopropylmalonamate,
(S)-1-[[(2S,3S)-3-ethyl-4-oxo-2-oxetanyl]methyl]octadecyl (S or R)-2-t-butylmalonamate
(S)-1-[[(2S,3S)-3-ethyl-4-oxo-2-oxetanyl]methyl]octadecyl 1-carbamoylcyclopentanecarboxylate,
(S)-1-[[(2S,3S)-3-hexyl-4-oxo-2-oxetanyl]methyl]dodecyl (RS)-2-benzylmalonamate,
(S)-1-[[(2S,3S)-3-hexyl-4-oxo-2-oxetanyl]methyl]dodecyl 3-[(2-carbamoylethyl)thio]propionate,
5-oxo-D-proline (S)-1-[[(2S,3S)-3-ethyl-4-oxo-2-oxetanyl]methyl]octadecyl ester,
5-oxo-L-proline (S)-1-[[(2S,3S)-3-ethyl-4-oxo-2-oxetanyl]methyl]octadecyl ester.

**9.** A process according to claim 2, characterized in that compounds from the following group are manufactured:

(S)-1-[[(2S,3S)-3-Hexyl-4-oxo-2-oxetanyl]methyl]dodecyl (S or R)-2-isopropylmalonamate,
(S)-1-[[(2S,3S)-3-hexyl-4-oxo-2-oxetanyl]methyl]dodecyl (RS)-2-carbamoylvalerate (epimers 1:1),
(all Z,S)-1-[[(2S,3S)-3-ethyl-4-oxo-2-oxetanyl]methyl]-9,12-octadecadienyl (S or R)-2-isopropylmalonamate
(S)-1-[[(2S,3S)-3-hexyl-4-oxo-2-oxetanyl]methyl]dodecyl (RS)-2-carbamoyl-4-methylvalerate (epimers 1:1),
(S)-1-[[(2S,3S)-3-hexyl-4-oxo-2-oxetanyl]methyl]dodecyl 1-carbamoylcyclohexanecarboxylate,
(S)-1-[[(2S,3S)-3-hexyl-4-oxo-2-oxetanyl]methyl]dodecyl (RS)-2-methylmalonamate (epimers 1:1),
(S)-1-[[(2S,3S)-3-hexyl-4-oxo-2-oxetanyl]methyl]dodecyl (RS)-2-ethylmalonamate (epimers 1:1),
(S)-1-[[(2S,3S)-3-hexyl-4-oxo-2-oxetanyl]methyl]dodecyl (RS)-2-butylmalonamate (epimers 1:1),
(S)-1-[[(2S,3S)-3-ethyl-4-oxo-2-oxetanyl]methyl]octadecyl 1-carbamoylcyclohexanecarboxylate,
(S)-1-[(2S,3S or 2R,3R)-4-oxo-3-pentylthio-2-oxetanyl] methyl]dodecyl [S:R or R:S(2:1)]-2-isopropylmalonamate,
(S)-1-[[(2R,3R)-3-benzyl-4-oxo-2-oxetanyl]methyl]dodecyl (S or R)-2-isopropylmalonamate.

**10.** A process for the manufacture of medicaments, especially for the control or prevention of obesity, hyperlipemia, atherosclerosis and arteriosclerosis, characterized by bringing an oxetanone of formula I and, if desired, one or more other therapeutically active substances into a galenical administration form.

**11.** The use of an oxetanone according to claim 1 for the manufacture of a medicament for the control or prevention of obesity, hyperlipemia, atherosclerosis and arteriosclerosis.

**Revendications**

**Revendications pour les Etats contractants suivants : AT, BE, CH, DE, DK, FR, GB, IT, LI, LU, NL, SE**

**1.** Oxétanones de formule

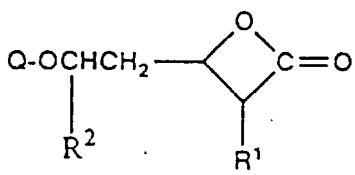

I

dans laquelle Q représente un groupe de formules

$(R^3,R^4)NCO(X)n\text{-}CO\text{-}$ Q1

$(R^3,R^4)NCO\text{-}X'\text{-}$ Q2

ou

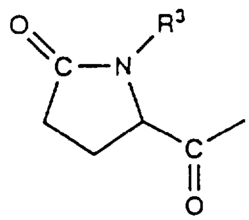

Q3

et $R^1$ et $R^2$ représentent un alkyle substitué par de 1 à 3 atomes d'halogène et ayant jusqu'à 18 atomes de carbone ou des groupes alkyle, alcényle, alcynyle ou alcadiényle ayant jusqu'à 20 atomes de carbone, éventuellement interrompus par un groupe 1,4-arylène, éventuellement substitués par un groupe aryle en position $\overline{\omega}$ et éventuellement par un groupe aryl-alkyle en $C_1$ à $C_4$, où $R^1$ peut être interrompu dans une position autre que la position $\alpha$ par rapport à un atome de carbone insaturé par un atome de O ou S ou par un groupe sulfinyle ou sulfonyle, ou
$R^1$ représente un groupe aryl-NH- ou aryl-alkyle en $C_1$ à $C_4$-OCONH-,
$R^3$ et $R^4$ représentent un hydrogène ou un alkyle en $C_1$ à $C_4$ ou forment, ensemble avec l'atome d'azote auquel ils sont liés, un noyau saturé à trois à six chaînons contenant éventuellement un atome de O ou de S dans une position autre que la position $\alpha$ par rapport à l'atome de N, n représente le nombre 1 ou 0,
X est un groupe alkylène éventuellement interrompu par un atome de O ou de S ou par un groupe sulfinyle ou sulfonyle, qui contient jusqu'à 6 atomes de carbone et qui est éventuellement substitué par un groupe hydroxy, mercapto, aryle, aryloxy, arylthio, aryl-alkyle en $C_1$ à $C_4$, aryl-alcoxy en $C_1$ à $C_4$, aryl-alkylthio en $C_1$ à $C_4$, aryl-alkylidène en $C_1$ à $C_4$, cycloalkylidène en $C_3$ à $C_7$ ou alkylidène en $C_1$ à $C_6$, ou par un ou deux groupes alkyle en $C_1$ à $C_6$, alcoxy en $C_1$ à $C_6$, ou alkylthio en $C_1$ à $C_6$, où deux groupes alkyle en $C_1$ à $C_6$, alcoxy en $C_1$ à $C_6$ ou alkylthio en $C_1$ à $C_6$ liés sur le même atome de carbone ou sur deux atomes de carbone voisins peuvent former un noyau à trois à sept chaînons éventuellement monoinsaturé et un groupe hydroxy ou mercapto éventuellement présent ou un atome de carbone insaturé éventuellement présent doit se situer dans une position autre que la position $\alpha$ par rapport à un atome de O ou de S éventuellement présent ou un groupe sulfonyle ou sulfinyle éventuellement présent, ou
X représente un groupe de formule

$-CHN\ (R, R°)$ ou $-CHN(R,R°)CH_2-$

R et R° représentent un hydrogène, un alkyle en $C_1$ à $C_4$, un alkyle en $C_1$ à $C_4$(CO ou OCO)-, un aryle, un aryle(CO ou OCO)-, un aryle-alkyle en $C_1$ à $C_4$ ou un aryle-alkyle en $C_1$ à $C_4$(CO ou OCO)- et X' est un groupe alkylène contenant jusqu'à 6 atomes de carbone qui peut être substitué par un groupe alcoxy en $C_1$ à $C_4$, aryle, aryloxy, arylthio, aryl-alkyle en $C_1$ à $C_4$, aryl-alcoxy en $C_1$ à $C_4$, ou aryl-alkylthio en $C_1$ à $C_4$ ou par un ou deux groupes alkyle en $C_1$ à $C_6$, où deux groupes alkyle en $C_1$ à $C_6$ liés sur des atomes de carbone voisins peuvent former un noyau à trois à sept chaînons.

**2.** Oxétanones de formule I selon la revendication 1, dans laquelle Q représente un groupe de formule $Q^1$, $R^1$ et $R^2$ sont des groupes alkyle, alcényle ou alcadiényle ayant jusqu'à 20 atomes de carbone, éventuellement interrompus par un groupe 1,4-phénylène, éventuellement substitués par un groupe phényle en position $\overline{\omega}$ et éventuellement substitués par un groupe phényl-alkyle en $C_1$ à $C_4$ , où $R^1$ peut être interrompu dans une position autre que la position $\alpha$ par rapport à un atome de carbone insaturé par un atome de O ou S, X est un groupe alkylène éventuellement interrompu par un groupe O ou S, qui contient jusqu'à 6 atomes de carbone et qui est éventuellement substitué par un groupe hydroxy, mercapto, phényle, phénoxy, phénylthio, phényl-alkyle en $C_1$ à $C_4$, phényl-alcoxy en $C_1$ à $C_4$, phényl-alkylthio en $C_1$ à $C_4$, phényl-alkylidène en $C_1$ à $C_4$, cycloalkylidène en $C_3$ à $C_7$ ou alkylidène en $C_1$ à $C_6$ ou par un ou deux groupes alkyle en $C_1$ à $C_6$, alcoxy en $C_1$ à $C_6$ ou alkylthio en $C_1$ à $C_6$ , où deux groupes alkyle en $C_1$ à $C_6$, alcoxy en $C_1$ à $C_6$ ou alkylthio en $C_1$ à $C_6$ liés sur le même atome de carbone peuvent former un noyau à 3 à 7 chaînons, et un groupe hydroxy ou mercapto éventuellement présent doit se situer dans une position autre que la position $\alpha$ par rapport à un atome de O ou de S éventuellement présent, et ou X représente un groupe =CHN(R,R°), R et R° représentent un hydrogène, un alkyle en $C_1$ à $C_4$, un alkyle en $C_1$ à $C_4$-(CO ou OCO)-, un phényle ou phényl-(CO ou OCO)- et n, $R^3$ et $R^4$ ont la signification donnée dans la revendication 1.

**3.** Oxétanones de formule I selon la revendication 1, dans laquelle Q représente un groupe $Q^1$, $R^1$ et $R^2$ représentent des groupes alkyle, alcényle, alcynyle ou alcadiényle éventuellement substitués par un groupe aryle en position $\overline{\omega}$ et ayant jusqu'à 20 atomes de carbone, où $R^1$ peut être interrompu dans une position autre que la position $\alpha$ par rapport à un atome de carbone insaturé, par un atome de S ou $R^1$ représente un anilino, un alkyle substitué par un atome d'halogène ayant jusqu'à 18 atomes de carbone ou un groupe phényl-alkyle en $C_1$ à $C_4$-OCONH-, $R^3$ et $R^4$ représentent un hydrogène ou un alkyle en $C_1$ à $C_4$ ou forment ensemble avec l'atome d'azote auquel ils sont liés un noyau à six chaînons saturé contenant un atome de O ou de S dans une position autre que la position $\alpha$ par rapport à l'atome de N, n représente le nombre 1 ou 0, X est un groupe alkylène éventuellement interrompu par un atome de O ou de S ou par un groupe sulfinyle, qui contient jusqu'à 6 atomes de carbone et qui est éventuellement substitué par un ou deux groupes alkyle en $C_1$ à $C_6$ ou alcoxy en $C_1$ à $C_6$, où deux groupes alkyle en $C_1$ à $C_6$ ou alcoxy en $C_1$ à $C_6$ liés sur le même atome de carbone ou sur deux atomes de carbone voisins peuvent former un noyau à 3 à 7 chaînons éventuellement monoinsaturé, ou X représente un groupe =CHN(R,R°) ou -CHN(R,R°)$CH_2$- et R et R° représentent un hydrogène, un alcanoyle en $C_2$ à $C_5$ ou un benzyloxycarbonyle.

**4.** Oxétanones selon la revendication 3, dans lesquels $R^1$ représente un méthyle, éthyle, propyle, hexyle, 2-butényle, 3-méthyl-2-butényle, 2-propynyle, méthylthio, pentylthio, 5-chloropentyle, benzyle, phénylthio, benzylthio, pentafluorobenzyle, anilino ou benzyloxycarbonylamino, $R^2$ représente un undécyle, heptadécyle ou 8,11-heptadécadiényle, $R^3$ et $R^4$ représentent un hydrogène, un méthyle ou un isopropyle ou forment ensemble avec l'atome de N un groupe morpholino ou thiomorpholino, n représente le nombre 1 ou 0 et X représente le groupe $(CH_2)_{1-8}$, un éthylidène, propylidène, isopropylidène, butylidène, isobutylidène, pentylidène, isopentylidène, t-butylméthylène, diméthyl-vinylidène, cyclopentylidène, cyclohexylidène, phénylidène, phénylpropylidène, 1,2-cyclohexylène, cyclohex-3-ène-1,6-ylène, acétamido-méthylène, benzyloxycarbonylaminométhylène, 1-benzyloxycarbonylamino-1,2-éthylène, méthylènoxyméthylène, méthylènethiométhylène, méthylènesulfinylméthylène, éthylènethioéthylène, éthylènesulfinyléthylène, méthoxyméthylène ou éthylène- ou propylènedioxyméthylène.

**5.** Oxétanones de forme I selon la revendication I, dans laquelle Q représente un groupe $Q^2$; $R^1$ et $R^2$ un alkyle en $C_1$ à $C_{20}$, $R^3$ et $R^4$ un hydrogène et X' un groupe alkylène contenant jusqu'à 6 atomes de carbone qui peut être substitué par un alcoxy en $C_1$ à $C_4$ ou par un ou deux groupes alkyle en $C_1$ à $C_6$ où deux groupes alkyle en $C_1$ à $C_6$ liés sur des atomes de carbone voisins peuvent former un noyau de 3 à 7 chaînons.

**6.** Oxétanones selon la revendication 5, dans lesquels $R^1$ représente un hexyle, $R^2$ un undécyle et X' un éthylène, 1-méthoxy-1,2-éthylène ou 1,2-cyclohexylène.

**7.** Oxétanones de formule I selon la revendication 1, dans laquelle Q représente un groupe $Q^3$; $R^3$ un hydrogène et $R^1$ et $R^2$ un alkyle en $C_1$ à $C_{20}$, en particulier un hexyle ou un undécyle.

**8.** Oxétanones selon la revendication 1 du groupe des composés suivants:

(S)-1-[[2S,3S)-3-hexyl-4-oxo-2-oxétanyl]méthyl] dodécyl-(S)-2 isopropyl-malonamate,
(S)-1-[[2S,3S)-3-hexyl-4-oxo-2-oxétanyl]méthyl] dodécyl-(S ou R)-2-carbamoylvalérate,
(tout Z,S)-1-[[2S,3S]-3-éthyl-4-oxo-2-oxétanyl]méthyl] -9,12-octadécadiényl-(2)-2-isopropylmalonamate,
(S)-1-[[2S,3S ou 2R,3R)-4-oxo-3-pentylthio-2-oxétanyl]méthyl]dodécyl-(S)-2-isopropylmalonamate,
(S)-1-[(2S,3S) ou 2R,3R)-4-oxo-3-pentylthio-2-oxétanyl]méthyl]docécyl-[S:R(2:1)]-2-isopropylmalonamate,
(S)-1-[[(2S,3S)-3-éthyl-4-oxo-2-oxétanyl]méthyl] octadécyl-(S ou R)-2-t-butylmalonamate,
(S)-1-[[(2S,3S)-3-éthyl-4-oxo-2-oxétanyl]méthyl] octadécyl-l-carbamoyl-cyclopentanecarboxylate,
(S)-1-[[(2S,3S)-3-hexyl-4-oxo-2-oxétanyl]méthyl] dodécyl-(RS)-2-benzylmalonamate,
(S)-1-[[(2S,3S)-3-hexyl-4-oxo-2-oxétanyl]méthyl] dodécyl-3- [(2-carbamoyléthyl) thio]propionate,
5-oxo-D-proline-(S)-1-[[(2S,3S)-3-éthyl-4-oxo-2-oxétanyl]méthyl]octadécyl-ester,
5-oxo-L-proline-(S)-1-[[(2S,3S)-3-éthyl-4-oxo-2-oxétanyl]méthyl]octadécyl-ester.

**9.** Oxétanones selon la revendication 2 du groupe des composés suivants:

(S)-1-[[2S,3S)-3-hexyl-4-oxo-2-oxétanyl]méthyl] dodécyl-(S ou R)-2-isopropylmalonamate,
(S)-1-[[2S,3S)-3-hexyl-4-oxo-2-oxétanyl]méthyl] dodécyl-(RS)-2-carbamoylvalérate (Epimère 1:1),
(tout Z,S)-1-[[2S,3S]-3-éthyl-4-oxo-2-oxétanyl]méthyl] -9,12-octadécadiényl-(S ou R)-(2)-2-isopropylmalonamate,
(S)-1-[[2S,3S)-3-hexyl-4-oxo-2-oxétanyl]méthyl] dodécyl-(RS)-2-carbamoyl-4-méthylvalérate (Epimère 1:1),
(S)-1-[(2S,3S)-3-hexyl-4-oxo-2-oxétanyl]méthyl] docécyl-1-carbamoylcyclohexanecarboxylate,
(S)-1-[[(2S,3S)-3-hexyl-4-oxo-2-oxétanyl]méthyl] dodécyl-(RS)-2-méthylmalonamate (Epimère 1:1),
(S)-1-[[(2S,3S)-3-hexyl-4-oxo-2-oxétanyl]méthyl] dodécyl-(RS)-2-éthylmalonamate (Epimère 1:1),
(S)-1-[[(2S,3S)-3-hexyl-4-oxo-2-oxétanyl]méthyl] dodécyl-(RS)-2-butylmalonamate (Epimère 1:1),
(S)-1-[[(2S,3S)-3-éthyl-4-oxo-2-oxétanyl]méthyl] octadécyl-1-carbamoylcyclohexanecarboxylate,
(S)-1-[[(2S,3S ou 2R,3R)-4-oxo-3-pentylthio-2-oxétanyl]méthyl]dodécyl-[S:R ou R:S(2:1)]-2-isopropylmalonamate,
(S)-1-[[(2R,3R)-3-benzyl-4-oxo-2-oxétanyl]méthyl] dodécyl-(S ou R)-2-isopropylmalonamate.

**10.** Oxétanones selon la revendication 1 aux fins d'application comme substances actives pharmaceutiques.

**11.** Procédé de préparation des oxétanones de formule I, caractérisé en ce que:

a) on estérifie un alcool de formule

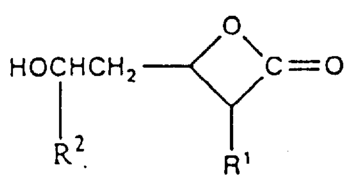

IIa

avec un acide de formule $Q^a$-OH, où $Q^a$ est un groupe de formule $Q^1$ ou $Q^3$, ou

b) on cyclise un acide de formule

$$(Q\text{-}O,R^2)CHCH_2CH(OH)CH(R^1)\text{-}COOH \quad IIb$$

ou

c) dans un acide de formule

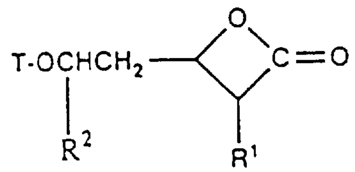

IIc

dans laquelle T est un groupe de formule

HOCO(X)$_n$-CO- $T^1$

ou

HOCO-X'- $T^2$

on transforme le groupe carboxy du groupe T en un groupe amide ($R^3$,$R^4$)NCO- et

d) si on le désire, on sépare un mélange d'épimères de formule I en les épimères isolés.

**12.** Médicament contenant une oxétanone selon l'une des revendications 1-9 et un support thérapeutiquement inerte.

**13.** Application des oxétanones selon l'une des revendications 1-9 à la préparation d'un médicament pour combattre ou prévenir l'obésité, l'hyperlipidémie, l'athérosclérose et l'artériosclérose.

**Revendications pour l'Etat contractant suivant : ES**

**1.** Procédé de préparation des oxétanones de formule

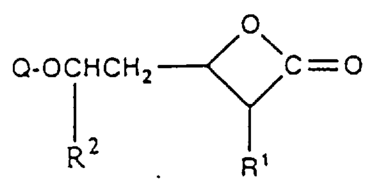

I

dans laquelle Q représente un groupe de formules

($R^3$,$R^4$)NCO(X)n-CO- Q1

($R^3$,$R^4$)NCO-X'- Q2

ou

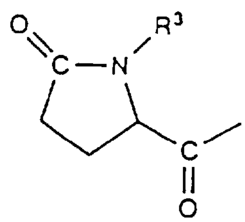

Q3

et $R^1$ et $R^2$ représentent un alkyle substitué par de 1 à 3 atomes d'halogène et ayant jusqu'à 18 atomes de carbone ou des groupes alkyle, alcényle, alcynyle ou alcadiényle ayant jusqu'à 20 atomes de carbone, éventuellement interrompus par un groupe 1,4-arylène, éventuellement substitués par un groupe aryle en position $\overline{\omega}$ et éventuellement par un groupe aryl-alkyle en $C_1$ à $C_4$, où $R^1$ peut être interrompu dans une position autre que la position α par rapport à un atome de carbone insaturé par un atome de O ou S ou par un groupe sulfinyle ou sulfonyle, ou $R^1$ représente un groupe aryl-NH- ou aryl-alkyle en $C_1$ à $C_4$-OCONH-,
$R^3$ et $R^4$ représentent un hydrogène ou un alkyle en $C_1$ à $C_4$ ou forment, ensemble avec l'atome d'azote auquel ils sont liés, un noyau saturé à trois à six chaînons contenant éventuellement un atome de O ou de S dans une position autre que la position α par rapport à l'atome de N, n représente le nombre 1 ou 0,

X est un groupe alkylène éventuellement interrompu par un atome de O ou de S ou par un groupe sulfinyle ou sulfonyle, qui contient jusqu'à 6 atomes de carbone et qui est éventuellement substitué par un groupe hydroxy, mercapto, aryle, aryloxy, arylthio, aryl-alkyle en $C_1$ à $C_4$, aryl-alcoxy en $C_1$ à $C_4$, aryl-alkylthio en $C_1$ à $C_4$, aryl-alkylidène en $C_1$ à $C_4$, cycloalkylidène en $C_3$ à $C_7$ ou alkylidène en $C_1$ à $C_6$, ou par un ou deux groupes alkyle en $C_1$ à $C_6$, alcoxy en $C_1$ à $C_6$, ou alkylthio en $C_1$ à $C_6$, où deux groupes alkyle en $C_1$ à $C_6$, alcoxy en $C_1$ à $C_6$ ou alkylthio en $C_1$ à $C_6$ liés sur le même atome de carbone ou sur deux atomes de carbone voisins peuvent former un noyau à trois à sept chaînons éventuellement monoinsaturé et un groupe hydroxy ou mercapto éventuellement présent ou un atome de carbone insaturé éventuellement présent doit se situer dans une position autre que la position $\alpha$ par rapport à un atome de O ou de S éventuellement présent ou un groupe sulfonyle ou sulfinyle éventuellement présent, ou

X représente un groupe de formule

$$-CHN(R,R^\circ) \text{ ou } -CHN(R,R^\circ)CH_2-$$

R et R° représentent un hydrogène, un alkyle en $C_1$ à $C_4$, un alkyle en $C_1$ à $C_4$(CO ou OCO)-, un aryle, un aryle (CO ou OCO)-, un aryle-alkyle en $C_1$ à $C_4$ ou un aryle-alkyle en $C_1$ à $C_4$(CO ou OCO)- et

X' est un groupe alkylène contenant jusqu'à 6 atomes de carbone qui peut être substitué par un groupe alcoxy en $C_1$ à $C_4$, aryle, aryloxy, arylthio, aryl-alkyle en $C_1$ à $C_4$, aryl-alcoxy en $C_1$ à $C_4$, ou aryl-alkylthio en $C_1$ à $C_4$ ou par un ou deux groupes alkyle en $C_1$ à $C_6$, où deux groupes alkyle en $C_1$ à $C_6$ liés sur des atomes de carbone voisins peuvent former un noyau à trois à sept chaînons,

caractérisé en ce que

a) on estérifie un alcool de formule

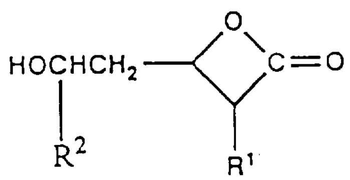

IIa

avec un acide de formule $Q^a$-OH, où $Q^a$ est un groupe de formule $Q^1$ ou $Q^3$, où

b) on cyclise un acide de formule

$$(Q\text{-}O,R^2)CHCH_2CH(OH)CH(R^1)\text{-}COOH \qquad IIb$$

ou

c) dans un acide de formule

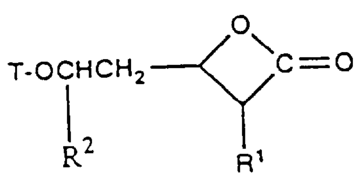

IIc

dans laquelle T est un groupe de formule

$$HOCO(X)_n\text{-}CO- \qquad T^1$$

ou

$$HOCO\text{-}X'\text{-} \qquad T^2$$

on transforme le groupe carboxy du groupe T en un groupe amide $(R^3,R^4)$NCO- et

d) si on le désire, on sépare un mélange d'épimères de formule I en les épimères isolés.

**2.** Procédé selon la revendication 1, dans laquelle Q représente un groupe de formule $Q^1$,

$R^1$ et $R^2$ sont des groupes alkyle, alcényle ou alcadiényle ayant jusqu'à 20 atomes de carbone, éventuellement interrompus par un groupe 1,4-phénylène, éventuellement substitués par un groupe phényle en position $\bar{\omega}$ et éventuellement substitués par un groupe phényl-alkyle en $C_1$ à $C_4$ où $R^1$ peut être interrompu dans une position autre que la position $\alpha$ par rapport à un atome de carbone insaturé par un atome de O ou S,

X est un groupe alkylène éventuellement interrompu par un atome de O ou de S, qui contient jusqu'à 6 atomes de carbone et qui est éventuellement substitué par un groupe hydroxy, mercapto, phényle, phénoxy, phénylthio, phé-

nyl-alkyle en $C_1$ à $C_4$, phényl-alcoxy en $C_1$ à $C_4$, phényl-alkylthio en $C_1$ à $C_4$, phényl-alkylidène en $C_1$ à $C_4$, cycloalkylidène en $C_3$ à $C_7$ ou alkylidène en $C_1$ à $C_6$ ou par un ou deux groupes alkyle en $C_1$ à $C_6$, alcoxy en $C_1$ à $C_6$ ou alkylthio en $C_1$ à $C_6$ , où deux groupes alkyle en $C_1$ à $C_6$, alcoxy en $C_1$ à $C_6$ ou alkylthio en $C_1$ à $C_6$ liés sur le même atome de carbone peuvent former un noyau à 3 à 7 chaînons, et un groupe hydroxy ou mercapto éventuellement présent doit se situer dans une position autre que la position α par rapport à un atome de O ou de S éventuellement présent, ou
X représente un groupe =CHN(R,R°),
R et R° représentent un hydrogène, un alkyle en $C_1$ à $C_4$, un alkyle en $C_1$ à $C_4$-(Co ou OCO)-, un phényle ou phényl-(CO ou OCO)- et
n, $R^3$ et $R^4$ ont la signification donnée dans la revendication 1.

3. Procédé selon la revendication 1, dans laquelle Q représente un groupe $Q^1$, $R^1$ et $R^2$ représentent des groupes alkyle, alcényle, alcynyle ou alcadiényle éventuellement substitués par un groupe aryle en position $\overline{\omega}$ et ayant jusqu'à 20 atomes de carbone, où $R^1$ peut être interrompu dans une position autre que la position α par rapport à un atome de carbone insaturé, par un atome de S ou
$R^1$ représente un anilino, un alkyle substitué par un atome d'halogène ayant jusqu'à 18 atomes de carbone ou un groupe phényl-alkyle en $C_1$ à $C_4$-OCONH-,
$R^3$ et $R^4$ représentent un hydrogène ou un alkyle en $C_1$ à $C_4$ ou forment ensemble avec l'atome d'azote auquel ils sont liés un noyau à six chaînons saturé contenant un atome de O ou de S dans une position autre que la position α par rapport à l'atome de N,
n représente le nombre 1 ou 0,
X est un groupe alkylène éventuellement interrompu par un atome de O ou de S ou par un groupe sulfinyle, qui contient jusqu'à 6 atomes de carbone et qui est éventuellement substitué par un ou deux groupes alkyle en $C_1$ à $C_6$ ou alcoxy en $C_1$ à $C_6$, où deux groupes alkyle en $C_1$ à $C_6$ ou alcoxy en $C_1$ à $C_6$ liés sur le même atome de carbone ou sur deux atomes de carbone voisins peuvent former un noyau à 3 à 7 chaînons éventuellement monoinsaturé, ou
X représente un groupe =CHN(R,R°) ou -CHN(R,R°)$CH_2$- et
R et R° représentent un hydrogène, un alcanoyle en $C_2$ à $C_5$ ou un benzyloxycarbonyle.

4. Procédé selon la revendication 3, dans lequel $R^1$ représente un méthyle, éthyle, propyle, hexyle, 2-butényle, 3-méthyl-2-butényle, 2-propynyle, méthylthio, pentylthio, 5-chloropentyle, benzyle, phénylthio, benzylthio, pentafluorobenzyle, anilino ou benzyloxycarbonylamino, $R^2$ représente un undécyle, heptadécyle ou 8,11-heptadécadiényle, $R^3$ et $R^4$ représentent un hydrogène, un méthyle ou un isopropyle ou forment ensemble avec l'atome de N un groupe morpholino ou thiomorpholino, n représente le nombre 1 ou 0 et X représente le groupe $(CH_2)_{1-8}$, un éthylidène, propylidène, isopropylidène, butylidène, isobutylidène, pentylidène, isopentylidène, t-butylméthylène, diméthyl-vinylidène, cyclopentylidène, cyclohexylidène, phénylidène, phénylpropylidène, 1,2-cyclohexylène, cyclohex-3-ène-1,6-ylène, acétamido-méthylène, benzyloxycarbonylaminométhylène, 1-benzyloxycarbonylamino-1,2-éthylène, méthylènoxyméthylène, méthylènethiométhylène, méthylènesulfinylméthylène, éthylènethioéthylène, éthylènesulfinyléthylène, méthoxyméthylène ou éthylène- ou propylènedioxyméthylène.

5. Procédé selon la revendication l, dans laquelle Q représente un groupe $Q^2$; $R^1$ et $R^2$ un alkyle en $C_1$ à $C_{20}$, $R^3$ et $R^4$ un hydrogène et X' un groupe alkylène contenant jusqu'à 6 atomes de carbone qui peut être substitué par un alcoxy en $C_1$ à $C_4$ ou par un ou deux groupes alkyle en $C_1$ à $C_6$ , où deux groupes alkyle en $C_1$ à $C_6$ liés sur des atomes de carbone voisins peuvent former un noyau de 3 à 7 chaînons.

6. Procédé selon la revendication 5, dans lequel $R^1$ représente un hexyle, $R^2$ un undécyle et X' un éthylène, 1-méthoxy-1,2-éthylène ou 1,2-cyclohexylène.

7. Procédé selon la revendication 1, dans laquelle Q représente un groupe $Q^3$; $R^3$ un hydrogène et $R^1$ et $R^2$ un alkyle en $C_1$ à $C_{20}$, en particulier un hexyle ou un undécyle.

8. Procédé selon la revendication 1, caractérisé en ce que l'on prépare des composés du groupe des composés suivants:

(S)-1-[[2S,3S)-3-hexyl-4-oxo-2-oxétanyl]méthyl] dodécyl-(S)-2-isopropyl-malonamate,
(S)-1-[[2S,3S)-3-hexyl-4-oxo-2-oxétanyl]méthyl] dodécyl-(S ou R)-2-carbamoylvalérate,
(tout Z,S)-1-[[2S,3S]-3-éthyl-4-oxo-2-oxétanyl]méthyl] -9,12-octadécadiényl-(S)-2-isopropylmalonamate,
(S)-1-[[2S,3S ou 2R,3R)-4-oxo-3-pentylthio-2-oxétanyl]méthyl]dodécyl-(S)-2-isopropylmalonamate,

(S)-1-[(2S,3S) ou 2R,3R)-4-oxo-3-pentylthio-2-oxétanyl]méthyl]docécyl-[S:R(2:1)]-2-isopropylmalonamate,
(S)-1-[[(2S,3S)-3-éthyl-4-oxo-2-oxétanyl]méthyl] octadécyl-(S ou R)-2-t-butylmalonamate,
(S)-1-[[(2S,3S)-3-éthyl-4-oxo-2-oxétanyl]méthyl] octadécyl-1-carbamoyl-cyclopentanecarboxylate,
(S)-1-[[(2S,3S)-3-hexyl-4-oxo-2-oxétanyl]méthyl] dodécyl-(RS)-2-benzylmalonamate,
(S)-1-[[(2S,3S)-3-hexyl-4-oxo-2-oxétanyl]méthyl] dodécyl-3-[(2-carbamoyléthyl)thio]propionate,
5-oxo-D-proline-(S)-1-[[(2S,3S)-3-éthyl-4-oxo-2-oxétanyl]méthyl]octadécyl-ester,
5-oxo-L-proline-(S)-1-[[(2S,3S)-3-éthyl-4-oxo-2-oxétanyl]méthyl]octadécyl-ester.

**9.** Procédé selon la revendication 2, caractérisé en ce que l'on prépare des composés du groupe des composés suivants:

(S)-1-[[2S,3S)-3-hexyl-4-oxo-2-oxétanyl]méthyl] dodécyl-(S-ou R)-2-isopropylmalonamate,
(S)-1-[[2S,3S)-3-hexyl-4-oxo-2-oxétanyl]méthyl] dodécyl-(RS)-2-carbamoylvalérate (Epimère 1:1),
(tout Z,S)-1-[[2S,3S]-3-éthyl-4-oxo-2-oxétanyl]méthyl] -9,12-octadécadiényl-(S ou R)-2-isopropylmalonamate,
(S)-1-[[2S,3S)-3-hexyl-4-oxo-2-oxétanyl]méthyl] dodécyl-(RS)-2-carbamoyl-4-méthylvalérate (Epimère 1:1),
(S)-1-[(2S,3S)-3-hexyl-4-oxo-2-oxétanyl]méthyl] docécyl-1-carbamoylcyclohexanecarboxylate,
(S)-1-[[(2S,3S)-3-hexyl-4-oxo-2-oxétanyl]méthyl] dodécyl-(RS)-2-méthylmalonamate (Epimère 1:1),
(S)-1-[[(2S,3S)-3-hexyl-4-oxo-2-oxétanyl]méthyl] dodécyl-(RS)-2-éthylmalonamate (Epimère 1:1),
(S)-1-[[(2S,3S)-3-hexyl-4-oxo-2-oxétanyl]méthyl] dodécyl-(RS)-2-butylmalonamate (Epimère 1:1),
(S)-1-[[(2S,3S)-3-éthyl-4-oxo-2-oxétanyl]méthyl] octadécyl-1-carbamoylcyclohexanecarboxylate,
(S)-1-[[(2S,3S ou 2R,3R)-4-oxo-3-pentylthio-2-oxétanyl]méthyl]dodécyl-[S:R ou R:S(2: 1)]-2-isopropylmalonamate,
(S)-1-[[(2R,3R)-3-benzyl-4-oxo-2-oxétanyl]méthyl] dodécyl-(S ou R)-2-isopropylmalonamate.

**10.** Application des oxétanones selon la revendication 1 à la préparation d'un médicament pour combattre ou prévenir l'obésité, l'hyperlipidémie, l'athérosclérose et l'artériosclérose.

**Revendications pour l'Etat contractant suivant : GR**

**1.** Procédé de préparation des oxétanones de formule

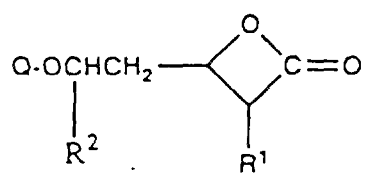

I

dans laquelle Q représente un groupe de formules

$(R^3,R^4)NCO(X)n\text{-}CO\text{-}$ Q1

$(R^3,R^4)NCO\text{-}X'\text{-}$ Q2

ou

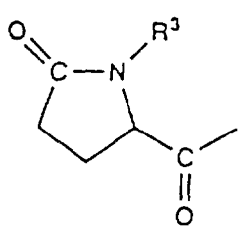

Q3

et $R^1$ et $R^2$ représentent un alkyle substitué par de 1 à 3 atomes d'halogène et ayant jusqu'à 18 atomes de carbone ou des groupes alkyle, alcényle, alcynyle ou alcadiényle ayant jusqu'à 20 atomes de carbone, éventuellement interrompus par un groupe 1,4-arylène, éventuellement substitués par un groupe aryle en position $\overline{\omega}$ et éventuellement un groupe aryl-alkyle en $C_1$ à $C_4$, où $R^1$ peut être interrompu dans une position autre que la position $\alpha$ par rapport à un atome de carbone insaturé par un atome de O ou S ou par un groupe sulfonyle ou sulfinyle, ou
$R^1$ représente un groupe aryl-NH ou aryl-alkyle en $C_1$ à $C_4$-OCONH-,

$R^3$ et $R^4$ représentent un hydrogène ou un alkyle en $C_1$ à $C_4$ ou forment, ensemble avec l'atome d'azote auquel ils sont liés, un noyau saturé à trois à six chaînons contenant éventuellement un atome de O ou de S dans une position autre que la position α par rapport à l'atome de N, n représente le nombre 1 ou 0,
X est un groupe alkylène éventuellement interrompu par un atome de O ou de S ou par un groupe sulfinyle ou sulfonyle, qui contient jusqu'à 6 atomes de carbone et qui est éventuellement substitué par un groupe hydroxy, mercapto, aryle, aryloxy, arylthio, aryl-alkyle en $C_1$ à $C_4$, aryl-alcoxy en $C_1$ à $C_4$, aryl-alkylthio en $C_1$ à $C_4$, aryl-alkylidène en $C_1$ à $C_4$, cycloalkylidène en $C_3$ à $C_7$, alkylidène en $C_1$ à $C_6$, ou par un ou deux groupes alkyle en $C_1$ à $C_6$, alcoxy en $C_1$ à $C_6$, ou alkylthio en $C_1$ à $C_6$, où deux groupes alkyle en $C_1$ à $C_6$, alcoxy en $C_1$ à $C_6$ ou alkylthio en $C_1$ à $C_6$ liés sur le même atome de carbone ou sur deux atomes de carbone voisins peuvent former un noyau à trois à sept chaînons éventuellement monoinsaturé et un groupe hydroxy ou mercapto éventuellement présent ou un atome de carbone insaturé éventuellement présent doit se situer dans une position autre que la position α par rapport à un atome de O ou de S éventuellement présent ou un groupe sulfonyle ou sulfinyle éventuellement présent, ou
X représente un groupe de formule

$$-CHN(R,R^{o}) \text{ ou } -CHN(R,R^{o})CH_2-$$

R et $R^o$ représentent un hydrogène, un alkyle en $C_1$ à $C_4$, un alkyle en $C_1$ à $C_4$(CO ou OCO), un aryle, un aryl(CO ou OCO), un aryle-alkyle en $C_1$ à $C_4$ ou un aryle-alkyle en $C_1$ à $C_4$(CO ou OCO)- et
X' est un groupe alkylène contenant jusqu'à 6 atomes de carbone qui peut être substitué par un groupe alcoxy en $C_1$ à $C_4$, aryle, aryloxy, arylthio, aryl-alkyle en $C_1$ à $C_4$, aryl-alcoxy en $C_1$ à $C_4$, ou aryl-alkyle en $C_1$ à $C_4$ ou par un ou deux groupes alkyle en $C_1$ à $C_6$, où deux groupes alkyle en $C_1$ à $C_6$ liés sur des atomes de carbone voisins peuvent former un noyau à trois à sept chaînons,
caractérisé en ce que

a) on estérifie un alcool de formule

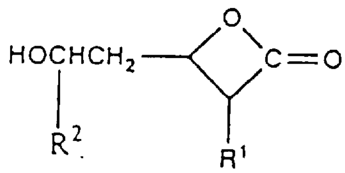

IIa

avec un acide de formule $Q^a$-OH, où $Q^a$ est un groupe de formule $Q^1$ ou $Q^3$, où
b) on cyclise un acide de formule

$$(Q\text{-}O,R^2)CHCH_2CH(OH)CH(R^1)\text{-}COOH \qquad IIb$$

ou
c) dans un acide de formule

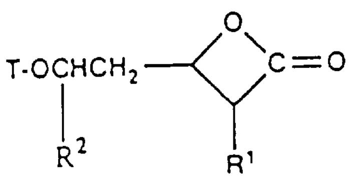

IIc

dans laquelle T est un groupe de formule

$$HOCO(X)_n\text{-}CO- \qquad T^1$$

ou

$$HOCO\text{-}X'- \qquad T^2$$

on transforme le groupe carboxy du groupe T en un groupe amide $(R^3,R^4)NCO$- et
d) si on le désire, on sépare un mélange d'épimères de formule I en les épimères isolés.

**2.** Procédé selon la revendication 1, dans laquelle Q représente un groupe de formule $Q^1$,
$R^1$ et $R^2$ sont des groupes alkyle, alcényle ou alcadiényle ayant jusqu'à 20 atomes de carbone, éventuellement interrompus par un groupe 1,4-phénylène, éventuellement substitués par un groupe phényle en position $\overline{\omega}$ et éventuellement substitués par un groupe phényl-alkyle en $C_1$ à $C_4$ , où $R^1$ peut être interrompu dans une position autre que la position α par rapport à un atome de carbone insaturé par un atome de O ou S,

X est un groupe alkylène éventuellement interrompu par un atome de O ou de S, qui contient jusqu'à 6 atomes de carbone et qui est éventuellement substitué par un groupe hydroxy, mercapto, phényle, phénoxy, phénylthio, phényl-alkyle en $C_1$ à $C_4$, phényl-alcoxy en $C_1$ à $C_4$, phényl-alkylthio en $C_1$ à $C_4$, phényl-alkylidène en $C_1$ à $C_4$, cycloalkylidène en $C_3$ à $C_7$ ou alkylidène en $C_1$ à $C_6$ ou par un ou deux groupes alkyle en $C_1$ à $C_6$, alcoxy en $C_1$ à $C_6$ ou alkylthio en $C_1$ à $C_6$ où deux groupes alkyle en $C_1$ à $C_6$, alcoxy en $C_1$ à $C_6$ ou alkylthio en $C_1$ à $C_6$ liés sur le même atome de carbone peuvent former un noyau à 3 à 7 chaînons, et un groupe hydroxy ou mercapto éventuellement présent doit se situer dans une position autre que la position $\alpha$ par rapport à un atome de O ou de S éventuellement présent, et ou
X représente un groupe =CHN(R,R°),
R et R° représentent un hydrogène, un alkyle en $C_1$ à $C_4$, un alkyle en $C_1$ à $C_4$-(Co ou OCO)-, un phényle ou un phényl(CO ou OCO)- et
n, $R^3$ et $R^4$ ont la signification donnée dans la revendication 1.

**3.** Procédé selon la revendication 1, dans laquelle Q représente un groupe $Q^1$, $R^1$ et $R^2$ représentent des groupes alkyle, alcényle, alcynyle ou alcadiényle éventuellement substitués par un groupe aryle en position $\bar{\omega}$ et ayant jusqu'à 20 atomes de carbone, où $R^1$ peut être interrompu dans une position autre que la position $\alpha$ par rapport à un atome de carbone insaturé par un atome de S ou
$R^1$ représente un anilino, un alkyle substitué par un atome d'halogène ayant jusqu'à 18 atomes de carbone ou un groupe phényl-alkyle en $C_1$ à $C_4$-OCONH-,
$R^3$ et $R^4$ représentent un hydrogène ou un alkyle en $C_1$ à $C_4$ ou forment ensemble avec l'atome d'azote auquel ils sont liés un noyau à six chaînons saturé contenant un atome de O ou de S dans une position autre que la position $\alpha$ par rapport à l'atome de N,
n représente le nombre 1 ou 0,
X est un groupe alkylène éventuellement interrompu par un atome de O ou de S ou par un groupe sulfinyle, qui contient jusqu'à 6 atomes de carbone et qui est éventuellement substitué par un ou deux groupes alkyle en $C_1$ à $C_6$, alcoxy en $C_1$ à $C_6$, où deux groupes alkyle en $C_1$ à $C_6$ ou alcoxy en $C_1$ à $C_6$ liés sur le même atome de carbone ou sur deux atomes de carbone voisins peuvent former un noyau à 3 à 7 chaînons éventuellement monoinsaturé, ou
X représente un groupe =CHN(R,R°) ou -CHN(R,R°)$CH_2$- et
R et R° représentent un hydrogène, un alcanoyle en $C_2$ à $C_5$ ou un benzyloxycarbonyle.

**4.** Procédé selon la revendication 3, dans lesquels $R^1$ représente un méthyle, éthyle, propyle, hexyle, 2-buténylе, 3-méthyl-2-buténylе, 2-propynyle, méthylthio, pentylthio, 5-chloropentyle, benzyle, phénylthio, benzylthio, pentafluorobenzyle, anilino ou benzyloxycarbonylamino, $R^2$ représente un undécyle, heptadécyle ou 8,11-heptadécadiényle, $R^3$ et $R^4$ représentent un hydrogène, un méthyle ou un isopropyle ou forment ensemble avec l'atome de N un groupe morpholino ou thiomorpholino, n représente le nombre 1 ou 0 et X représente le groupe $(CH_2)_{1-8}$, un éthylidène, propylidène, isopropylidène, butylidène, isobutylidène, pentylidène, isopentylidène, t-butylméthylène, diméthylvinylidène, cyclopentylidène, cyclohexylidène, phénylidène, phénylpropylidène, 1,2-cyclohexylène, cyclohex-3-ène-1,6-ylène, acétamido-méthylène, benzyloxycarbonylaminométhylène, 1-benzyloxycarbonylamino-1,2-éthylène, méthylènoxyméthylène, méthylènethiométhylène, méthylènesulfinylméthylène, éthylènethioéthylène, éthylènesulfinyléthylène, méthoxyméthylène ou éthylène ou propylènedioxyméthylène.

**5.** Procédé selon la revendication l, dans laquelle Q représente un groupe $Q^2$; $R^1$ et $R^2$ un alkyle en $C_1$ à $C_{20}$, $R^3$ et $R^4$ un hydrogène et X' un groupe alkylène contenant jusqu'à 6 atomes de carbone qui peut être substitué par un alcoxy en $C_1$ à $C_4$ ou par un ou deux groupes alkyle en $C_1$ à $C_6$ , où deux groupes alkyle en $C_1$ à $C_6$ liés sur des atomes de carbone voisins peuvent former un noyau de 3 à 7 chaînons.

**6.** Procédé selon la revendication 5, dans lequel $R^1$ représente un hexyle, $R^2$ un undécyle et X' un éthylène, 1-méthoxy-1,2-éthylène ou 1,2-cyclohexylène.

**7.** Procédé selon la revendication 1, dans laquelle Q représente un groupe $Q^3$; $R^3$ un hydrogène et $R^1$ et $R^2$ un alkyle en $C_1$ à $C_{20}$, en particulier un hexyle ou un undécyle.

**8.** Procédé selon la revendication 1, caractérisé en ce que l'on prépare des composés du groupe des composés suivants:

(S)-1-[[2S,3S)-3-hexyl-4-oxo-2-oxétanyl]méthyl] dodécyl-(S)-2-isopropyl-malonamate,
(S)-1-[[2S,3S)-3-hexyl-4-oxo-2-oxétanyl]méthyl] dodécyl-(S ou R)-2-carbamoylvalérate,
(tout Z,S)-1[[2S,3S]-3-éthyl-4-oxo-2-oxétanyl]méthyl] -9,12-octadécadiényl-(2)-2-isopropylmalonamate,

(S)-1-[[2S,3S ou 2R,3R)-4-oxo-3-pentylthio-2-oxétanyl]méthyl]dodécyl-(S)-2-isopropylmalonamate,
(S)-1-[(2S,3S) ou 2R,3R)-4-oxo-3-pentylthio-2-oxétanyl]méthyl]docécyl-[S:R(2:1)]-2-isopropylmalonamate,
(S)-1-[[(2S,3S)-3-éthyl-4-oxo-2-oxétanyl]méthyl] octadécyl-(S ou R)-2-t-butylmalonamate,
(S)-1-[[(2S,3S)-3-éthyl-4-oxo-2-oxétanyl]méthyl] octadécyl-1-carbamoyl-cyclopentanecarboxylate,
(S)-1-[[(2S,3S)-3-hexyl-4-oxo-2-oxétanyl]méthyl] dodécyl-(RS)-2-benzylmalonamate,
(S)-1-[[(2S,3S)-3-hexyl-4-oxo-2-oxétanyl]méthyl] dodécyl-3- [(2-carbamoyléthyl)thio]propionate,
5-oxo-D-proline-(S)-1-[[(2S,3S)-3-éthyl-4-oxo-2-oxétanyl]méthyl]octadécyl-ester,
5-oxo-L-proline-(S)-1-[[(2S,3S)-3-éthyl-4-oxo-2-oxétanyl]méthyl]octadécyl-ester.

**9.** Procédé selon la revendication 2, caractérisé en ce que l'on prépare des composés du groupe des composés suivants:

(S)-1-[[2S,3S)-3-hexyl-4-oxo-2-oxétanyl]méthyl] dodécyl-(S ou R)-2-isopropylmalonamate,
(S)-1-[[2S,3S)-3-hexyl-4-oxo-2-oxétanyl]méthyl] dodécyl-(S ou R)-2-carbamoylvalérate (Epimère 1:1),
(tout Z,S)-1-[[2S,3S]-3-éthyl-4-oxo-2-oxétanyl]méthyl] -9,12-octadécadiényl-(S ou R)-2-isopropylmalonamate,
(S)-1-[[2S,3S)-3-hexyl-4-oxo-2-oxétanyl]méthyl] dodécyl-(RS)-2-carbamoyl-4-méthylvalérate (Epimère 1:1),
(S)-1-[(2S,3S)-3-hexyl-4-oxo-2-oxétanyl]méthyl] docécyl-1-carbamoylcyclohexanecarboxylate,
(S)-1-[[(2S,3S)-3-hexyl-4-oxo-2-oxatanyl]méthyl] dodécyl-(RS)-2-méthylmalonamate (Epimère 1:1),
(S)-1-[[(2S,3S)-3-hexyl-4-oxo-2-oxétanyl]méthyl] dodécyl-(RS)-2-éthylmalonamate (Epimère 1:1),
(S)-1-[[(2S,3S)-3-hexyl-4-oxo-2-oxétanyl]méthyl] dodécyl-(RS)-2-butylmalonamate (Epimère 1:1),
(S)-1-[[(2S,3S)-3-éthyl-4-oxo-2-oxétanyl]méthyl] octadécyl-1-carbamoylcyclohexanecarboxylate,
(S)-1-[[(2S,3S ou 2R,3R)-4-oxo-3-pentylthio-2-oxétanyl]méthyl]dodécyl-[S:R ou R:S(2:1)]-2-isopropylmalonamate,
(S)-1-[[(2R,3R) -3-benzyl-4-oxo-2-oxétanyl]méthyl] dodécyl-(S ou R)-2-isopropylmalonamate.

**10.** Procédé pour la préparation de médicaments, en particulier pour combattre ou prévenir l'obésité, l'hyperlipidémie, l'athérosclérose et l'artériosclérose, caractérisé en ce qu'on met sous une forme d'administration galénique une oxétanone de formule I et si on le désire une ou plusieurs autres substances thérapeutiquement utiles.

**11.** Application d'oxétanones selon la revendication 1 pour la préparation de médicaments pour combattre ou prévenir l'obésité, l'hyperlipidémie, l'athérosclérose et l'artériosclérose.